# EUROPEAN PATENT APPLICATION

(11) **EP 4 056 546 A1**
(43) Date of publication of application: **14.09.2022**
(21) Application number: 21162064.6
(22) Date of filing: 11.03.2021
(51) Int. Cl.: C07B 59/00, A61K 47/68, A61K 51/04

(54) **ALIPHATIC 18F-RADIOLABELING OF A TETRAZINE PRECURSOR**

(71) Applicant: University of Copenhagen, 1165 Copenhagen K (DK); Rigshospitalet, 2100 Copenhagen Ø (DK)
(72) Inventor: HERTH, Matthias Manfred, 21121 Malmö (DK); KJÆR, Andreas, 2000 Frederiksberg (DK); JØRGENSEN, Jesper Tranekjær, 1165 Copenhagen (DK); BATTISTI, Umberto Maria, 1165 Copenhagen (DK); BRATTEBY, Klas Erik, 1165 Copenhagen (DK)
(74) Representative: Budde Schou A/S

(57) **Abstract**

Up ontil now, only low reactivity Tzs can be radiolabeled via direct aliphatic S_{N}2. Unfortunately, these structures display too low reactivity for in vivo bioorthogonal chemistry approaches. Highly reactive structures such as mono-unsubstituted tetrazines (H-Tzs) have been reported to be highly sensitive to base. Extensive degradation is observed which prevents isolation of meaningful amounts for imaging studies.

In the present invention there is provided a method providing the possibility to radiolabel base sensitive tetrazine structures with significantly improved RCYs. Even tetrazines that were previously not accessible by applying "standard" aliphatic ¹⁸F-labeling strategies can be radiolabeled. This places new classes of ¹⁸F-fluorinated compounds within reach for application in PET imaginge studies such as for diagnosis of cancers.

## Description

### FIELD OF THE INVENTION

The present invention relates to aliphatic 18F-radiolabeling of tetrazine precursors, the precursors for such labeling and the compound obtained as well as their uses.

### BACKGROUND OF THE INVENTION

Nanomedicines such as monoclonal antibodies or other nanoparticles have received increased interest e.g. in the field of oncology and drug delivery within the last decades. They can be used as selective drug or radionuclide delivery vectors. Their unique targeting properties allow for specific accumulation up to 30-50% injected dose per kg (ID/kg) in patients and bear as such great potential to improve state-of-the-art treatment. To identify which patients would benefit from nanomedicines, the targeting abilities of each nanomedicine needs to be quantified on an individual basis (precision medicine). Patient-to-patient variations in tumor uptake, even within the same cancer type, represent a challenge to identify the optimal therapeutic dose. This is of particular interest for radionuclide therapies, where radiolabeled compounds are designed with the aim of delivering radiation doses to specific targets upon injection. Molecular imaging techniques such as positron emission tomography (PET) are commonly applied to estimate the maximum tolerated radiation dose of such therapies, in respect to highest effectiveness with tolerable site-effects (theranostic concept) as well as for diagnoses of disease and monitoring of treatment progress.

The most widely used PET radionuclide is fluorine-18 (¹⁸F), as it can be produced in large amounts (>300 GBq) and possesses almost ideal nuclear decay characteristics for molecular imaging. Its low positron energy ensures high image resolution, while the half-life of approximately 110 min allows for production of ¹⁸F-radiopharmaceuticals for a large number of patients and their distribution to remote sites several hundred kilometers away.

Nanomedicines usually possess slow pharmacokinetics, i.e. slow target accumulation as well as slow excretion. These processes can take days to weeks. For targeted radionuclide approaches, this displays a challenge since high radiation doses are then delivered to healthy tissues, which limits and often prohibits the clinical application of these compounds.

Pretargeting offers an intriguing alternative which circumvent the dose limitations that conventional nanomedicine-based radionuclide therapies possess. Pretargeted strategies allow to label nanomedicines when they have already reached their target and have cleared from the rest of the body. The targeting nanomedicine is modified with a bioorthogonal tag and injected. The nanomedicine is allowed to accumulate at the target and to clear from the rest of the body. Subsequently, a complementary tag is radiolabeled and administered. This tag will bioorthogonally react with the tagged nanomedicine *in vivo* - conceptionally only at the target site and within minutes while unreacted tags are excreted rapidly. Thus, good target-to-background ratios can already be obtained after minutes. Consequently, radiation dose to healthy tissue is minimized.
A number of different bioorthogonal reactions have been employed for such approaches. Currently, the tetrazine ligation between a tetrazine (Tz) and a *trans*-cyclooctene (TCO) is the most effective reaction in this respect.
Up until now, only low reactivity Tzs can be radiolabeled via direct aliphatic SN2. Unfortunately, these structures display too low reactivity for in vivo bioorthogonal chemistry approaches. Highly reactive structures such as monounsubstituted tetrazines (H-Tzs) have been reported to be highly sensitive to base. Extensive degradation is observed which prevents isolation of meaningful amounts for imaging studies. Using "standard" conditions, no or only trace amounts of the radiolabeled product could be observed.

In WO2012012612 the inventors attempted to perform aliphatic 18F-radiolabeling of a monounsubstituted tetrazine and only achieved trace amounts of labelled product insufficient for application in PET imaging.

Nucleophilic aliphatic ¹⁸F-fluorination (S_{N}2) is one of the most widely applied ¹⁸F-radiolabeling methods. However, the standard approach to purify and concentrate [¹⁸F]fluoride requires strong bases as descibed in (Orit Jacobson, et al. Bioconjugate Chem. 2015, 26, 1-18)
were the ¹⁸F-fluoride is first trapped on an anion exchange cartridge to remove the target water in which it is produced. The activity is then eluted using basic anions in an aqeous solvent that is azeotropically distilled to produce a dry salt containing the activated fluorine-18 that can be used to label the desired precursor in a suitable reaction solvent. The resulting basic environment hinders (or even prevents) ¹⁸F-fluorination of high reactive tetrazines while triggering side-reactions such as hydrolysis, elimination and/or decomposition of precursors/products.
To address this challenge, a wide variety of methods to perform S_{N}2 ¹⁸F-fluorinations under less basic conditions have been developed over the last decades.
One example of such a method is described in "Fast and reliable generation of [18F]triflyl fluoride, a gaseous [18F]fluoride source" (A. Pees et al. Chemical communications, Issue 72, 2018) were a gaseous ¹⁸F-synthon is produced in a very fast and efficient manner that can be distilled into a secondary reaction vial were the synthon decomposes to free ¹⁸F- with addition of very low amount of base. This was used to synthesize [¹⁸F]fluoroform in a high molar activity which is not compatible with "standard" highly basic methods.

Another example is described in: "Ring opening of epoxides with [18F]FeF species to produce [18F]fluorohydrin PET imaging agents" (Stefan Verhoog, et al. Chemical communications, Issue 45, 2019) were acidic conditions are used for elution of fluoride from the QMA. The eluted fluorine-18 then forms [¹⁸F]HF which is reacted with Fe(acac)₃ to form a reactive [¹⁸F]FeF species that could be used for a ring opening ¹⁸F-fluorination of sterically hindered epoxide precursors.

However, none of these methods appear to be ideal, since they need special and non-standard precursors/equipment, or are difficult to implement.

Therefore, alternative strategies have been applied to achieve aliphatic ¹⁸F-radiolabelled tetrazines for use in pretargeted PET imaging. One example is published by Outi Keinanen et al. ACS Med. Chem. Lett. 2016, 7, 62-66 in which the radiolabeling of the tetrazine was achieved in high yield, purity, and specific activity under mild reaction conditions via conjugation with pre-labelled deoxyribose (5-[18F]fluoro-5-deoxyribose), providing a glycosylated tetrazine derivative.

Thus, there is still a need for development of labelling strategies for direct aliphatic 18F-labeling of high reactive tetrazines with sufficient yield to be used in PET imaging.

### SUMMARY OF THE INVENTION

Accordingly, in a first aspect, the present invention relates to a method for aliphatic ¹⁸F-labelling of a precursor comprising the steps of:
a) Pre-conditioning an anion exchange cartridge by flushing the cartridge with a solution comprising a non-nucleophilic anion selected from the group comprising phosphate, hydrogen phosphate or dihydrogen phosphate
b) Trapping ¹⁸F ions on the anion exchange cartridge by passing an aqueous ¹⁸F fluoride solution through the anion exchange cartridge
c) Eluting the ¹⁸F ions by using a solution comprising a non-basic anion selected from the group comprising sulfonate esters such as MsO⁻, TsO⁻ or TfO⁻ in combination with a suitable counterion such as Bu₄N⁺ or K⁺/K₂₂₂.
d) Removing the solvent from the eluate from step c) by subjecting the eluate to a drying step
e) Labelling of the precursor molecule with the dried ¹⁸F from step d) in a solvent selected from the group comprising sterically hindered polar protic solvents such as *t*-BuOH, amyl alcohol or Thexyl alcohol or any combination thereof
wherein, the precursor molecule is a tetrazine compound with a reaction kinetic constant in the range of 30.000 M⁻¹ S⁻¹ to 200.000 M⁻¹ S⁻¹ for reacting with unsubstituted TCO measured in PBS at 37 °C and wherein the tetrazine compound comprises at least one aliphatic group comprising a leaving group for nucleophilic substitution.

In a second aspect, the present invention provides a Tetrazine compound, having the formula I: 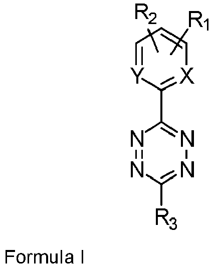
Wherein R1 is H or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
Wherein, R3 is H or 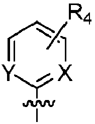 and wherein, X and Y are independently selected from: -CH₂- and -N- and wherein the curly sign indicates the link to the tetrazine,
And wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein, at least one of R2 or R4 is a moiety and wherein R5 is H or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 18F or 19F labelled C1-C6 aliphatic groups comprising fluoromethyl, fluoroethyl, 1-fluoropropyl, 1-fluorobutyl, 1-fluoropentyl, 1-fluorohexyl and wherein the tetrazine compound of formula I has a lipophilicity of cLogD₇.₄ < -0.5

In a third aspect, the invention provides a precursor molecule, having the formula II: 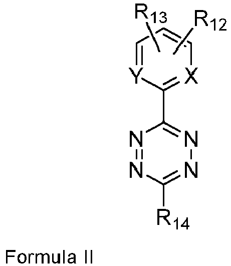
Wherein R12 is H or an C1-C6 aliphatic group such as methyl, ethyl, propyl, butyl, pentyl, hexyl comprising a sulfonate leaving group having any suitable substituent such as triflate, nosylate, mesylate or tosylate and wherein, X and Y are independently selected from: -CH₂- and -N- and
Wherein, R14 is H or 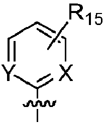 and wherein, X and Y are independently selected from: -CH₂- and -N- and wherein the curly sign indicates the link to the tetrazine,
And wherein R13 and R15 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R16, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein, at least one of R13 or R15 is a moiety and wherein R16 is H or C1-C6 aliphatic group such as methyl, ethyl, propyl, butyl, pentyl, hexyl comprising a sulfonate leaving group having any suitable substituent such as triflate, nosylate, mesylate or tosylate and wherein, at least one of R12 and R16 is an aliphatic group comprising a sulfonate leaving group having any suitable substituent such as triflate, nosylate, mesylate or tosylate and wherein, any functional groups comprising OH, NH or SH are substituted with a protective group selected from the group comprising Boc, Fmoc, CH2C5H5, (CH2)nCH3, C(CH3)3, COCH3, COCF3, C(C5H5)3 and wherein, the precursor molecule has a lipophilicity of ClogD_{7.4} < -0.5 after deprotection.

In a fourth aspect, the present invention relates to use of a Tetrazine compound for PET imaging, wherein said Tetrazine compound, having the formula III: 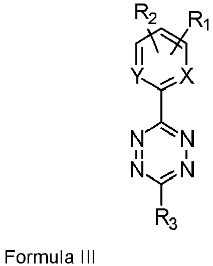
Wherein R1 is a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
Wherein, R3 is H or 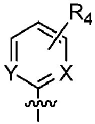 and wherein, X and Y are independently selected from: -CH₂- and -N- and wherein the curly sign indicates the link to the tetrazine,
And wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R5 is H or a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 18F labelled C1-C6 aliphatic groups comprising fluoromethyl, fluoroethyl, 1-fluoropropyl, 1-fluorobutyl, 1-fluoropentyl, 1-fluorohexyl and wherein, the tetrazine compound of formula I has a lipophilicity of cLogD_{7.4} < - 0.5.

In a fifth aspect, the present invention relates to use of a Tetrazine compound according to the second aspect for PET imaging wherein the tetrazine compound is labelled with 18F.

In a sixth aspect, the present invention relates to use of a Tetrazine compound for diagnostics, wherein said Tetrazine compound, having the formula III: 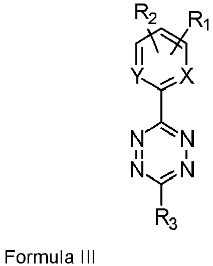
Wherein R1 is a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
Wherein, R3 is H or 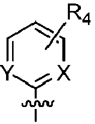 and wherein, X and Y are independently selected from: -CH₂- and -N- and wherein the curly sign indicates the link to the tetrazine,
And wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R5 is H or a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 18F labelled C1-C6 aliphatic groups comprising fluoromethyl, fluoroethyl, 1-fluoropropyl, 1-fluorobutyl, 1-fluoropentyl, 1-fluorohexyl and wherein, the tetrazine compound of formula I has a lipophilicity of cLogD_{7.4} < - 0.5.

In a seventh aspect, the present invention relates to use of a Tetrazine compound according to the second aspect for diagnostics wherein the tetrazine compound is labelled with 18F.

In an eight aspect, the present invention relates to use of a Tetrazine compound for quality control, wherein the Tetrazine compound, having the formula IV: 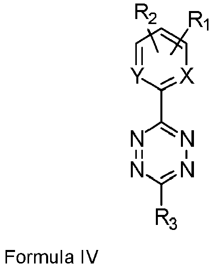
Wherein R1 is a group selected from the group consisting of 19F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
Wherein, R3 is H or 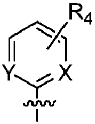 and wherein, X and Y are independently selected from: -CH₂- and -N- and wherein the curly sign indicates the link to the tetrazine,
And wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R5 is H or a group selected from the group consisting of 19F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 19F labelled C1-C6 aliphatic groups comprising fluoromethyl, fluoroethyl, 1-fluoropropyl, 1-fluorobutyl, 1-fluoropentyl, 1-fluorohexyl and wherein, the tetrazine compound of formula I has a lipophilicity of cLogD_{7.4} < - 0.5

In a ninth aspect, the present invention relates to use of a Tetrazine compound according to the second aspect for quality control wherein the tetrazine compound is labelled with 19F.

In a tenth aspect, the present invention relates to use of a precursor molecule for aliphatic ¹⁸F-labelling according to the method described in the first aspect, wherein the precursor molecule, have the formula V: 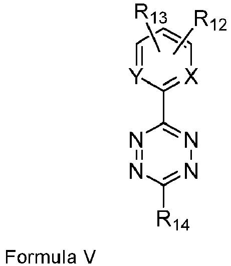
Wherein R12 is an C1-C6 aliphatic group such as methyl, ethyl, propyl, butyl, pentyl, hexyl comprising a sulfonate leaving group having any suitable substituent such as triflate, nosylate, mesylate or tosylate and wherein, X and Y are independently selected from: -CH₂- and -N- and
Wherein, R14 is H or 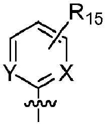 and wherein, X and Y are independently selected from: -CH₂- and -N- and wherein the curly sign indicates the link to the tetrazine,
And wherein R13 and R15 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R16, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R16 is H or C1-C6 aliphatic group such as methyl, ethyl, propyl, butyl, pentyl, hexyl comprising a sulfonate leaving group having any suitable substituent such as triflate, nosylate, mesylate or tosylate and wherein, at least one of R12 and R16 is an aliphatic group comprising a sulfonate leaving group having any suitable substituent such as triflate, nosylate, mesylate or tosylate and wherein, any functional groups comprising OH, NH or SH are substituted with a protective group selected from the group comprising Boc, Fmoc, CH2C5H5, (CH2)nCH3, C(CH3)3, COCH3, COCF3, C(C5H5)3 and wherein the precursor molecule has a lipophilicity of cLogD_{7.4} < -0.5 after deprotection.

In an eleventh aspect, the present invention relates to use of a precursor molecule according to the third aspect for aliphatic ¹⁸F-labelling according to the method described in the first aspect.

### BRIEF DESCRIPTION OF THE DRAWINGS

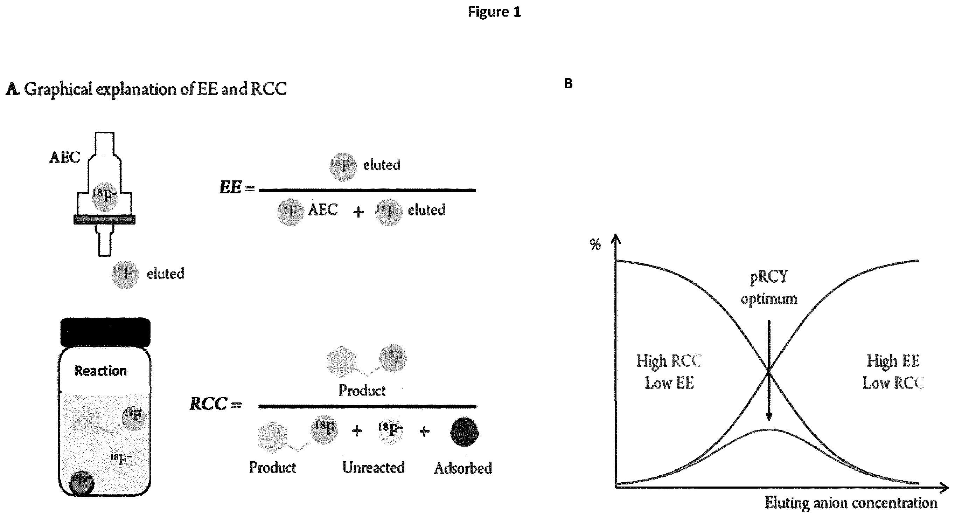

**Figure 1** illustrate the hypothesized relationship between the elution efficiency (EE), the radiochemical conversion (RCC) and the pseudo radiochemical yield (pRCY) for base-sensitive compounds. (**A**) Definition of EE and RCC. RCCs were calculated including resolubilization of ¹⁸F-fluoride, adsorbed to the glass vessel wall. (**B**) Typical dependence of EE and hypothetical dependence of RCC on the eluting anion concentration for base-sensitive compounds. Highest pRCY is a trade-off between the EE (as an indicator of the anion elution concentration) and the RCC, i.e. at an anion concentration resulting in sufficient elution with minimal influence on the base-sensitive reaction.

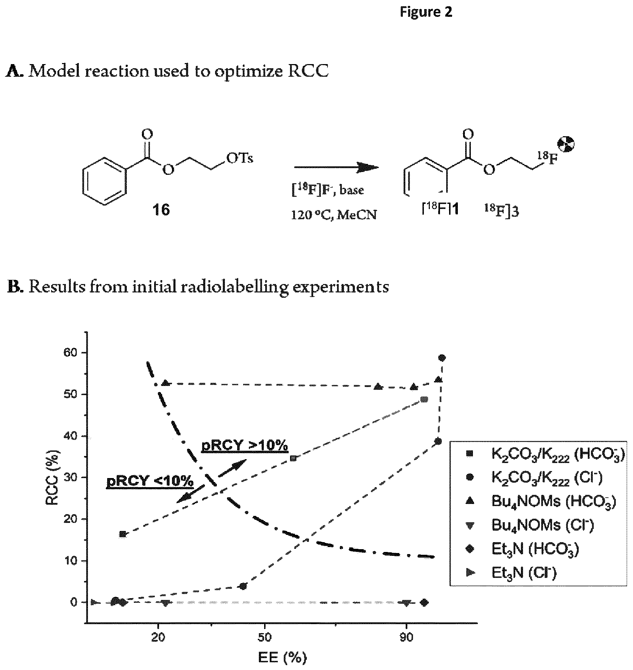

**Figure 2** show Model radiolabeling reaction using precursor **16** to form [¹⁸F]1. (A) Reaction scheme. (B) Results from initial screening of different elution conditions at 120 °C, 5 min in MeCN. Concentration range 2-200 µmol of eluting anion depending on elution efficiency with preconditioning anions in brackets. Higher EE correlates to a higher eluting anion concentration.

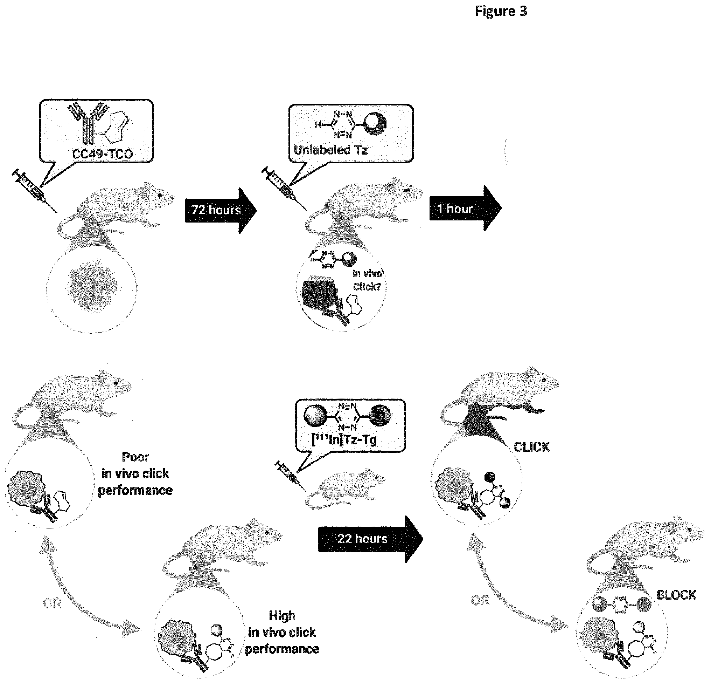

**Figure 3** show a schematic display of the blocking assay. The blocking effect of non-radiolabeled Tz was determined as the change in tumor uptake of [¹¹¹In]Tz-Tg 22 h p.i. Each Tz was administered 1 h prior to [¹¹¹In]Tz-Tg and the uptake normalized to a group of animals were no blocking was performed (control).

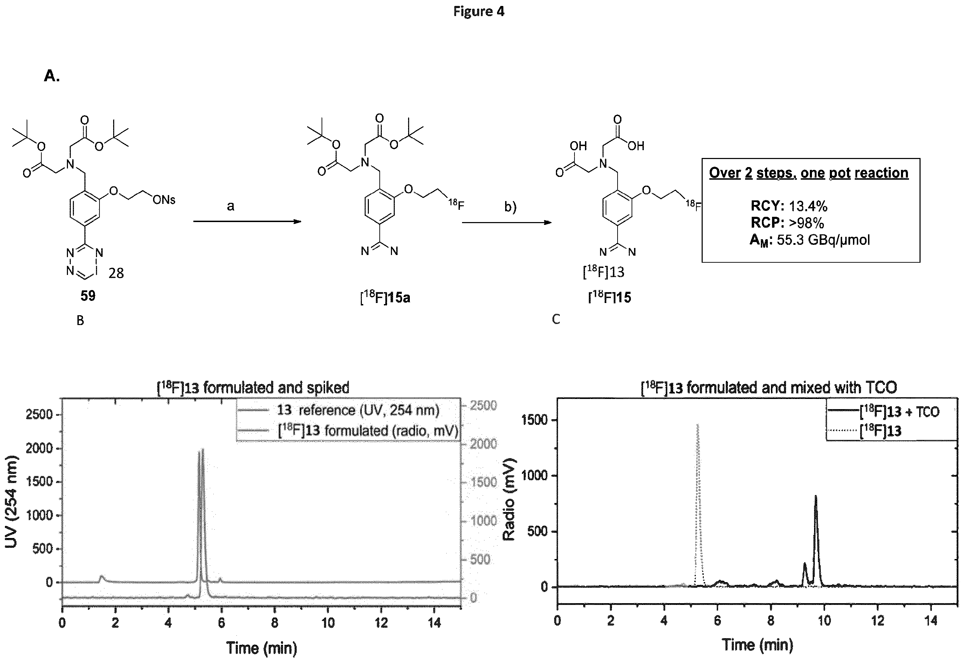

**Figure 4** show the Radiolabeling of [¹⁸F]13. A) Reaction sequence. *Reagents and conditions:* a) [¹⁸F]Bu₄NF/Bu₄NOMs PO₄³⁻, *t*-BuOH/DMSO, 100 °C, 5 min; b) TFA, CH₃CN, 80 °C, 10 min. B) Analytical chromatograms of formulated [¹⁸F]13 spiked with non-radioactive 13 for identification (rt: 5.29 min). C) Analytical chromatogram of the reaction between [¹⁸F]13 shows that the tetrazine core of is intact. Analytical chromatogram of [¹⁸F]13 before TCO-PNP addition (red) and after TCO-PNP addition (black).

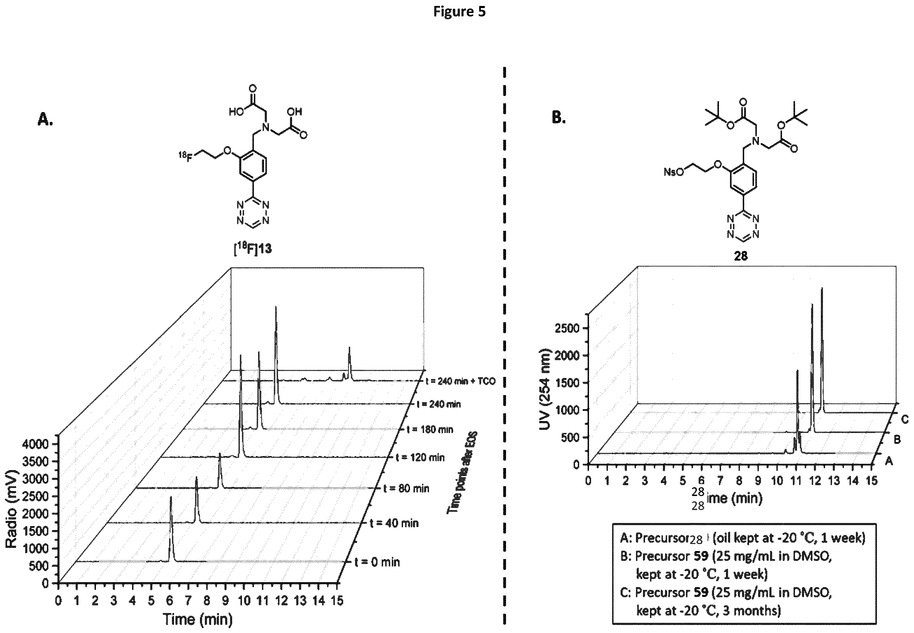

**Figure 5** (A) Stability of [¹⁸F]13 at r.t. over 4 hours. After 4 hours, no signs of decomposition were detected and [¹⁸F]13 was still able to react quantitatively with TCO-PNP. (B) Stability of the precursor 28 as a pure oil at rt, at - 20 °C or dissolved (25 mg/mL) in DMSO and frozen at -20 °C. DMSO frozen samples were stable over the course of at least 3 months.

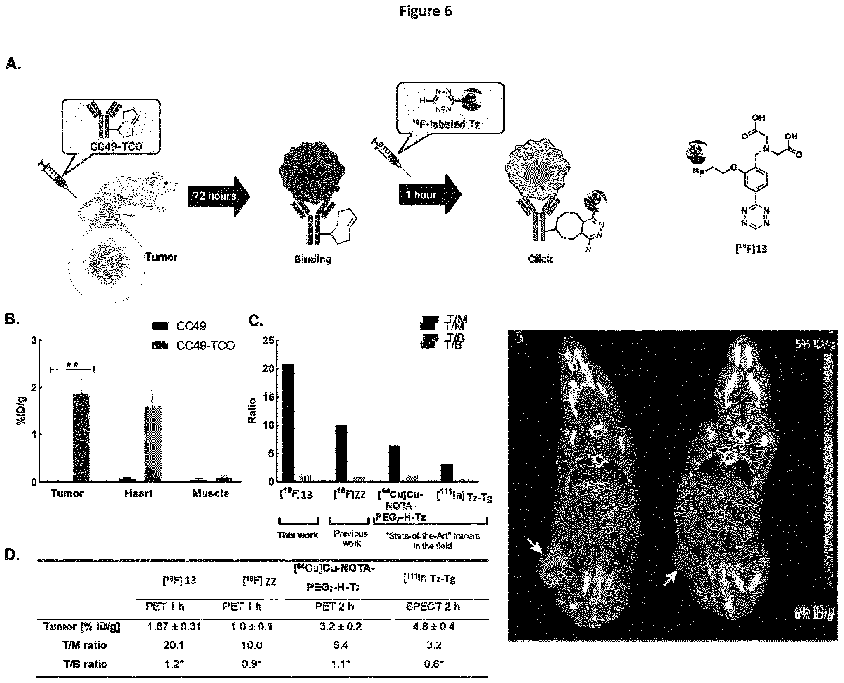

**Figure 6****.** PET/CT scan of CC49-TCO pretargeted [¹⁸F]**13** in LS174T tumor xenograft bearing mice. **A.** General pretargeted imaging approach. **B**. PET-Image derived mean %ID/g in tumor-, heart- and muscle tissue 1 h p.i. of [¹⁸F]**13**. mean ± S.E.M, n = 4/group. *p < 0.05 (Welch's t-test) (**C**. & **D**.) Image-derived tumor uptake (mean % ID/g), Tumor-to-muscle (T/M) and Tumor-to-Blood ratio (T/B) of [¹⁸F]**13** in comparison with "state-of-the-art" applied Tz imaging agents [¹⁸F]1 (PET 1 h p.i,., *n* = 3), **[⁶⁴Cu]Cu-NOTA-PEG₇-H-Tz** (PET 2 h p.i,., *n* = 4) and [¹¹¹ln]Tz-Tg (SPECT 2 h p.i., *n* = 4). Tumor uptake and ratios of [¹⁸F]**ZZ** ([¹⁸F]2,2'-((3-fluoro-5-(1,2,4,5-tetrazin-3-yl)benzyl)azanediyl)diacetic acid), **[⁶⁴Cu]Cu-NOTA-PEG7-H-Tz** and [¹¹¹In] Tz-Tg 2 h p.i. in nude BALB/c mice bearing subcutaneous LS174T tumor xenografts pretreated with CC40-TCO (100 µg) has recently been published. Data are shown as mean ± standard error of mean (SEM). *Image-derived uptake in heart from SPECT and PET images used as a surrogate for blood. **E.** Representative images from PET/CT-scans 1 h p.i. of [¹⁸F]**13**. Mice were administered with either non-modified CC49 (left) or CC49-TCO (right), 72 h prior to [¹⁸F]**13** injection. Arrows indicate LS174T tumor xenografts. Scale bar indicates mean %ID/g.

### DETAILED DESCRIPTION OF THE INVENTION

The method according to the first aspect of the invention provide the possibility to radiolabel base sensitive structures with significantly improved RCYs. Even structures that were previously not accessible by applying "standard" aliphatic ¹⁸F-labeling strategies could be radiolabeled. The developed methodology can easily be implemented on all synthesis modules and is only dependent on the preconditioning of the anion exchange cartridge, its non-basic elution and in the selection of the right reaction solvent. This places new classes of ¹⁸F-fluorinated compounds within reach using classical labeling approaches (S_{N}2 labeling).

The extensive work forming basis for the method according to the first aspect of the invention is described in details in the following.

The radiochemical yield (RCY) of a labeling procedure is a measure of the proportion of decay corrected and isolated product, with respect to the starting radioactivity. Consequently, all steps of a labeling procedure contribute to the RCY.
The RCY of an S_{N}2 ¹⁸F-fluorination is primarily determined by two factors:
1) The elution efficiency (EE) of the trapped [18F]fluoride and
2) the reaction efficiency (radiochemical conversion, RCC) (Figure 1).
In order to study the influence of preconditioning and elution conditions on the RCY, it was decided to investigate the EE and the RCC independently. To approximate the expected efficiency of the whole labeling procedure (RCY), a theoretical measure, named the pseudo radiochemical yield (pRCY) was defined.
In the context of the present invention, pRCY is defined as: pRCY = EE * RCC
This measure was used to evaluate the applied labeling conditions.

Initially, a broad set of different elution conditions were screened (>500 experiments) with the aim to identify a sufficient EE that simultaneously resulted in a low basicity eluate.
For simplicity, only the commonly used Sep-Pak Accell Plus QMA Plus Light Cartridge (130 mg resin loading) anion exchange cartridge (AEC) was investigated. The Sep-Pak Accell Plus QMA Plus Light Cartridge contain 130 mg silica-based, hydrophilic, strong anion-exchanger with a pore size of 300 Ångstöm and a particle size of 37-55µm and has an ion exchange capacity of 230µeq/gram and a hold up volume of 0.4 mL. However, any suitable anion exchange cartridge may be used for aliphatic ¹⁸F-radiolabeling according to the present invention and the skilled person will know how to adjust the volumes of the method steps relative to the specific anion exchange cartridge chosen.

Different preconditioning anions influence the EE, as this could be a major contributing factor in subsequent fluorinations (Figure 2A). It was decided to precondition cartridges with relatively non-basic Cl- and HCO3- anions. As elution solvents, water and two different MeCN/H2O mixtures one having a MeCN/H2O ration of 50/50 and one having a MeCN/H2O ration of 90/10 were studied. These elution solvents were chosen to find the best compromise between the better EEs of a higher water content and the considerably shorter azeotropic distillation process associated with a lower water content.
In all experiments, cyclotron produced aqueous [¹⁸F]fluoride was quantitatively trapped. The concentrations resulting in an EE of 90% were calculated by fitting the Hill equation to the data (see example 1). The inventors decided to use this value as the initial activity loss during the trapping and elution step should be minimized to ≤10%.
Various types and concentrations of eluting anions were screened to identify minimal concentrations. In addition to commonly applied eluting anions such as carbonates, bicarbonates or oxalates, also organic bases such as DBU, Et3N, DIPEA and DMAP were investigated. These bases deprotonate water molecules, forming OH- anions in situ, which displace [18F]fluoride from AECs. During the subsequent drying procedure, bases are removed through distillation, resulting in low basicity of the reaction mixture. The inventors also investigated a range of neutral salts as eluting anions. In all cases, the EE showed a sigmoidal curve progression with a sharp decrease at a specific concentration depending on the preconditioning of the AECs and the eluting anions (Figure 1B). Bicarbonate preconditioned AECs generally required lower eluting anion concentrations compared to chloride preconditioned AECs. This effect is driven by the weaker interaction of chloride with the quaternary methyl groups of the resin compared to bicarbonates. As expected, the EE was higher for solvent mixtures containing more water. This improvement in EE was especially pronounced for organic bases, as higher water concentrations promoted in situ formation of OH- ions. The most efficient eluting anions were bivalent "standard reagent" anions (K2CO3/K222 and K2C2O4/18C6) and "in situ formed OH--anions" with organic bases (DBU and DIPEA) in higher water concentrations, whereas the neutral salts generally required higher concentrations of anions. For this reason, it was decided to study the influence of eluting conditions on the RCC and used them together with the EE to determine pRCYs. A model reaction was chosen for this purpose that was not particularly base sensitive, but allowed for fast and efficient screening (Figure 2A).
The inventors hypothesized that the lowest acceptable base concentration that resulted in reasonable pRCYs of this reaction would allow them to decide on which conditions to test with base-sensitive reactions. A pRCY of 10% was defined as the lowest acceptable limit. This limit was set since it would theoretically allow isolation of 375 MBq final product from 5 GBq of starting activity with a 45 min synthesis time taken into account. This starting amount is accessible even at radiopharmaceutical centers without direct access to a cyclotron and that are dependent on ¹⁸F-deliveries. The radioactivity amount used for a single human PET scan is approximately 300 MBq and as such, 375 MBq of labeled tracer is sufficient as a lower limit for this purpose. To reduce the number of experiments, it was decided to determine the pRCY on elution conditions that result in an EE of 20, 50, 90 and ∼100%. Based on example 1, it was further decided to test only elutions based on a 50:50 MeCN/H2O mixture. This decision is a compromise between the diminishing EE observed with a 90:10 mixture and the prolonged drying procedure (∼30 min compared to 10-15 min) when pure water was used.

### Initial radiolabeling screen using a model reaction.

In order to determine the trade-off between the EE and the RCC, reactions were carried out to determine the minimal anion (base) concentration needed to obtain a pRCY of >10% for our model compound (Figure 2A). The anion concentration was varied, one eluting reagent from each category chosen - namely K2CO3/K222 for standard reagents, Et3N for organic bases and Bu4NOMs for neutral salts - and two preconditioning anions (HCO3- or Cl-) were tested for each combination. The results are summarized in Figure 3B. No trade-off between the EE (as an indicator of the anion elution concentration) and the RCC could be identified for any of the reactions see example 2 and figure 2B. For QMAs preconditioned with HCO3- and eluted with K2CO3/K222, a linear dependency between the EE and the RCC was observed (Figure 2B). Lower eluting anion concentration, and subsequent lower EE, was accompanied by lower RCC. This can be explained by the increased capability of the vessel's glass wall to adsorb [¹⁸F]fluoride when the (bi)carbonate concentration in the eluate decreases. Adsorbed [¹⁸F]fluoride is not accessible for labeling reactions and consequently, the RCC drops. A pRCY of >10% could be achieved using >3 mM K2CO3/K222 (interpolated from the elution curves, Figure 2B). Therefore this should be the minimal elution concentration as a starting-point to explore if base sensitive structures can be labeled using HCO3- preconditioned QMAs and the according K2CO3/K222 elution mixture. Lower K2CO3/K222 concentrations would not be expected to result in acceptable RCYs for base sensitive substrates. Consequently, ¹⁸F-labeling attempts would be futile if they cannot withstand 3 mM of HCO3-. QMA cartridges preconditioned with Cl- and eluted with K2CO3/K222 showed a similar trend. However, the RCC was further reduced in an exponential fashion with lower EE. This decrease stems from the capacity of the cationic QMA resin to adsorb (bi)carbonate ions. At lower concentrations, no or very little amounts of (bi)carbonate ions can pass through the QMA and are as such not available in the eluate to promote the 18F-fluorination.

In contrast, Cl- ions from the Cl- preconditioned QMA are released from the resin during the elution process leading to competing chlorination and thus reducing the RCC further (Figure 2B). Elutions using Et3N or other organic bases such as DBU or DIPEA resulted in loss of 18F-activity (5-50%) during azeotropic distillation of the eluate and no ¹⁸F-incorporation of the remaining activity into the precursor was observed (Figure 2B). This indicates that the organic bases do not generate conditions that are basic enough to promote SN2 fluorinations.

Surprisingly, elution of the HCO3- preconditioned QMA using Bu4NOMs resulted in stable RCCs of around 50% independent of the elution anion concentration. pRCY >10% could be reached for all tested conditions. Since ¹⁸F-fluorination requires base and the OMs⁻ eluting anion is non-basic, the basicity must stem from the HCO3-preconditioning anion that co-elutes with the [¹⁸F]fluoride when eluting the QMA with Bu4NOMs. No product was formed using the same conditions but preconditioning with the non-basic anions: OMs⁻ and SO42⁻. This prompted us to investigate further how preconditioning of the QMA cartridge combined with neutral elution could promote high pRCYs for low-base conditions.

### Investigating the role of the preconditioning anion.

As described above, our data suggest that it is possible to utilize the basicity of the QMA cartridge preconditioning anion to promote 18F-fluorinations when using non-basic salts for an efficient elution process. This combination could be used to minimize the base concentration in the reaction and protect base-sensitive precursors/tracers against degradation or to reduce base-promoted site-reactions. Therefore, it was decided to test a number of preconditioning anions in combination with Bu4NOMs elution to determine their influence on the EE, RCC and ultimately, the pRCY (Example 2). Interestingly, the EE was mainly dependent on the valency of the preconditioning anion rather than the pKa, with a higher valency increasing the EE (see example 2, Table 2). Nucleophilic preconditioning anions such as C₂O₄²⁻, AcO- or Cl- should be avoided as they lower the RCC by outcompeting the [¹⁸F]fluoride nucleophile, as confirmed by LC-MS analysis. As for any ¹⁸F-fluorination, a certain basicity of the preconditioning anion is needed to promote the reaction. The inventors learned that the reaction could proceed if preconditioning anions with a pKa of around 4 were used. For univalent preconditioning anions, a higher pKa resulted in a higher EE. This observation follows the electroselectivity theory which is based on the Donnan potential. It allows to determine the electroselectivity of anions in heterogeneous systems, i.e. the selectivity coefficient between ions in solution and bound to the resin. For anions of the same valency at low concentrations, the dominating factor for the affinity to the resin is the Debye-Huckel activity coefficient which in turn is proportional to the pKa, i.e. compounds with higher pKa values bind stronger to the resin. As such, preconditioning anions with a higher pKa than the fluoride ion facilitate elution of [¹⁸F]fluoride from the QMA cartridge, since eluting anions can more easily displace fluoride from the resin compared to the more strongly bound preconditioning anions.

### Quantifying the breakthrough of precondition anions.

Given that the amount of base in the reaction mixture is determined by the EE of the preconditioning anion when non-basic elution approaches are used, a precise quantification of the amount of preconditioning anion that is eluted into the reaction vessel would allow us to understand more thoroughly how these anions affect ¹⁸F-fluorinations, especially for base-sensitive structures. In order to quantify the breakthrough of the preconditioning anions from the QMA cartridge, we estimated their concentration in the eluate (i) by pH measurements and (ii) by quantitative NMR (qNMR). In general, qNMR measurements provided higher precision than pH measurements, but could only be applied to the monovalent anions, HCO₃-, H₂PO⁴⁻ and OMs⁻. Respective quantifications showed that the monovalent HCO₃⁻ preconditioning anion was proportionally displaced by OMs-, whereas the di- and trivalent CO₃²⁻ and PO₄³⁻ showed only minor displacement, even with high concentrations of OMs-. This observation can be explained by the Donnan potential. Due to their multiple charge, multivalent anions interact with the cationic groups on the anion-exchange resin more strongly than monovalent anions. This effect is stronger than the one promoted by the pKa-dependent Debye-Huckel activity effect. Finally, qNMR results also showed that the more acidic H2PO4- anion (pKa: 2.14 in H2O) remained in its di-protonated form after it was eluted from the QMA. As such, it is able to reduce the basicity of the reaction mixture. However, the mixture remains basic enough to promote the 18F-labeling step.

### Improved resolubilization of [¹⁸F]fluoride using Bu₄NOMs as eluting anions.

Adsorption of [¹⁸F]fluoride on the wall of glass reaction vessels is a commonly observed phenomenon reducing RCCs under low basicity conditions. In comparison to standard systems using cryptands such as [¹⁸F]KF/K222, tetraalkylammonium[18F]fluoride is more lipophilic (cLogD7.4 calculated with Chemicalize software for Bu4NF is 1.32 and for the KF/K222 -0.41). Consequently, the solubility of such salts is higher in organic, polar aprotic solvents which are commonly used for fluorinations. For example, the use Bu4NOMs resulted in 10% less glass absorption as compared to using the corresponding K+/K222-mixture . As a result, [18F]fluoride adsorption to glass walls is minimized and the amount available in the reaction solution increased. To further explore the potential of tetraalkylammonium salts in respect to reaction basicity and to increase the resolubilization process of [18F]fluoride, three additional salts (Bu₄NOTf, Bu₄NH₂PO₄ and Et₄NHCO₃) with different physicochemical properties were studied. Bu₄NOTf was chosen because the lower pKa of OTf compared to the OMs⁻ of Bu₄NOMs should displace lower amounts of preconditioning anions during the elution process, result in a less basic eluate and enable therefore labeling tracers under milder reaction conditions. Bu₄NH₂PO₄ was chosen due to the buffering capabilities of Bu₄NH₂PO₄. This salt should neutralize more basic preconditioning anions. Finally, Et₄NHCO₃ was chosen because it is commonly used for elution in nucleophilic ¹⁸F-radiolabeling, and is used as a comparison.

Furthermore, it was decided to study the influence of these tetraalkylammonium salts, in combination with the most promising preconditioning anions (carbonate, bicarbonate, phosphate and hydrogen phosphate) that were identified in the preconditioning screening (Example 1) and three solvents (DMSO, MeCN and tBuOH) which are commonly used solvents for aliphatic 18F-fluorinations. The selection of solvents was based on their different ability to act as hydrogen bond donors (HBD) and/or hydrogen bond acceptors (HBA) i.e. DMSO being aprotic and protophilic and having no HBD or HBA abilities, MeCN being aprotic and protophobic and having no HBD and very weak HBA abilities and finaly, tBuOH (mixed with 17%v/v MeCN to make it liquid at room temperature) being protic and amphiprotic and having both HBD and HBA properties. These factors can affect the solubility of the anions, thus influence the basicity and thus RCYs when labeling base-sensitive structures.

### Multiparametric radiolabeling screen using selected preconditioning anions and elution reagents.

All possible combinations of preconditioning anions, eluting reagents and reaction solvent were tested (Example 3). The non-basic eluting anions OMs- and OTf- resulted in the highest pRCY in combination with multi-charged preconditioning anions, especially phosphates. These conditions led to very low preconditioning anion breakthrough and consequently lower base concentration in the eluate. This resulted in surprisingly high pRCYs while retaining high amounts of intact precursor **(16)** from figure 2A. H₂PO⁴⁻, as an acidic eluting anion, only resulted in good pRCYs when applied with carbonate or bicarbonate preconditioning anions. As indicated by the qNMR experiments (described above), H₂PO⁴⁻ can lower the basicity of the carbonates and act as a buffer. Consequently, increasing the concentration of H2PO4- from 20 mM to ≥50 mM diminished the pRCY with the (bi)carbonate preconditioning anions as the eluate became too acidic to promote 18F-labeling. This could explain the inconsistent pRCYs of the same elution of the phosphate preconditioned QMAs considering the possible variations in elution of the basic preconditioning anion (PO43-) at this concentration range. The balance of acidic elution and basic preconditioning is regulated for the carbonates by H2CO3 formation escaping as CO2. However, for phosphate preconditioning all acidity from the elution remains in the eluate. This could probably be optimized with lower concentrations of Bu4H2PO4 but then at the expense of a lower EE. Finally, the more frequently used elution reagent Et4NHCO3 resulted, as expected for base-insensitive precursors/tracers, in good pRCY for all preconditioning anions, comparable with the aforementioned high yielding elution conditions. Surprisingly, the use of tBuOH/MeCN only resulted in relatively low RCC for all tested elution conditions. However, the amount of intact precursor at the end of the reaction was significantly higher compared to reactions using MeCN or DMSO and otherwise identical conditions. This indicates that use of tBuOH in the solvent could be beneficial for very base-sensitive substrates, since the resulting mild labeling conditions to less degradation of the precursor/tracer, but at the expense of less efficient ¹⁸F-incorporation.

### Reaction time, temperature, precursor concentration and leaving groups.

It is known that the reaction time, temperature, precursor concentration as well as the chosen leaving group have a strong - but structure dependent - influence on RCYs. Therefore, it was decided not to optimize these parameters for the model reaction (figure 2A) this should be investigated for individual syntheses.

### Improving the labeling procedures of known radiopharmaceuticals/synthons.

Next, the findings from Example 3 were applied to the synthesis of a set of well-described PET tracers and radiolabeled building blocks to increase the RCYs of those structures. Selection of compounds to be studied were based on the following two criteria for:
I) Selected structures should possess a reported RCY <50% and more importantly
II) base-insensitive and base-sensitive structures should be included to study the beneficial effect of the identified conditions.

In addition a broad set of structural motifs were covered which could be affected by a basic environment (see example 4). Preconditioning and elution conditions were selected on a rational analysis or by reported data of the base-sensitivity of compounds to be labeled and selected from example 3.
First, four relatively base-insensitive tracers were tested. The inventors hypothesized that even these structures could benefit from elution with tetraalkylammonium salts in respect to increasing the ¹⁸F-resolubilization from the glass wall into the reaction solvent. [¹⁸F]FETO, [¹⁸F]FTC-146, [¹⁸F]F-PEG3-N3 and [¹⁸F]FE-TCO have been reported to be stable under "standard" basic labeling conditions. No degradation adducts were observed using those conditions. The inventors assumed that eluting a QMA which was preconditioned with HCO3- or the slightly more basic CO₃²⁻ (conditions 1C or 3C, from example 3) with tetralkylammonium salts would result in higher ¹⁸F-resolubilization while simultaneously the preconditioning anion would provide enough basicity to promote the labeling step. For all four compounds an increased isolated RCY was achieved spanning from approximately 40% to 170% increase using the optimized conditions (se example 4). Retrospective analysis of the ¹⁸F resolubilization data showed that this parameter was indeed in all reaction increased and significant contributed to the improvement RCY (10 - 30% of the observed increase). One additional factor that might have improved the yields is the lower base content used. This condition rather favors S_{N}2 labeling over E2 elimination - a possible side-reaction, which is typically facilitated at higher base concentrations.

The first relatively base-sensitive structure that was investigated was [¹⁸F]FTHA. This compound is labeled at a secondary carbon atom and thus, is more prone to undergo E2 elimination, especially under strongly basic conditions. The inventors hypothesized that less basic conditions should consequently lead to a high RCY. Preconditioning with PO₄³⁻ and using Bu₄OMS for elution resulted in the lowest basicity of the eluent (conditions 4A, example 3). Applying these conditions doubled the isolated RCY compared to the reference procedure using "standard" conditions (example 4).

The next compound tested was a building block which can be used to label a broad set of radiopharmaceuticals. [18F](2R,3R,4S,5R,6R)-2-Azido-6-(fluoromethyl)tetrahydro-2H-pyran-3,4,5-triyl triacetate ([18F]Sugarazide) is labeled via a two-step labeling procedure. First, a hydroxy-group protected precursor is ¹⁸F-labeled and then deprotected. The acetyl protection groups are base-labile. In the reported labeling procedure, partial hydrolysis of those protecting groups occurred using "standard" basic labeling conditions. Free hydroxy groups typically form H-bonds with ¹⁸F- and reduce its nucleophilicity, thus decreasing RCYs. The low basicity conditions using a PO43-preconditioned QMA and Bu4NOTf (condition 4B, example 3) for elution were applied in order to reduce the basicity and consequently reduce premature deprotection. No deprotection was observed using these conditions and the isolated RCY increased approximately two-fold to 41.8±7.8% (example 4).

Finally, [18F]FE-PE2l was tested. This tracer is regularly produced for clinical applications. The complex cocaine-scaffold along with a vinylic iodine has been shown to degrade in the reaction crude, presumably due to basic conditions. To investigate if lower basicity leads to higher RCYs, the low basicity conditions applying a PO43-preconditioned QMA and Bu4NOMs for elution (condition 4A, example 3) were applied. The final isolated RCY was increased by over 150% using this set-up (example 4).

### Labeling of base-sensitive structures that are not accessible via "standard" aliphatic ¹⁸F-labeling conditions.

The inventors fund that the mildest labeling conditions (condition 4A, example 3) in combination with a tBuOH-mixture could provide sufficiently low basicity labeling conditions to label a H-Tz. Initial attempts using a mesylate precursor resulted in an increase from traces of labeled product to a pRCY of approximately 2% of [18F]**2** (example 5).
In a next step, the influence of different leaving groups was investigated. In addition to the mesylate (OMs) group, tosylate (OTs) and nosylate (ONs)-based precursors were tested (example 5). As mentioned previously, different leaving groups can influence the labeling yield substantially, but no trend with respect to increased RCY has been observed, and the yields varied by case-by-case scenario depending on the individual molecular structure of the precursor.

To facilitate the reaction further, the solvent was changed to tBuOH mixed with DMSO which decreased the evaporation of solvent during automated synthesis while maintaining the RCC (example 5). The nosylate precursor with tBuOH/DMSO and the low basicity elution condition (condition 4A, example 3) resulted in a RCY of approximately 22% (Figure 4). Control experiments were also carried out to investigate if low basicity conditions (4A in combination with a tBuOH solvent) are needed to promote the reaction.

These experiments yielded in no or only trace amount of the 18F-labeled Tz ([18F]**2** having a reactivity 59842 M-1s-1 for [18F]**2)** (example 5). In order to test the applicability of the identified conditions to label H-Tzs, we decided to radiolabel an even more reactive Tz. The chosen structure displays a 2-fold increased reactivity towards TCO (134855 M-1s-1 for [18F]**3**) and should as such be even more difficult to label, since reactivity is proportional to the Tz's base stability.
In line with the aforementioned observations, the more reactive Tz ([18F]**3**) could only be radiolabeled using the mildest labeling conditions. As expected, the compound could be isolated from the ONs precursor in a lower RCY (ca. 5% RCY) than the less reactive Tz. This reflects the higher base sensitivity of the structure.

By carefully studying the key parameters involved in the trapping of [18F]fluoride on an anion exchange cartridge and its subsequent elution, we were able to identify conditions that result in low basicity elutions. These conditions made it possible to radiolabel base sensitive structures with significantly improved RCYs. Even structures that were previously not accessible by applying "standard" aliphatic 18F-labeling strategies could be radiolabeled. The developed methodology can easily be implemented on all synthesis modules and is only dependent on the preconditioning of the anion exchange cartridge, its non-basic elution and in the selection of the right reaction solvent. This places new classes of 18F-fluorinated compounds within reach using classical labeling approaches (SN2 labeling).

Thus, in the first aspect, the invention relates to a method for aliphatic ¹⁸F-labelling of a precursor comprising the steps of:
a) Pre-conditioning an anion exchange cartridge by flushing the cartridge with a solution comprising a non-nucleophilic anion selected from the group comprising phosphate, hydrogen phosphate or dihydrogen phosphate
b) Trapping ¹⁸F ions on the anion exchange cartridge by passing an aqueous ¹⁸F fluoride solution through the anion exchange cartridge
c) Eluting the ¹⁸F ions by using a solution comprising a non-basic anion selected from the group comprising sulfonate esters such as MsO⁻, TsO⁻ or TfO⁻ in combination with a suitable counterion such as Bu₄N⁺ or K⁺/K₂₂₂.
d) Removing the solvent from the eluate from step c) by subjecting the eluate to a drying step
e) Labelling of the precursor molecule with the dried ¹⁸F from step d) in a solvent selected from the group comprising sterically hindered polar protic solvents such as *t*-BuOH, amyl alcohol or Thexyl alcohol or any combination thereof
wherein, the precursor molecule is a tetrazine compound with a reaction kinetic constant in the range of 30.000 M⁻¹ S⁻¹ to 200.000 M⁻¹ S⁻¹ for reacting with unsubstituted TCO measured in PBS at 37 °C and wherein the tetrazine compound comprises at least one aliphatic group comprising a leaving group for nucleophilic substitution Preferably, the non-nucleophilic anion of the preconditioning in step a) is selected from the group consisting of phosphate, hydrogen phosphate or dihydrogen phosphate.

The combination of a specific anion for preconditioning (i.e. the non-nucleophilic anion of step a)) of the anion exchange cartridge and the non-basic anion selected for the elution in step c) greately affect both the yield of ¹⁸F and the conditions under which the precursor is labelled in step e). Thus, preferably, the non-basic anion of step c) is selected from the group consisting of the tetraalkylammonium salts; Bu₄NH₂PO₄, Bu₄NOMs and Bu₄NOTf. As described above, the higher lipophillizity of the Bu4N-cation is more easily soluble in the solvents used for ¹⁸F labeling. The Bu4N-salts have a lower melting point that makes them liquid at the drying temperature, which facilitates dissolvning of the ¹⁸F in the ionic liquid rather than percipitting the salt on the glass surface. This also lowers the surface that the activity covers which lowers glass adsorption.

The solvent chosen for dissolving the non-basic anion in step c) affect the solubility of both the preconditioning anions (i.e. the non-nucleophilic anion of step a)) and the elution anions (i.e. the non-basic anion of step c)) and their properties in the solvent such as their basicity and thereby influence the content of both anions as well as ¹⁸F in the eluate resulting from step c) greately impacting on the labelling conditions achieved in step e) and thereby the resulting RCY. Thus, preferably, the non-basic anion of step c) is dissolved in MeCN/H₂O, MeOH, EtOH or corresponding water mixture to form a non-basic anion solution.

In a preferred embodiment in line with the above, the non-nucleophilic anion of the preconditioning in step a) is selected from the group consisting of phosphate,hydrogen phosphate or dihydrogen phosphate and the non-basic anion of step c) is selected from the group consisting of the tetraalkylammonium salts; Bu₄NH₂PO₄, Bu₄NOMs and Bu₄NOTf and is dissolved in MeCN/H₂O, MeOH, EtOH or corresponding water mixture to form a non-basic anion solution.

The concentration of the preconditioning anion in solution may be any concentration sufficiently high for the anion exchange cartridge to be saturated with the preconditioning ion during flushing of the anion exchange cartridge. Any excess ions will be removed by washing of the cartridge before trapping of the ¹⁸F ions on the cartridge. Thus, the concentration of the preconditionin anion in solution may be in the range of 0µmol to 20µm, such as 1 µmol to 20µmol, preferrably 5µmol to 20µmol, more preferred 10µmol to 20µmol.

The concentration of the elution anion in the non-basic anion solution influences the effeciency of ¹⁸F elution from the cartridge as well as the composition of the eluate resulting from the elution step c) since excess anions will remain in solution and form part of the eluate. Thus, preferably, the non-basic anion solution has a concentration of non-basic anion in the range of 10-100mM, such as 10-50mM, preferably 15-40mM, more preferably 20-30mM and most preferably 20mM.

In the context of the present invention, removing the solvent from the eluate is to be understood as removing sufficient of the solvent of the eluate to achieve dryness. The skilled person will be familiar with the meaning of drying an eluate to dryness. Any residual solvent, in particular water, might impact the subsequent labelling step negatively, and it is therefore important to subject the eluate to a drying step for a periode of time sufficient to remove as much of the elution solvent as possible in a time efficient manner.
Drying of the eluate in step d) may be done by any suitable drying method known to the skilled person, such as heating the reaction to 80-100C under reduced pressure and/or inert gas-flow for 2-15 min depending on the solvent and volume used, preferably by heating the reaction to 100 degree celsius under reduced pressure and N2 flow for 5-7min wherein 1 ml MeOH was used as elution solvent. The skilled person will know, that the drying conditions such as the time needed to achieve dryness will depend on several factors, such as the nature of the solvent and volume used for the elution step.

The solvent used in the labelling step e) was found to impact the labelling reaction effeciency i.e. the radiochemical conversion (RCC).
Polar protic solvents used like tBuOH was found to be a mild solvent as a lot of remaining precursor was observed by HPLC analysis at the end of the reaction. However, using these solvents is limited to moderate RCCs in our hands. Polar protic solvents in this context is a solvent that contains an OH-group or similar that can donate H-bonds to the precursor, base and/or the fluoride. Although, these solvents should be sterically hindered to prevent nucleophilic attacks from the OH-group that would be detrimental to the precursor.

Thus, the sterically hindered polar protic solvent is preferably selected from the group consiting of sterically hindered propanol, butanol, pentanol or hexanol such as tBuOH, amyl- or thexyl alcohol.

Polar aprotic solvent is commonly used in radiochemistry such as MeCN or DMSO. These solvents provide high RCC but are harsh towards the precursor as lower concentrations are observed with HPLC analysis at the end of the reaction. Polar aprotic solvents don't contain any free OH-groups and can thus not donate H-bonds to the precursor, base and/or fluride e.g. ¹⁸F. The polar aprotic solvent is preferably selected from the group consisting of DMSO, MeCN, DMF, NMP or DMA.

It was found that a combination of a polar protic solvent and a polar aprotic solvent provided the mild conditions that were observed with only tBuOH. However, the solubility of different precursors is limited in pure tBuOH why dissolving the precursor in DMSO first and then adding it to tBuOH solved this problem (no pun intended). tBuOH is also solid at room temperature which is very impractical when setting up automated syntheses. By adding MeCN or DMSO the tBuOH becomes liquid at room temperature. The boiling point for tBuOH is 82C which results in a reduction of the solvent volume during the reaction which lowers the yield. Mixing it with MeCN did not solve this problem but mixing it with DMSO resulted in a higher boiling point which makes reactions at 100C suitable.

Thus, in a preferred embodiment, the labelling of the precursor molecule in step e) is performed in a solvent mixture comprising at least one sterically hindered polar protic solvent such as *t*-BuOH, amyl alcohol or Thexyl alcohol in combination with at least one polar aprotic solvent such as DMSO, MeCN, DMF, NMP or DMA preferably in a polar protic/polar aprotic ratio in the range of 10/90 to 100/0, such as in the range of 20/80 to 90/10, or in the range of 30/70 to 85/15, preferred in the range of 40/60 to 85/15, more preferred in the range of 50/50 to 85/15, yet more preferred in the range of 60/40 to 85/15, even more preferred in the range of 70/30 to 85/15, most preferred 83/17. In a most preferred embodiment the labelling of the precursor molecule in step e) is performed in a solvent mixture consisting of tBuOH and DMSO in a ratio of 83/17.

The method according to the present invention allow for aliphatic ¹⁸F labelling of high reactive tetrazine compounds in the form of a precursor molecule comprising a suitable leaving group for nucleophilic substitution, preferably, a sulfonate leaving group having any suitable substituent such as triflate, nosylate, mesylate or tosylate.

In the context of the present invention a high reactive teatrazine is to be understood as a tetrazine molecule having a reaction kinetic constant in the range of 30.000 M⁻¹ S⁻¹ to 200.000 M⁻¹ S⁻¹ for reacting with unsubstituted TCO measured in PBS at 37 °C further details on the reaction kinetic measurement may be found in example 7. Tetrazine compounds having a reaction kinetic constant of 30.000 M⁻¹ S⁻¹ or more react fast enough with TCO in vivo to effeciently react with any pre-targeted TCO and are at the same time sufficiently unstable in order to be cleared from the body before the body is subjected to PET imaging. Tetrazine compounds having a reaction kinetic constant of 200.000 M⁻¹ S⁻¹ or more are so unstable that thay cannot be labelled with 18F and even if labelled, such compounds will degrade in vivo before reaching any pre-targeted TCO and therefore will be unsuitable for use as PET tracers.
Furthermore, it has been found that the higher the reaction kinetic constant correlate with the sensitivity of the compound to base and consequently, the more difficult it is to label the compound by nucleophilic substitution.

Thus, in a preferred embodiment, the precursor molecule for labeling in the method according to the present invention is a tetrazine compound with a reaction kinetic constant in the range of 40.000 M⁻¹ S⁻¹ to 150.000 M⁻¹ S⁻¹, preferably 50.000 M⁻¹ S⁻¹ to 150.000 M⁻¹ S⁻¹, more preferably 60.000 M⁻¹ S⁻¹ to 150.000 M⁻¹ S⁻¹, even more preferred 70.000 M⁻¹ S⁻¹ to 150.000 M⁻¹ S⁻¹ and most preferred 80.000 M⁻¹ S⁻¹ to 150.000 M⁻¹ S⁻¹. Such tetrazine compound will effeciently react with any pre-targeted TCO in vivo and any unreacted tetrazine will quickly be cleared from the body so to facilitate good background to target ration providing clear PET images with high resolution.

The preferred features and embodiments of the separate method steps a) to e) described in the first aspects may be combined so to achieve a preferred method according to the invention.

To test the applicability of high reactive tetrazines that were aliphatic ¹⁸F labelled, 5 compounds were designed see example 7.
All 5 tetrazines displayed rate constant values >55.000 M⁻¹s⁻¹ and are as such considered high reactive tetrazines having increased chance of being suitable for used in pretargeted *in vivo* chemistry (see example 7).

### Ex vivo Blocking Assay

The inventors have recently developed a blocking assay that allows to assess the *in vivo* ligation performance of unlabeled tetrazine derivatives. This assay omits time-consuming development of radiolabeled tetrazines for every ligand to be tested. In this assay, binding of [¹¹¹In]**Tz-Tg** (see structure in example 7), a literature described pretargeted imaging agent, is blocked *in vivo* to the pretargeting vector CC49-TCO (described in Stéen, J.; Jørgensen, J. T.; Christoph, D.; Battisti, U. M.; Nørregaard, K.; Edem, P.; Bratteby, K.; Shalgunov, V.; Martin, W.; Svatunek, D.; Poulie, C. B. M.; Hvass, L.; Simon, M.; Thomas, W.; Rossin, R.; Robillard, M.; Kristensen, J. L.; Mikula, H.; Kjaer, A.; Herth, M. Lipophilicity and Click Reactivity Determine the Performance of Bioorthogonal Tetrazine Tools in Pretargeted in Vivo Chemistry; 2020/12/24/, 2020.), which was administered 72 h prior. The blocking effect of the unlabeled tetrazine derivatives is afterwards quantified via an *ex vivo* biodistribution study and normalized to the binding of [¹¹¹In]**Tz-Tg** determined without any blocking. The setup is displayed in Figure 3.

Investigated tetrazines displayed a blocking effect ranging from 69 to 98% (se example 7). No significant differences were observed for positional isomers.
In a next step, the tolerability of the tetrazine towards various substituents at position 3 of the phenyl ring were explored. Therefore, a hydroxy (compound **uuu**), methoxy (compound iii) and fluoroethoxy (compound **13**) group were introduced in this position. All compounds also showed a blocking effect > 50.000 M⁻¹s⁻¹ with TCO in PBS (example 7).

### Precursor Synthesis and Radiolabeling

Encouraged by these results, it was decided to develop compound **13** into a PET tracer.
The precursor (compound **28**) for this ligand could be synthesized in a yield of 3% over 5 synthesis steps (example 8). It was decided to use a nosylate leaving group since this was found to increased RCYs up 10-fold compared to mesylate or tosylate leaving groups. Radiolabeling of [¹⁸F]**13** was carried out in a one-pot, two-step reaction sequence according to the method described in the present invention (see example 9). A protection/deprotection strategy was chosen since unprotected carboxylic acids prevent ¹⁸F-fluorinations. Radiolabeling was only possible using low basicity labeling conditions according to the method described in the present invention.
3-Substituted 1,2,4,5-tetrazines are reported to be too sensitive for standard ¹⁸F-fluorination approaches. [¹⁸F]13 was labeled in a radiochemical yield (RCY) of 13 ± 4 % (n = 3) with a radiochemical purity (RCP) of >98% and a molar activity (A_{M}) of 55 ± 8 GBq/µmol. The total synthesis was approximately 90 min including separation and formulation of the final product. Maximum isolated amount was 1.1 GBq (Figure 4).

### Bench stability of [¹⁸F]13 and its precursor (28)

The stability of [¹⁸F]**13** and it precursor (**28**) was investigated by analytical HPLC. [¹⁸F]**13** was stable for at least 4 hours and therefore, sufficient for most applications (Figure 5A). In contrast, the precursor (**28**) decomposed over time. The process could be prevented storing the precursor (**28**) at -20 °C as a solid - at least for 3 months (Figure 5B).

### PET-imaging

The ability of [¹⁸F]**13** to be used as a pretargeting imaging agent was evaluated using a similar pretargeted setup that was used for the *ex vivo* blocking study in example 7. Mice were administered with CC49-TCO 72 h prior to administration of [¹⁸F]**13** where after PET/CT scanning was performed 1 h later. Control animals were injected with unconjugated mAb CC49 instead of CC49-TCO, but were otherwise treated exactly the same. After PET/CT scanning, animals were euthanized and an *ex vivo* biodistribution performed. The data are shown in Figure 6. PET/CT data showed that pretreated mice with CC49-TCO had a significantly higher tumor uptake (1.87 ± 0.31 %ID/g) compared to control (0.01 ± 0.01 %ID/g) (mean ± S.E.M, *n* = 4, *p* = 0.006) (Figure 6). The tumor uptake was clearly visible. A tumor-to-muscle (T/M) ratio of 20.1 and a tumor-to-blood (T/B) ratio of 1.2 from the image derived data (heart uptake used as surrogate for blood uptake) was determined. The relatively low T/B ratio is likely due to ligation of [¹⁸F]**13** with remnant CC49-TCO still circulating in the blood. Clearance of CC49-TCO from blood will most likely increase the ratio drastically. Uptake in tissues beside tumor and blood was low. *Ex vivo* biodistribution confirmed the results from the imaging experiment (Figure 6).

Pretargeted imaging of nanomedicines has the potential to revolutionize state-of-art nuclear imaging. In this study, the inventors have developed the first aliphatic ¹⁸F-Tetrazine suitable for *in vivo* pretargeted PET imaging. [¹⁸F]**13** has been synthesized in sufficient yield, purity and molar activity for *in vivo* evaluation. These studies showed that [¹⁸F]**13** displayed favorable pharmacokinetics and good target-to-background ratios in pretargeted experiments. [¹⁸F]**13** possess the potential to be clinically translated for *in vivo* pretargeted PET imaging. [¹⁸F]**13** shows in PET imaging the highest tumor-to-musle ratio compared to standardly applied radiometal based structures ([64Cu]**Cu-NOTa-PEG7-H-Tz** and [111In]**Tz-Tg**) as well as our thus far best identified probe ZZ (18F-aromatic labeled) (Figure 6).

Thus, in the second aspect of the invention there is provided a Tetrazine compound, having the formula I: 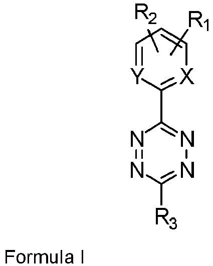
Wherein R1 is H or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
Wherein, R3 is H or 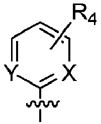 and wherein, X and Y are independently selected from: -CH₂- and -N- and wherein the curly sign indicates the link to the tetrazine,
And wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein, at least one of R2 or R4 is a moiety and wherein R5 is H or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 18F or 19F labelled C1-C6 aliphatic groups comprising fluoromethyl, fluoroethyl, 1-fluoropropyl, 1-fluorobutyl, 1-fluoropentyl, 1-fluorohexyl and wherein the tetrazine compound of formula I has a lipophilicity of cLogD₇.₄ < -0.5

In general, when a 18F or 19F labelled C1-C6 aliphatic group including fluoromethyl, fluoroethyl, 1-fluoropropyl, 1-fluorobutyl, 1-fluoropentyl, 1-fluorohexyl, is connected to an aromatic ring in the tetrazine compound, said group is preferably connected to the Tetrazine compound by means of direct covalent bonding to the aromatic ring or connected to the aromatic ring via COO, (CH₂)ₙCOO, CONH, (CH₂)ₙCNH, CONCH₃, (CH₂)ₙCONCH₃, O, (CH₂)ₙO, NH, (CH₂)ₙNH, NCH₃, (CH₂)ₙNCH₃, S or (CH₂)ₙS where n could be 1, 2, 3, 4.

Thus, when R1 in formula I is a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl, R1 is preferably connected to the Tetrazine compound by means of direct covalent bonding to the aromatic ring or connected to the aromatic ring via COO, (CH₂)ₙCOO, CONH, (CH₂)ₙCNH, CONCH₃, (CH₂)ₙCONCH₃, O, (CH₂)ₙO, NH, (CH₂)ₙNH, NCH₃, (CH₂)ₙNCH₃, S or (CH₂)ₙS where n could be 1, 2, 3, 4.

In general, when a 18F or 19F labelled C1-C6 aliphatic group including fluoromethyl, fluoroethyl, 1-fluoropropyl, 1-fluorobutyl, 1-fluoropentyl, 1-fluorohexyl, is connected to the Tetrazine compound by means of conjugation to a benzylic amine, said group is preferably connected to the Tetrazine compound by means of conjugation to a benzylic amine via direct covalent bonding or via COO, (CH2)nCOO, CONH, (CH2)nCNH, CONCH3, (CH2)nCONCH3, (CH2)nO, (CH2)nNH, (CH2)nNCH3, or (CH2)nS wherein n is 1, 2, 3, 4

Thus, when R5 in formula I is a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl, R5 is preferably connected to the Tetrazine compound by means of conjugation to a benzylic amine via direct covalent bonding or via COO, (CH2)nCOO, CONH, (CH2)nCNH, CONCH3, (CH2)nCONCH3, (CH2)nO, (CH2)nNH, (CH2)nNCH3, or (CH2)nS wherein n is 1, 2, 3, 4.

It has been found that when the tetrazine compounds of Formula I have a sufficiently high polarity, they will not cross the cell membrane and therefore will more effeciently reach pretargeting vectors which do not internalize, furthermore, intracellular accumulation of the tetrazine molecule is prevented which contribute to a higher background to target ratio providing better quality PET images. Thus, the low lipophilicity is beneficial for the use of the tetrazine compounds as imaging agents and as diagnostic agents in relation to certain diseased tissue in animals and humans, such as cancer tissue, cancerous cells, infected cells or pathogens. Thus, preferably, the tetrazine compound of formula I has a lipophilicity of cLogD₇.₄ < -2, more preferably cLogD_{7.4} < -3 most preferred cLogD_{7.4} < -6.

In order for the tetrazine compounds of Formula I to have a lipophilicity of cLogD_{7.4} < -0.5 the moiety is preferably selected from: -OH, NR6R7, CH2N(CH2COOH)2, CH2NHCH2COOH, CH2NR7CH2COOH, COOH, CONR6R7, SO3H, SO2NH2, and SO2NH wherein R6 is H, CH3, CH2CH3, CH2CH2CH3 or H2COOH; and R7 is H, CH3, CH2CH3, CH2CH2CH3, CH2COOH, or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl.

In a preferred embodiment, the moiety is selected from CH2N(CH2COOH)2, CH2NHCH2COOH, CH2NR7CH2COOH, COOH, wherein R7 is H, CH3, CH2CH3, CH2CH2CH3, CH2COOH or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl

In preferred embodiments, the moiety is selected from the polar groups: 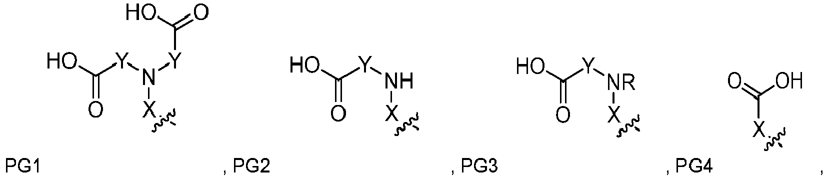 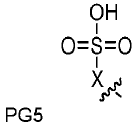 Wherein X is CO(CH2)n, SO2(CH2)n, (CH2)n or (OCH2CH2)n and n = 0, 1, 2 or 3; and wherein Y is (CH2)n, (OCH2CH2)n or CO(CH2)n and n = 0, 1, 2 or 3; and wherein R is (CH2)nCH3, (OCH2CH2)nOH, CO(CH2)nCOOH, (OCH2CH2)nOCH2COOH or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and n = 0, 1, 2 or 3; and wherein the curly sign indicates the link to the aromatic ring.

In a preferred embodiment, the tetrazine compounds of formula I is selected from: 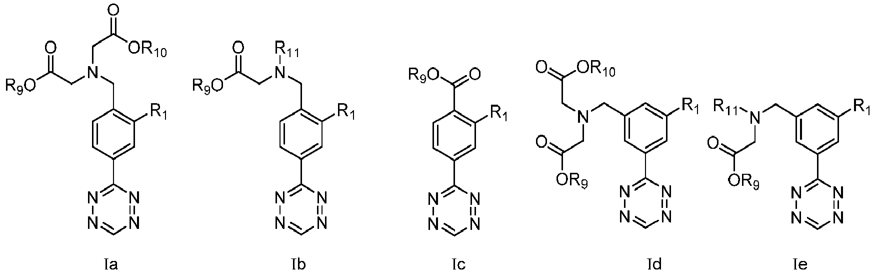 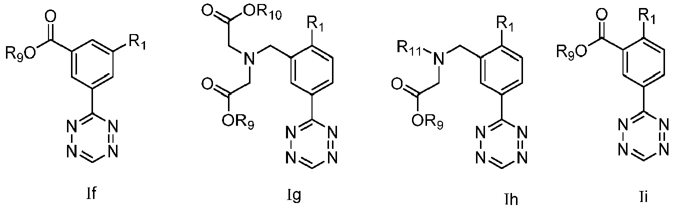 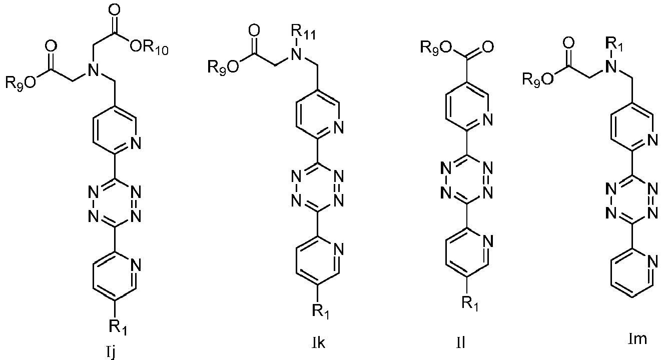 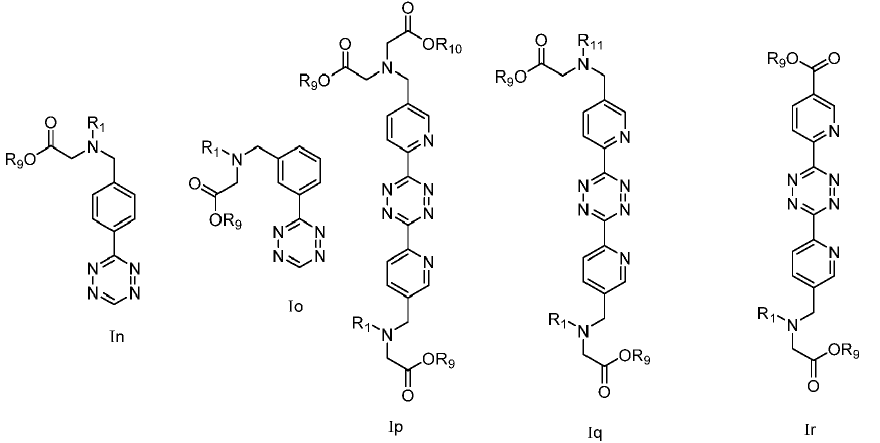 Wherein R1 is a group selected from the group consisting of 18F or 19F labelled C1-C6 aliphatic groups including fluoromethyl, fluoroethyl, 1-fluoropropyl, 1 -fluorobutyl, 1-fluoropentyl, 1-fluorohexyl and wherein R9 R10 and R11 are independently selected from H, (CH2)nCH3, CH2CH2(OCH2CH2)nOH, (CH2)nCO(CH2)nCOOH or CH2CH2(OCH2CH2)nOCH2COOH and n = 0, 1, 2 or 3.

In a preferred embodiment, the tetrazine compounds of formula I is selected from; 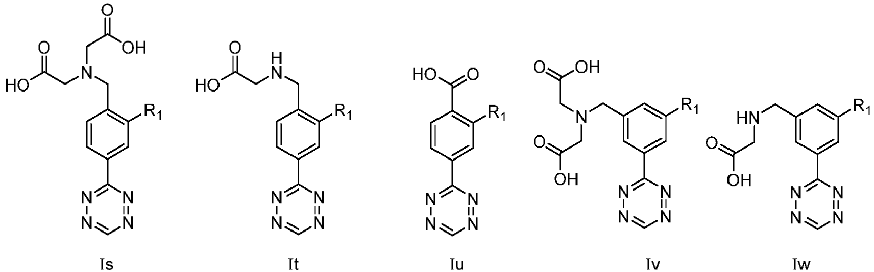 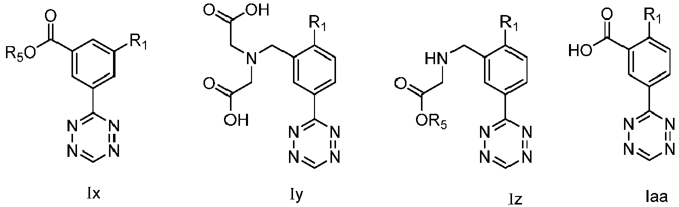 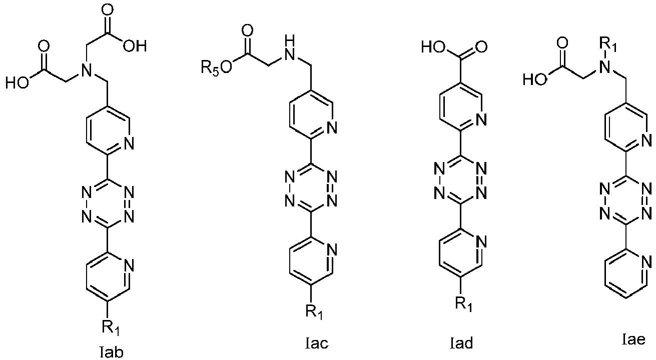 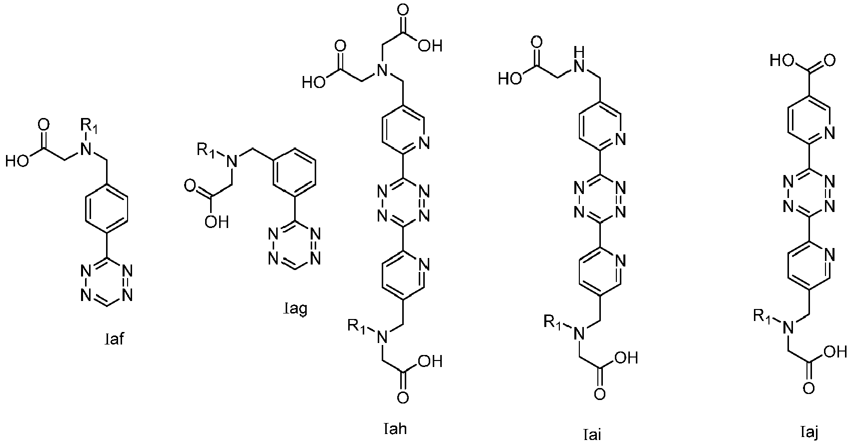 Wherein R1 is a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl.

In line with the above, when R1 in any of the preferred formula I is connected to the Tetrazine compound at the aromatic ring, said connection is preferably by means of direct covalent bonding to the aromatic ring or connected to the aromatic ring via COO, (CH2)nCOO, CONH, (CH2)nCNH, CONCH3, (CH2)nCONCH3, O, (CH2)nO, NH, (CH2)nNH, NCH3, (CH2)nNCH3, S or (CH2)nS where n could be 1, 2, 3, 4.

Likewise, when R1 in any of the preferred formula I is connected to the Tetrazine compound by means of conjugation to a benzylic amine, said connection is preferably via direct covalent bonding or via COO, (CH2)nCOO, CONH, (CH2)nCNH, CONCH3, (CH2)nCONCH3, (CH2)nO, (CH2)nNH, (CH2)nNCH3, or (CH2)nS wherein n is 1, 2, 3, 4.

In another preferred embodiment the tetrazine compounds of formula I is selected from; 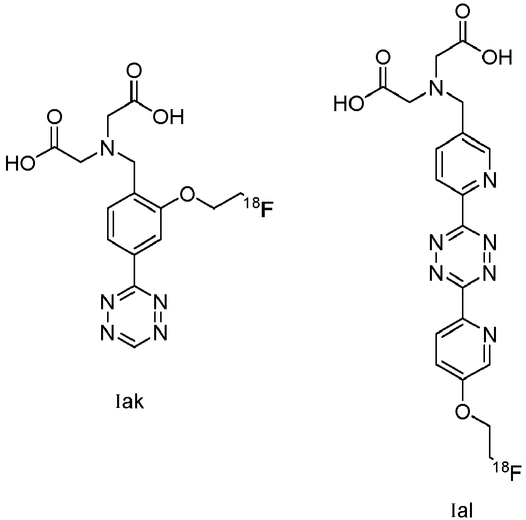 lak and lal illustrate the 18F-labelled compound, the corresponding 19F-labelled compounds are the most preferred reference compound.

In a third aspect, the invention provides a precursor molecule, having the formula II: 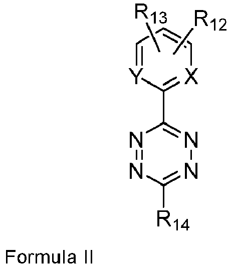
Wherein R12 is H or an C1-C6 aliphatic group such as methyl, ethyl, propyl, butyl, pentyl, hexyl comprising a sulfonate leaving group having any suitable substituent such as triflate, nosylate, mesylate or tosylate and wherein, X and Y are independently selected from: -CH₂- and -N- and
Wherein, R14 is H or 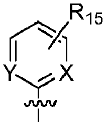 and wherein, X and Y are independently selected from: -CH₂- and -N- and wherein the curly sign indicates the link to the tetrazine,
And wherein R13 and R15 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R16, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein, at least one of R13 or R15 is a moiety and wherein R16 is H or C1-C6 aliphatic group such as methyl, ethyl, propyl, butyl, pentyl, hexyl comprising a sulfonate leaving group having any suitable substituent such as triflate, nosylate, mesylate or tosylate and wherein, at least one of R12 and R16 is an aliphatic group comprising a sulfonate leaving group having any suitable substituent such as triflate, nosylate, mesylate or tosylate and wherein, any functional groups comprising OH, NH or SH are substituted with a protective group selected from the group comprising Boc, Fmoc, CH2C5H5, (CH2)nCH3, C(CH3)3, COCH3, COCF3, C(C5H5)3 and wherein, the precursor molecule has a lipophilicity of ClogD_{7.4} < -0.5 after deprotection.

All the precursors of formula II can be used to provide the compounds of formula I by subjecting the precursor molecule to the method described in the first aspect of the invention. Thus, in line with the preferred embodiments of formula I, the following embodiments outline the corresponding preferred precursor molecules of formula II.

In general, when a C1-C6 aliphatic group such as methyl, ethyl, propyl, butyl, pentyl, hexyl comprising a sulfonate leaving group having any suitable substituent such as triflate, nosylate, mesylate or tosylate, is connected to an aromatic ring in the tetrazine compound, said group is preferably connected to the Tetrazine compound by means of direct covalent bonding to the aromatic ring or connected to the aromatic ring via COO, (CH₂)ₙCOO, CONH, (CH₂)ₙCNH, CONCH₃, (CH₂)ₙCONCH₃, O, (CH₂)ₙO, NH, (CH₂)ₙNH, NCH₃, (CH₂)ₙNCH₃, S or (CH₂)ₙS where n could be 1, 2, 3, 4.

Thus, when R12 in formula II is a C1-C6 aliphatic group such as methyl, ethyl, propyl, butyl, pentyl, hexyl comprising a sulfonate leaving group having any suitable substituent such as triflate, nosylate, mesylate ortosylate, R12 is preferably connected to the Tetrazine compound by means of direct covalent bonding to the aromatic ring or connected to the aromatic ring via COO, (CH₂)ₙCOO, CONH, (CH₂)ₙCNH, CONCH₃, (CH₂)ₙCONCH₃, O, (CH₂)ₙO, NH, (CH₂)ₙNH, NCH₃, (CH₂)ₙNCH₃, S or (CH₂)ₙS where n could be 1, 2, 3, 4.

In general, when a C1-C6 aliphatic group such as methyl, ethyl, propyl, butyl, pentyl, hexyl comprising a sulfonate leaving group having any suitable substituent such as triflate, nosylate, mesylate or tosylate, is connected to the Tetrazine compound by means of conjugation to a benzylic amine, said group is preferably connected to the Tetrazine compound by means of conjugation to a benzylic amine via direct covalent bonding or via COO, (CH2)nCOO, CONH, (CH2)nCNH, CONCH3, (CH2)nCONCH3, (CH2)nO, (CH2)nNH, (CH2)nNCH3, or (CH2)nS wherein n is 1, 2, 3, 4.

Thus, when R16 in formula II is a C1-C6 aliphatic group such as methyl, ethyl, propyl, butyl, pentyl, hexyl comprising a sulfonate leaving group having any suitable substituent such as triflate, nosylate, mesylate ortosylate, R16 is preferably connected to the Tetrazine compound by means of conjugation to a benzylic amine via direct covalent bonding or via COO, (CH2)nCOO, CONH, (CH2)nCNH, CONCH3, (CH2)nCONCH3, (CH2)nO, (CH2)nNH, (CH2)nNCH3, or (CH2)nS wherein n is 1, 2, 3, 4.

In a preferred embodiment, the precursor molecule has a lipophilicity of cLogD_{7.4} < -2 after deprotection, such as cLogD_{7.4} < -3 after deprotection or such as cLogD_{7.4} < -6 after deprotection.

In one embodiment, the moiety in the compound of formula II is selected from: -OH, NR17R18, CH2N(CH2COOH)2, CH2NHCH2COOH, CH2NR19CH2COOH, COOH, CONR17R16, SO3H, SO2NH2, and SO2NH wherein R17 is H, CH3, CH2CH3, CH2CH2CH3 or H2COOH; and wherein R18 is H, CH3, CH2CH3, CH2CH2CH3, CH2COOH or a C1-C6 aliphatic group such as methyl, ethyl, propyl, butyl, pentyl, hexyl comprising a sulfonate leaving group having any suitable substituent such as triflate, nosylate, mesylate or tosylate and wherein R19 is H, CH3, CH2CH3, CH2CH2CH3, CH2COOH, or a C1-C6 aliphatic group such as methyl, ethyl, propyl, butyl, pentyl, hexyl comprising a sulfonate leaving group having any suitable substituent such as triflate, nosylate, mesylate or tosylate.

Preferably, the moiety in the compound of formula II is selected from CH2N(CH2COOH)2, CH2NHCH2COOH, CH2NR19CH2COOH, COOH, wherein R19 is H, CH3, CH2CH3, CH2CH2CH3, CH2COOH or a C1-C6 aliphatic group such as methyl, ethyl, propyl, butyl, pentyl, hexyl comprising a sulfonate leaving group having any suitable substituent such as triflate, nosylate, mesylate or tosylate.

In a preferred embodiment, the moiety in the compound of formula II is selected from the protected polar groups: 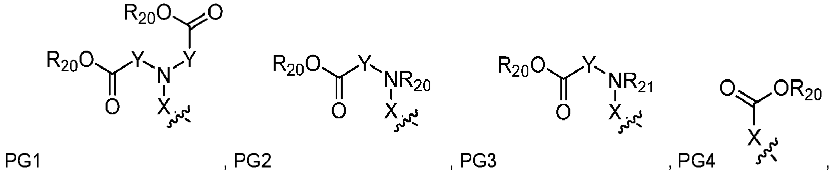 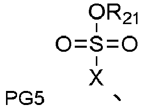
Wherein X is CO(CH2)n, SO2(CH2)n, (CH2)n or (OCH2CH2)n and n = 0, 1, 2 or 3; and
wherein Y is (CH2)n, (OCH2CH2)n or CO(CH2)n and n = 0, 1, 2 or 3; and wherein R20 is independently selected from Boc, Fmoc, CH2C5H5, (CH2)nCH₃, C(CH3)3, COCH3, COCF3 or C(C5H5)3 and wherein R21 is (CH2)nCH3, (OCH2CH2)nOH, CO(CH2)nCOOH, (OCH2CH2)nOCH2COOH or a C1-C6 aliphatic group such as methyl, ethyl, propyl, butyl, pentyl, hexyl comprising a sulfonate leaving group having any suitable substituent such as triflate, nosylate, mesylate or tosylate and n = 0, 1, 2 or 3; and wherein the curly sign indicates the link to the aromatic ring.

In a preferred embodiment, the precursor molecule of formula II is selected from: 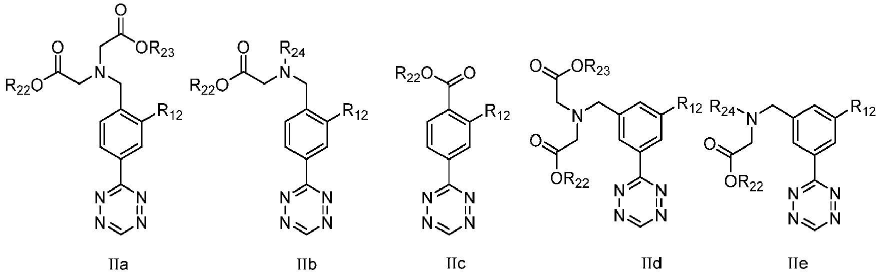 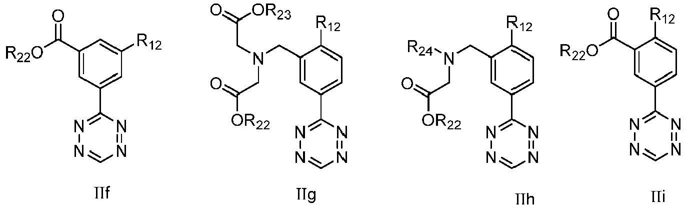 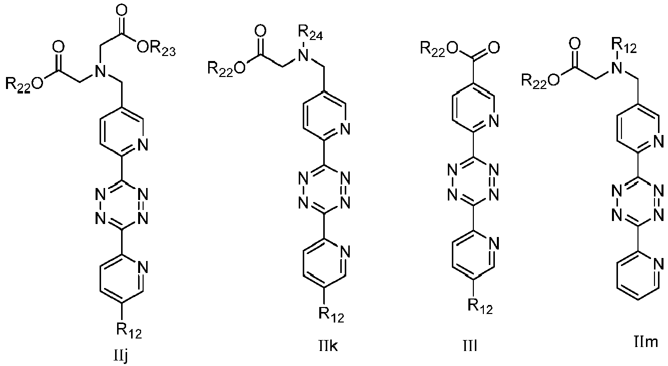 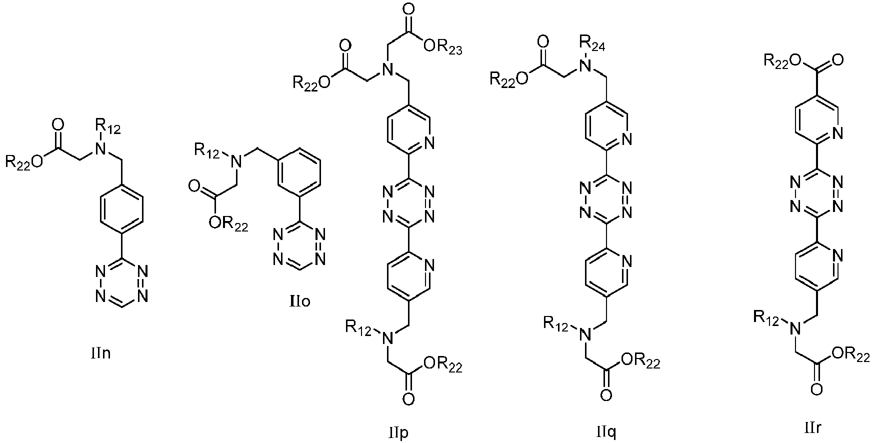 Wherein R12 is an C1-C6 aliphatic group such as methyl, ethyl, propyl, butyl, pentyl, hexyl comprising a sulfonate leaving group having any suitable substituent such as triflate, nosylate, mesylate or tosylate and wherein R22 R23 and R24 are independently selected from (CH2)nCH3, C(CH3)3, CH2C5H5 CH2CH2(OCH2CH2)nOH, (CH2)nCO(CH2)nCOO(CH2)nCH3, (CH2)nCO(CH2)nCOOC(CH3)3, (CH2)nCO(CH2)nCOOCH2C5H5, CH2CH2(OCH2CH2)nOCH2COO(CH2)nCH3, CH2CH2(OCH2CH2)nOCH2COOC(CH3)3, CH2CH2(OCH2CH2)nOCH2COOC5H5, COOC(CH3)3 and n = 0, 1, 2 or 3.

In a preferred embodiment, the precursor molecule of formula II is selected from; 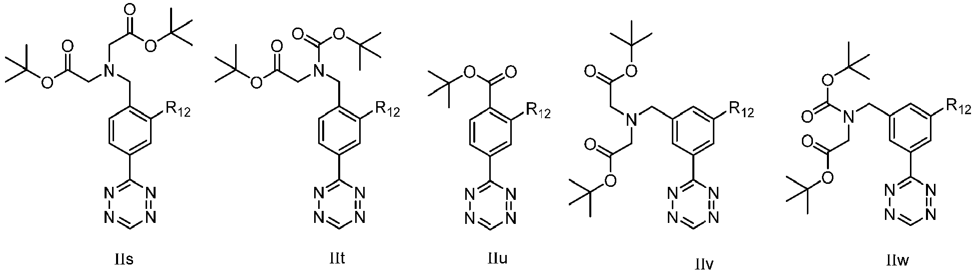 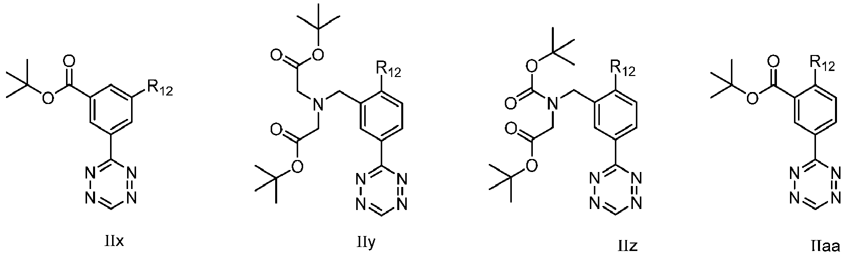 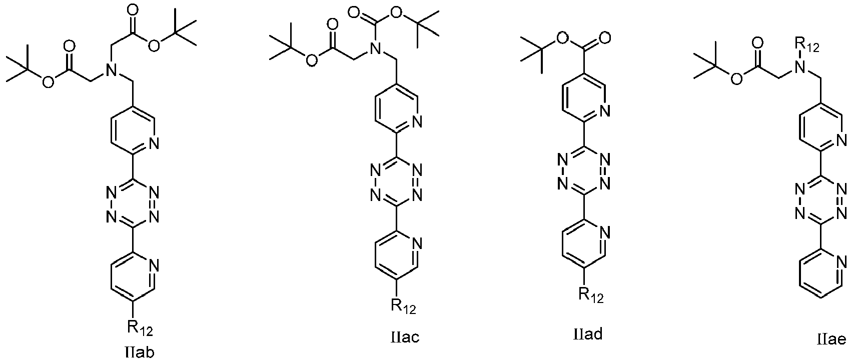 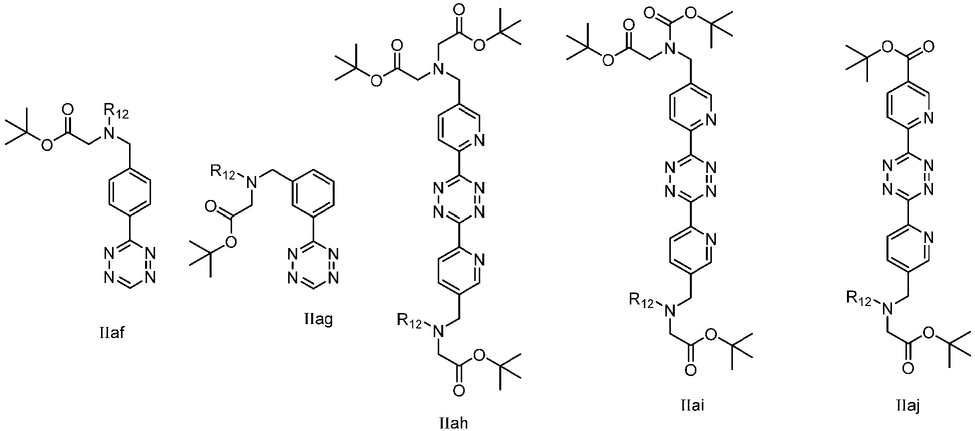 Wherein R12 an C1-C6 aliphatic group such as methyl, ethyl, propyl, butyl, pentyl, hexyl comprising a sulfonate leaving group having any suitable substituent such as triflate, nosylate, mesylate or tosylate.

In line with above, when R12 in any of the preferred formula II is connected to the Tetrazine compound at the aromatic ring, said connection is preferably by means of direct covalent bonding to the aromatic ring or connected to the aromatic ring via COO, (CH₂)ₙCOO, CONH, (CH₂)ₙCNH, CONCH₃, (CH₂)ₙCONCH₃, O, (CH₂)ₙO, NH, (CH₂)ₙNH, NCH₃, (CH₂)ₙNCH₃, S or (CH₂)ₙS where n could be 1, 2, 3, 4.

Likewise, when R12 in any of the preferred formula II is connected to the Tetrazine compound by means of conjugation to a benzylic amine said connection is preferably via direct covalent bonding or via COO, (CH2)nCOO, CONH, (CH2)nCNH, CONCH3, (CH2)nCONCH3, (CH2)nO, (CH2)nNH, (CH2)nNCH3, or (CH2)nS wherein n is 1,2,3,4.

In a most preferred embodiment, the precursor molecule of formula II is selected from;

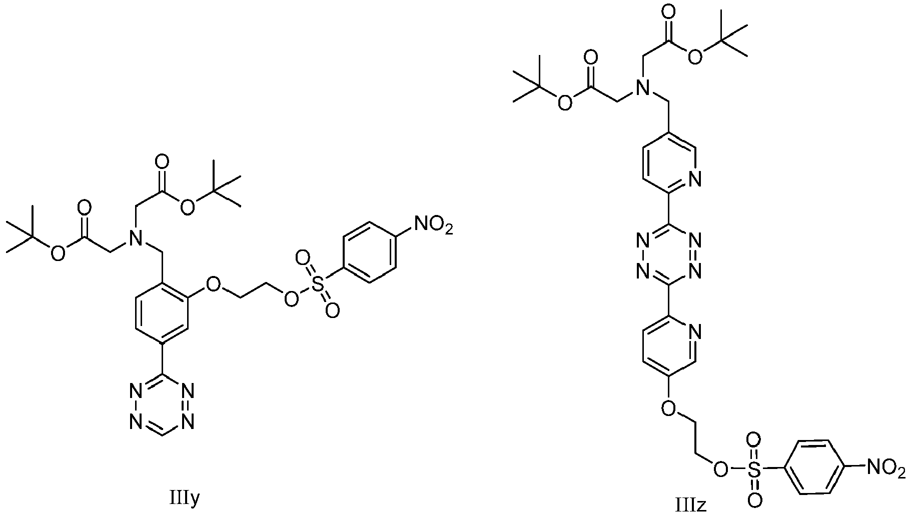

As shown herein, the tetrazine compounds of Formula I are excellent for use in bioorthogonal chemistry for pretargeting strategy to PET imaging of e.g. cancer tissue. PET imaging is a valuable tool in diagnosis e.g. of cancer and may also be used to continuously follow the development of e.g. a cancer and thus also the effect of a treatment of the same. It is moreover shown in the present Examples, that the compounds of Formula I provides very good imaging results in mice being infested with a human cancer type as shown with PET scanning.

Accordingly, in a fourth aspect, the present invention relates to use of a Tetrazine compound for PET imaging, wherein said Tetrazine compound, having the formula III: 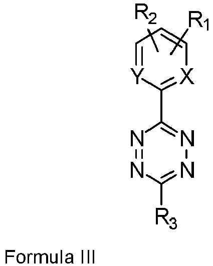
Wherein R1 is a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and Wherein, R3 is H or 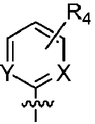 and wherein, X and Y are independently selected from: -CH₂- and -N- and wherein the curly sign indicates the link to the tetrazine,
And wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R5 is H or a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 18F labelled C1-C6 aliphatic groups comprising fluoromethyl, fluoroethyl, 1-fluoropropyl, 1-fluorobutyl, 1-fluoropentyl, 1-fluorohexyl and wherein, the tetrazine compound of formula I has a lipophilicity of cLogD₇.₄ < - 0.5

Preferably, the imaging is of cancer tissue.

In a fifth aspect, the present invention relates to use of a Tetrazine compound according to the second aspect for PET imaging wherein the tetrazine compound is labelled with ¹⁸F.

Preferably, the imaging is of cancer tissue.

In a sixth aspect, the present invention relates to use of a Tetrazine compound for diagnostics, wherein said Tetrazine compound, having the formula III: 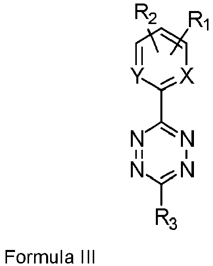
Wherein R1 is a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
Wherein, R3 is H or 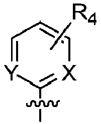 and wherein, X and Y are independently selected from: -CH₂- and -N- and wherein the curly sign indicates the link to the tetrazine,
And wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R5 is H or a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 18F labelled C1-C6 aliphatic groups comprising fluoromethyl, fluoroethyl, 1-fluoropropyl, 1-fluorobutyl, 1-fluoropentyl, 1-fluorohexyl and wherein, the tetrazine compound of formula I has a lipophilicity of cLogD₇.₄ < - 0.5

Preferably, said diagnostics is of a cancer.

In a seventh aspect, the present invention relates to use of a Tetrazine compound according to the second aspect for diagnostics wherein the tetrazine compound is labelled with ¹⁸F.

Preferably, said diagnostics is of a cancer.

In order to be able to identify a 18F-labelled tetrazine of formula I or III or to determine the specific activity (activity/mol), it is necesarry to determine the retention time as well as its concentration via HPLC measurrments according to the present invention. For this purpose of quality control a 19F-labeled version (reference compounds) of the 18F-labeled compound is synthesized and is used accordingly. The structure of the reference compounds will be determined via NMR analysis and then used to determine its precise HPLC retention time. Co-injection of the reference compound and the 18F-labeled compound allows the structural identification of the 18F-labeled indirectly when the reference compound and the 18F-labeled compound co-elute. A area under the curve correlation with injected amount of reference compound allows further the determine the concentration of the 18F-labeled compound in question.

Accordingly in an eight aspect, the present invention relates to use of a Tetrazine compound for quality control, wherein the Tetrazine compound, having the formula IV: 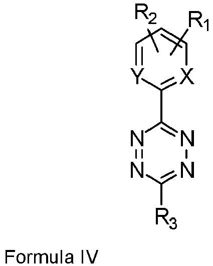
Wherein R1 is a group selected from the group consisting of 19F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
Wherein, R3 is H or 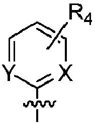 and wherein, X and Y are independently selected from: -CH₂- and -N- and wherein the curly sign indicates the link to the tetrazine,
And wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R5 is H or a group selected from the group consisting of 19F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 19F labelled C1-C6 aliphatic groups comprising fluoromethyl, fluoroethyl, 1-fluoropropyl, 1-fluorobutyl, 1-fluoropentyl, 1-fluorohexyl and wherein, the tetrazine compound of formula I has a lipophilicity of cLogD₇.₄ < - 0.5

In a ninth aspect, the present invention relates to use of a Tetrazine compound according to the second aspect for quality control wherein the tetrazine compound is labelled with ¹⁹F.

In a tenth aspect, the present invention relates to use of a precursor molecule for aliphatic ¹⁸F-labelling according to the method described in the first aspect, wherein the precursor molecule, have the formula V: 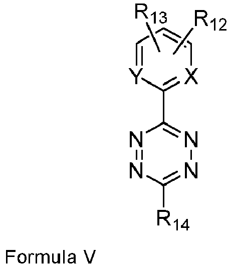
Wherein R12 is an C1-C6 aliphatic group such as methyl, ethyl, propyl, butyl, pentyl, hexyl comprising a sulfonate leaving group having any suitable substituent such as triflate, nosylate, mesylate or tosylate and wherein, X and Y are independently selected from: -CH₂- and -N- and
Wherein, R14 is H or 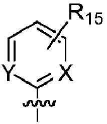 and wherein, X and Y are independently selected from: -CH₂- and -N- and wherein the curly sign indicates the link to the tetrazine,
And wherein R13 and R15 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R16, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R16 is H or C1-C6 aliphatic group such as methyl, ethyl, propyl, butyl, pentyl, hexyl comprising a sulfonate leaving group having any suitable substituent such as triflate, nosylate, mesylate or tosylate and wherein, at least one of R12 and R16 is an aliphatic group comprising a sulfonate leaving group having any suitable substituent such as triflate, nosylate, mesylate or tosylate and wherein, any functional groups comprising OH, NH or SH are substituted with a protective group selected from the group comprising Boc, Fmoc, CH2C5H5, (CH2)nCH3, C(CH3)3, COCH3, COCF3, C(C5H5)3 and wherein the precursor molecule has a lipophilicity of cLogD_{7.4} < -0.5 after deprotection.

In an eleventh aspect, the present invention relates to use of a precursor molecule according to the third aspect for aliphatic ¹⁸F-labelling according to the method described in the first aspect.

### EXAMPLES

### Example 1 Screening of preconditioning and elution anion pairs

Results from EE screening using different preconditioning- and eluting anions over a range of concentrations. Table 1 displays concentrations of eluting anions in mM required to elute 90% of [¹⁸F]fluoride from the QMA cartridge. These values were determined by fitting the Hill equation to a set of 7 elutions (5-100 mM of the eluting anion in 1 mL of eluting solvent (5-100 µmol). Anion exchange cartridge (Sep-Pak^{®} Light QMA cartridge 130 mg sorbent, chloride as counter ion were prepared by flushing with of EtOH (2 mL) followed by a solution of the appropriate preconditioning anion (0.5 M solution, 10 mL). The cartridge was then washed with H₂O (10 mL) and dried with air. [¹⁸F]fluoride was delivered from the cyclotron and activity concentration was adjusted to 100-300 MBq/mL by diluting with milliQ water. The [¹⁸F]Fluoride solution was applied to the cartridge (0.2-2 mL, 20-100 MBq) using a 1 mL syringe followed by flushing the cartridge with air and activity stuck to the cartridge was measured using a dose calibrator. The cartridge was then eluted in the same direction using 1 mL of the eluting anion solution. The cartridge was flushed with air and activity was measured of both the eluted cartridge and the collected eluate. [¹⁸F]Fluoride recovery was calculated as the decay corrected activity difference between the cartridge before elution and the vial with the eluate. Colors indicate concentrations required to obtain EE 90%, with white representing the lowest concentration and gradually darker blue for higher concentrations. K222 = Kryptofix^{®} 222, 18C6 = 18-Crown-6.

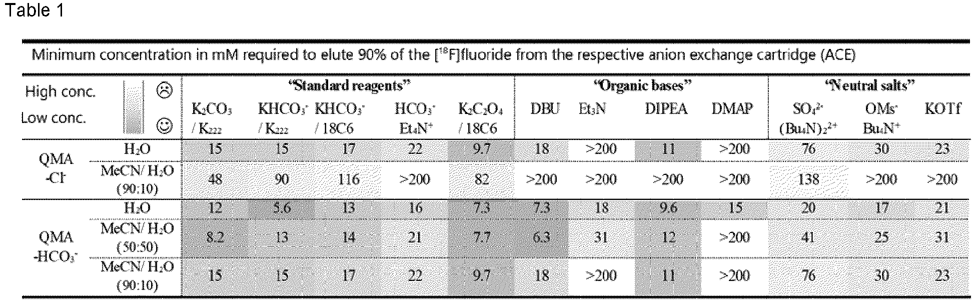

### Example 2

To determine the effect of the preconditioning anion on the reaction conditions, a series of reactions were conducted using precursor **16** to form [¹⁸F]**1**. [¹⁸F]Fluoride solution was prepared in the same manner as for the elution experiments (Example 1). Anion exchange cartridge (with different preconditioning anios according to table 2) was eluted into a 4 mL V-shaped glass vial using a 1 mL solution of Bu₄NOMs (20 mM in 50% MeCN in H₂O). The vial was placed in a heating block and heated to 100°C under a stream of nitrogen for 10 min to remove the eluting solvent. Azeotropic drying by MeCN added 2x0.5 mL followed by 5 min drying at 100°C under a stream of nitrogen. The RCC was determined by TCL and the identiy of the labeled product was determined by analytical HPLC.

**Table 2. Radiolabeling of precursor 16 in MeCN at 120°C, 5 min reaction time with Bu₄NOMs elution (20mM in 50% MeCN/H₂O, 1 mL) using different preconditioning of the QMA.**

| Screening of different preconditioning anions for elution by Bu₄NOMs | | | | |
|---|---|---|---|---|
| Preconditioning anion | pKa⁵⁴ | EE (%) | RCC (%) | pRCY (%) |
| Cl⁻ | -7.0 | 24 | 0 | 0 |
| OMs⁻ | -1.9 | 28 | 0 | 0 |
| SO₄²⁻ | 2.0 (-9.0)^{a} | 96 | 0 | 0 |
| H₂PO₄⁻ | 2.1 | 86^{b} | 0 | 0 |
| C₂O₄²⁻ | 4.2 (1.3)^{a} | 99 | >5% | >5% |
| AcO⁻ | 4.7 | 45 | Traces | Traces |
| HCO₃ | 6.4 | 91.0 ± 5.4^{c} | 56.7 ± 8.9^{c} | 52.1 ± 6.9^{c} |
| HPO₄²⁻ | 7.2 | 95.6 ± 0.9^{c} | 53.4 ± 4.3^{c} | 51.0 ± 5.4^{c} |
| CO₃²⁻ | 10.3 | 92.0 ± 6.3^{c} | 55.1±1.1^{c} | 50.7 ± 4.3^{c} |
| PO₄³⁻ | 12.7 | 97.0 ± 0^{c} | 74.3 ± 14.2^{c} | 72.0 ± 11.3^{c} |

| | | | | |
|---|---|---|---|---|
| ^{a}pKa for second protonation if only one of the divalent anion was investigated. ^{b}Higher elution could be due to a mixture of mono- and divalent anions formed in aqueous solution. ^{c} Reactions performed in triplicates. | | | | |

### Example 3 Multiparametric radiolabeling screen using selected preconditioning anions and elution reagents

To determine the effect of combining different eluting- and preconditioning anions in three different solvents a multiparametric screening was conducted using precursor **16** to form [¹⁸F]1. All reactions were performed according to the general procedure described in Example 2. The results are presented in Table 3.

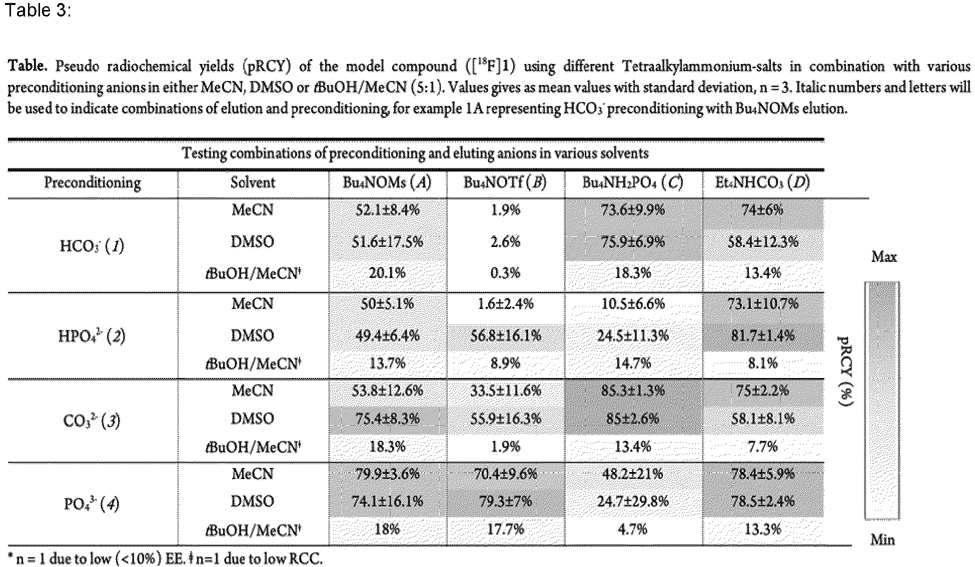

### Example 4 Improving the labeling procedures of known radiopharmaceuticals/synthons

To test the applicability of the derived mild conditions from example 3 a series of clinically relevant tracers and synthons were tested using these elution conditions. First the tracers were labeled with our derived conditions manually according to the procedure described in Example 2. The tracers were then labeled manually according to the previously published proceedures to enable a relevant comparison in our hands to the published conditions. The tracers were then labeled according to the derived conditions in automated synthesis using a Scansys synthesis module to isolate the tracers as a formulated product and compare the isolated RCY to the published proceedures. The results are presented in Table 4.

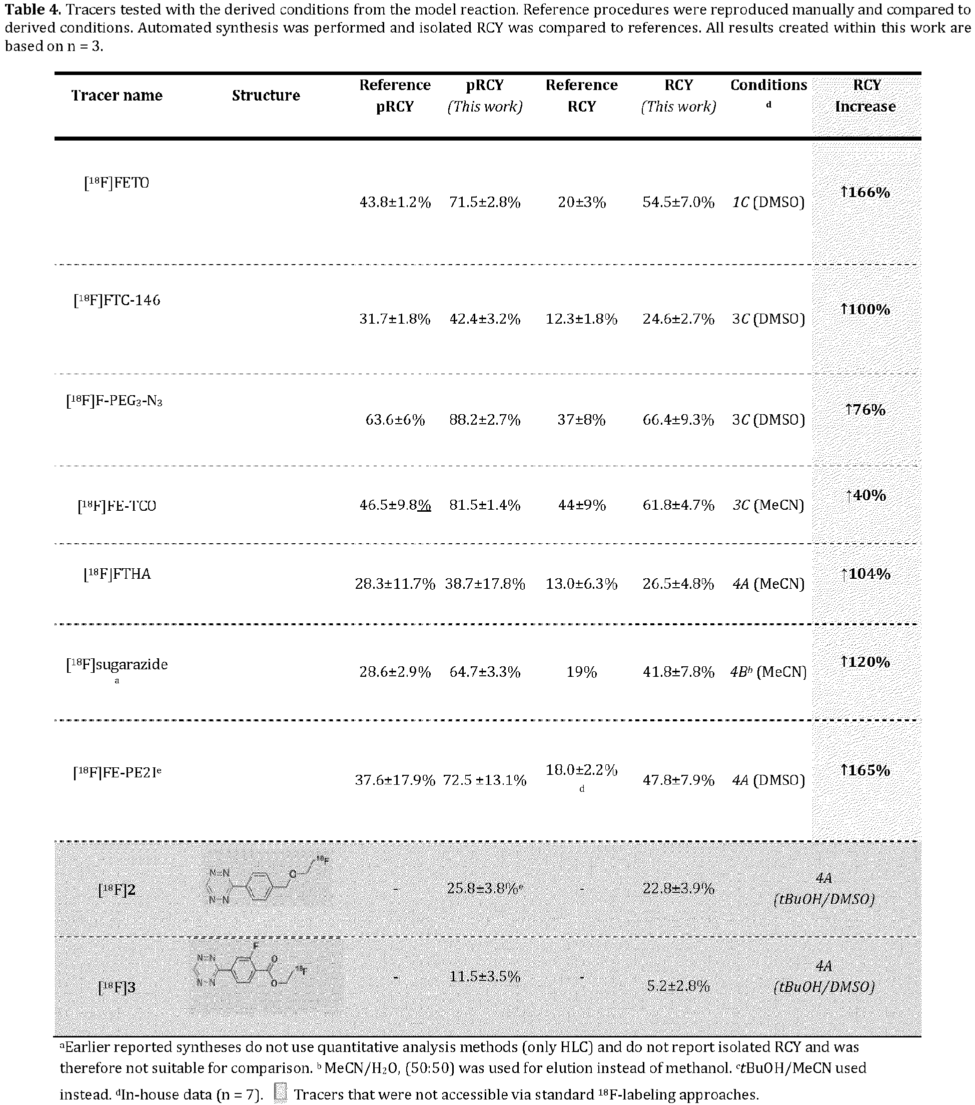

### Example 5 The impact of solvent on RCY

To test the low-base conditions labeling of the notoriously base sensitive mono-substitued tetrazine scaffold was evaluated. The reactions were conducted according to the procedure described in Example 2 to label the specified precursor to form the labeld terazine [¹⁸F]**2** or [¹⁸F]**3**. A selection of the tested conditions are presented in Table 6 . For the labeling of [¹⁸F]**2** we used the OMs precursor (**18**), OTs precursor (**19**) and the ONs precursor (**20**). For the labeling of [¹⁸F]**3** we used the OTs precursor (**21**) and the ONs precursor (**22**). All reactions were carried out at 100°C, the results are presented in Table 6.

**Table 6 Results from manual radiolabeling reactions to obtain the ¹⁸F-radiolabelled terazines [¹⁸F]2 and [¹⁸F]3.**

| **Target product** | **Precursor** | **Preconditioning** | **Elution** | **Solvent** | **pRCY** |
|---|---|---|---|---|---|
| **[¹⁸F]2** | **18** | PO₄³⁻ | Bu₄NOMs | tBuOH/MeCN | 1.8% |
| **[¹⁸F]2** | **19** | PO₄³⁻ | Bu₄NOMs | tBuOH/MeCN | 4.5% |
| **[¹⁸F]2** | **20** | PO₄³⁻ | Bu₄NOMs | tBuOH/MeCN | 25.8±3.8%^{a} |
| **[¹⁸F]2** | **20** | PO₄³⁻ | Bu₄NOMs | tBuOH/DMSO | 23.6% |
| **[¹⁸F]2** | **20** | CO₃²⁻ | Bu₄NOMs | tBuOH/MeCN | 0% |
| **[¹⁸F]2** | **20** | PO₄³⁻ | Et₄NHCO₃ | tBuOH/MeCN | 0% |
| **[¹⁸F]2** | **20** | CO₃²⁻ | Bu₄NH₂PO₄ | tBuOH/MeCN | 0% |
| **[¹⁸F]2** | **20** | CO₃²⁻ | Et₄NHCO₃ | MeCN | 0% |
| **[¹⁸F]2** | **20** | HPO₄²⁻ | Bu₄NOMs | MeCN | 0% |
| **[¹⁸F]2** | **20** | HPO₄²⁻ | Bu₄NOMs | tBuOH/DMSO | 23.0% |
| **[¹⁸F]2** | **20** | PO₄³⁻ | Bu₄NOMs | DMSO | 2.5% |
| **[¹⁸F]2** | **20** | PO₄³⁻ | Bu₄NOMs | MeCN | 4.5% |
| **[¹⁸F]3** | **21** | PO₄³⁻ | Bu₄NOMs | tBuOH/DMSO | 4.6% |
| **[¹⁸F]3** | **22** | PO₄³⁻ | Bu₄NOMs | tBuOH/DMSO | 11.5±3.5%^{a} |
| **[¹⁸F]3** | **22** | CO₃²⁻ | Et₄NHCO₃ | MeCN | 0% |

| | | | | | |
|---|---|---|---|---|---|
| ^{a}Reaction performed in triplicates, Reaction time: 15 min, reaction temperature: 85°C, precursor amount: 9 µmol. | | | | | |

The results show that the only combination of conditions that yield relevant amounts of ¹⁸F-labeled tetrazine is the low-base conditions with PO₄³⁻preconditioned QMA eluted with Bu₄NOMs and performing the reaction in a mixture of tBuOH with either MeCN, DMSO or a similar aprotic polar organic solvent. The synthesis was performed automated on a Scansys synthesis module giving 22.8±3.9% and 5.2±2.8% RCY for [¹⁸F]**2** and [¹⁸F]**3** respecively.

### Example 6: Labelling conditions for [¹⁸F]2 and [¹⁸F]3

[¹⁸F]**2** and [¹⁸F]**3**: Synthesis was performed on a Scansys research module (Scansys Laboratorieteknik). The aqueous [¹⁸F]fluoride solution received from the cyclotron was passed through a preconditioned anion exchange resin (Sep-Pak Light QMA cartridge). The QMA was preconditioned by flushing it with 10 mL 0.5 M K₃PO₄ and washing it with 10 mL H₂O afterwards. [¹⁸F]F⁻ was eluted from the QMA into a 4 mL v-shaped vial with 1 mL Bu₄NOMs (20 mM) dissolved in MeOH. The eluate was dried at 100 °C for 5 min under N₂-flow. The precursor (3.1 umol) was dissolved in a mixture of tBuOH/DMSO (5:1, 1 mL). The solution was added to the dried fluoride solution an allowed to react for 5 min at 100 °C. The reaction was cooled to 50 °C with air before addition of 2 mL 0.1% TFA in H₂O.

Purification for [¹⁸F]**2**: Semipreparative HPLC, with a C-18 column (Luna^{®} 5µm C18(2) 100Å, 250 x 10 mm) using an isocratic method (45% MeCN with 0.1% TFA in H₂O (v/v), 3.0 mL/min, rt: 16.5 min. Fraction containg the product was collected in 60 mL H₂O and applied to a Sep-Pak C18 Plus and subsequently formulated by eluting with 1 mL EtOH into 9 mL sterile water for injection. Analytical HPLC for manual and automated synthesis according to the general procedure. UV detection at 254 nm, rt: 9.00 min, TLC eluent: Heptane/EtOAc, 66:34, rf: 0.6. Aₘ: 34.1 ± 14.0 GBqlµmol.

Purification for [¹⁸F]**3**: Semipreparative HPLC, with a C-18 column (Luna^{®} 5µm C18(2) 100Å, 250 x 10 mm) using an isocratic method (45% MeCN with 0.1% TFA in H₂O (v/v), 3.0 mL/min, rt: 18.5 min. Fraction containing the product was collected in 60 mL H₂O and applied to a Sep-Pak C18 Plus and subsequently formulated by eluting with 1 mL EtOH into 9 mL sterile water for injection. Analytical HPLC for manual and automated synthesis according to the general procedure. UV detection at 254 nm, rt (radio): 9.25 min, TLC eluent: Heptane/EtOAc, 50:50, rf: 0.5. Aₘ: 96.3 ± 5.8 MBqlµmol.

### Example 7:

To test the applicability of high reactive tetrazines that were aliphatic 18F labelled, 15 compounds were designed see table 7 below. The compounds were synthesized according to example 8 and their properties evaluated by measuring their reaction kinetics and tumor blocking effect. Results are summarized in the table 7 below.

### Reaction Kinetics Measurement

Reaction kinetics of tetrazines with standard TCO were determined using pseudo-first-order measurements with an excess of the TCO compound in PBS (pH = 7.4) at 37.0 ± 0.1 °C following the decrease of tetrazine absorbance at 535 nm. Measurements were performed in triplicates using an SX20 stopped-flow photometer (Applied Photophysics, UK) equipped with a 535 nm LED light source. Data analysis was performed using Prism 6 (Graphpad) to determine the observed rate constants which were converted into secondorder rate constants through dividing by the TCO concentration. Concentrations, observed rate constants and calculated second-order rate constants are shown in the table 7 below.

### Reactivity of the Tz Library

Second-order rate constants for the reaction with TCO in PBS at 37 °C were determined by pseudo-first order measurements in a SX20 stopped flow photometer (Applied Photophysics). All tetrazines displayed rate constant values >55.000 M⁻¹s⁻¹ and are as such within the limit of suggested values that increase the chance of a tetrazine to be used for pretargeted *in vivo* chemistry (Table 7).

### Blocking assay and ex vivo studies

### Establishing tumor xenografts in mice

All animal studies were approved by the Danish Animal Welfare Council, ministry of Justice. Five weeks old female nude BALB/c mice (Charles River, Sulzfeld, Germany) were allowed to acclimatize for one week with access to water and chow ad libitum. Human colon cancer cell line (LS174T; obtained from ATCC) was cultured in minimum essential medium (MEM) supplemented with 10% fetal bovine serum, 1% L-glutamine, 1% sodium pyruvate, 1% non-essential amino acids, and 1% penicillin-streptomycin at 37 °C and 5% CO₂. Cells were harvested by trypsinization at a confluence of 70-90% and subcutaneous tumors were established in the left flank of the 6 weeks old animals by inoculation of ~ 5 x 10⁶ LS174T cells resuspended in sterile PBS (100 µL) and allowed to grow for 7-10 days. Tumors were measured using a caliper and the volume calculated using the formula volume = ½ (length x width²).

### Blocking experiments

Tumor-bearing animals were matched in groups based on their tumor volume (tumor volumes of ∼ 100-300 mm³, *n* = 3 in each group) and were administered 100 µg/100 µL of CC49-TCO (100 µg/100 µL, ∼7 TCO/mAb) per mouse. After 3 days, animals were injected with non-radioactive Tz (10-20 nmol). After 1 h, they were administered with [¹¹¹In]**Tz-Tg** (∼13 MBq/100 µL, 1-2 nmol) via the tail vein. [¹¹¹In]Tz-Tg was obtained and radiolabeled as previously described in Börjesson, P. K.; Jauw, Y. W.; de Bree, R.; Roos, J. C.; Castelijns, J. A.; Leemans, C. R.; van Dongen, G. A.; Boellaard, R. Radiation dosimetry of 89Zr-labeled chimeric monoclonal antibody U36 as used for immuno-PET in head and neck cancer patients. J Nucl Med 2009, 50, 1828-36. The mice were euthanized after 22 h and tumor, blood, heart, lung, liver, spleen, kidney, and muscle were resected. All tissues were weighted, and the radioactivity measured in a gamma counter (Wizard2, Perkin Elmer) and data was corrected for decay, tissue weight and injected amount of radioactivity. Figure 3 illustrate the workflow of the blocking assay.

The following table summarize the structural scaffolds, calculated physicochemical properties (TPSA, clog*D*_{7.4}), measured second-order rate constants for the IEDDA reaction with TCO, and blocking efficiencies of all investigated tetrazine derivatives.

**Table 7**

| Tetrazine^{a} | Side Chain Position | R₁ | R₂ | ClogD_{7.4}^{b} | TPSA^{c} | Rate constant (M⁻¹ s⁻¹)^{d} | Normalized Tumor blocking effect ^{g} |
|---|---|---|---|---|---|---|---|
| 9^{a} | 5 | -CH2CH2F | -F | -2.67 | 92.10 | 87722 | 76 |
| 10^{a} | 5 | -CH2CH2F | -H | -2.73 | 92.10 | 62464 | 75 |
| 11^{a} | 4 | -CH₂CH₂F | -F | -2.77 | 92.10 | 74592 | 69 |
| 12^{a} | 4 | -CH2CH2F | -H | -2.73 | 92.10 | 54999 | 69 |
| uuu | 4 | -CH₂COOH | -OH | -7.43 | 149.63 | n.d. | n.d. |
| iii | 4 | -CH₂COOH | -OMe | -7.24 | 138.63 | n.d. | n.d. |
| 13^{a} | 4 | -CH2COOH | -OCH2CH2F | -6.83 | 138.63 | 68478 | 98 |
| Tz-Tg | - | - | - | -4.13^{f} | 358.03 | 73742 | 99 |
| | 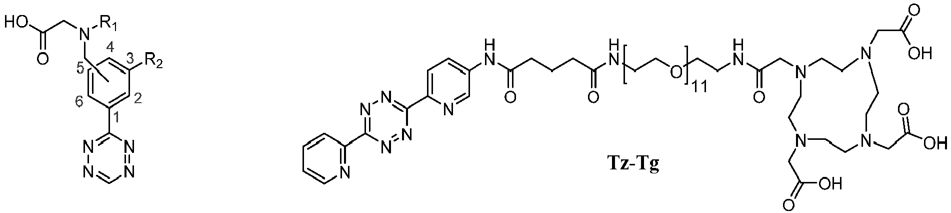 | | | | | | |

| | | | | | | | |
|---|---|---|---|---|---|---|---|
| ^{a} The compounds were obtained as trifluoroacetate salt. ^{b} Calculated distribution coefficient at physiological pH (7.4) in Chemicalize software. ^{c} Calculated in Chemicalize software. ^{d} Second order rate constants are determined with TCO at 37 °C in PBS (reaction kinetic measurement) ^{e} The compound was employed as a reference. ^{f} Calculated as chelated to a trivalent cation. n.d. = not determined. ^{g} Data represent mean from n = 3 mice/group | | | | | | | |

### Example 8 Reference Compounds and Precursors

### Synthesis of compound: 2-Fluoroethyl benzoate (1)

Benzoic acid (47 mg, 0.38 mmol 1.00 equiv) was mixed with Bu₄NOH (40%wt in MeOH, 408 µL, 0.57 mmol, 1.5 equiv), H₂O and toluene 2 mL respectively was added and the mixture was stirred for 5 min before it was evaporated. The reaction was dissolved in 5 mL dry MeCN and cooled to 0°C. 2-Fluoroethyl tosylate (100 mg, 0.46 mmol, 1.2 equiv) was dissolved in 1 mL of dry MeCN and added to the reaction dropwise under argon. The reaction was slowly heated to room temperature and left stirring for 3.5 hours. The reaction mixture was diluted with EtOAc (20 mL) and washed with Brine (20 mL) and water (2x20mL). The organic phase was dried over Na₂SO₄ and concentrated *in vacuo.* Purification by flash chromatography (n-Heptane/EtOAc 70:30) yielded 2-fluoroethyl benzoate (36 mg, 0.21 mmol, 56%) as a clear liquid. ¹H NMR (400 MHz, CDCl₃-*d*) δ 8.16 - 8.06 (m, 2H), 7.64 - 7.53 (m, 1H), 7.51 - 7.40 (m, 2H), 4.85 - 4.75 (m, 1H), 4.72 - 4.65 (m, 1H), 4.63-4.58 (m, 1H), 4.56 -4.50 (m, 1H). ¹³C NMR (101 MHz, CDCl₃) δ 166.53, 133.38, 129.91, 129.85, 128.57, 82.43, 80.73, 64.08, 63.88. ¹⁹F NMR (376 MHz, CDCl₃) δ -224.53; HRMS (MALDI-TOF) calculated for C₉H₉FO₂⁺ [M+H]+: 169.0659, found: 169.0653

### Synthesis of compound: 4-{[(2-Fluoroethoxy)methyl]-1,2,4,5-tetrazin-3-yl}benzene (2)

### 4-[(2-Hydroxyethoxy)methyl]benzonitrile

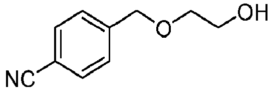

NaH (90% wt, 445 mg, 16.83 mmol, 1.10 equiv) was suspended in dry THF (10 mL) and ethylene glycol (8.56 mL, 153.0 mmol, 10.00 equiv) was added dropwise under argon at 0°C. The solution was left for 30 min at 0°C before dropwise addition of 4-(Bromomethyl)benzonitrile (3000 mg, 15.30 mmol, 1.00 equiv) in dry THF (30 mL) under argon at 0°C. The reaction was slowly heated to room temperature and left for 3 hours. The reaction was queched by adding EtOAc (50 mL) and the crude was washed with NH4CI (sat, 50mL x2) and water (50 mL). The organic phase was dried over Na2SO4 and concentrated in vacuo. Purification by flash chromatography (n-heptane/EtOAc, 40:60) yielded 2662 mg (98%) of 4-[(2-Hydroxyethoxy)methyl]benzonitrile as a colorless liquid. ¹H NMR (600 MHz,CDCl₃) δ 7.67 - 7.62 (m, 2H), 7.48 - 7.42 (m, 2H), 4.62 (s, 2H), 3.82-3.77 (m, 2H), 3.66-3.61 (m, 2H); ¹³C NMR (151 MHz, CDCl₃) δ 143.71, 132.42, 127.91, 118.89, 111.62, 72.40, 72.09, 62.00. HRMS (MALDI-TOF) calculated for C10H11NO2 [M+Na]+: 200.0682, found: 200.0687.

### 4-{[(2-Hydroxyethoxy)methyl]-1,2,4,5-tetrazin-3-yl}benzene

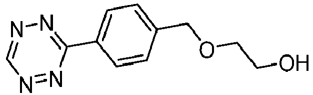

4-[(2-Hydroxyethoxy)methyl]benzonitrile (250 mg, 1.41 mmol, 1.00 equiv), CH₂Cl₂ (0.109 mL, 1.69 mmol, 1.20 equiv), sulfur (91 mg, 0.35 mmol, 0.25 equiv) and ethanol (3.0 mL) were mixed together in a microwave reaction vial. Hydrazine monohydrate (0.550 mL, 14.11 mmol, 10.00 equiv) was added dropwise with stirring. The vessel was sealed and the reaction mixture was heated to 50 °C for 24 hours. The reaction was diluted with 3 ml of CH₂Cl₂ and sodium nitrite (974 mg, 14.11 mmol, 10.00 equiv) in 20 ml of H₂O was added dropwise to the mixture under cooling. Excess acetic acid (2.5 mL) was then added slowly during which the solution turned bright red in color. The reaction mixture was extracted with DCM 3x100 mL. The organic phase was dried over MgSO₄ and concentrated *in vacuo.* The resulting residue was purified using flash chromatography (n-heptane/EtOAc, 60:40) and recrystallization in n-heptane/EtOAc to form 204 mg (62%) of 4-{[(2-hydroxyethoxy)methyl]-1,2,4,5-tetrazin-3-yl}benzene as a pink solid. ¹H NMR (600 MHz, CDCl₃) δ 10.21 (s, 1H), 8.64 - 8.59 (m, 2H), 7.58 (d, *J* = 8.1 Hz, 2H), 4.70 (s, 2H), 3.85 - 3.80 (m, 2H), 3.70 - 3.66 (m, 2H), ¹³C NMR (151 MHz, CDCl₃) δ 166.48, 157.95, 143.81, 131.10, 128.62, 128.38, 72.79, 71.99, 62.09; HRMS (MALDI-TOF) calculated for (*in situ* reduced to dihydro tetrazine) C₁₁H₁₄N₄O₂ [M+H]⁺: 235.1189, found: 235.1192.

### 4-{[(2-Fluoroethoxy)methyl]-1,2,4,5-tetrazin-3-yl}benzene

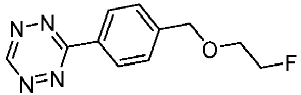

4-{[(2-Hydroxyethoxy)methyl]-1,2,4,5-tetrazin-3-yl}benzene (30 mg, 0.13 mmol, 1.00 equiv) was dissolved in 3 mL dry THF and PBSF (78 uL, 0.26 mmol, 2.00 equiv) and DIPEA (135 uL, 0.78 mmol, 6.00 equiv) was added. Et₃N*3HF (42 uL, 0.26 mmol, 2.00 equiv) was dissolved in 1 mL THF and added dropwise. The reaction was left at room temperature for 18 hours. The reaction was diluted with 10 mL DCM and washed with NH₄Cl (sat). The aqueous phase was extracted with DCM 2x10 mL and the combined organic layers were dried over MgSO₄ and concentrated *in vacuo.* Purification by flash chromatography (n-heptane/EtOAc, 50:50) yielded 4 mg (13%) of 4-{[(2-fluoroethoxy)methyl]-1,2,4,5-tetrazin-3-yl}benzene as a pink. ¹H NMR (400 MHz, CDCl₃) δ 10.21 (s, 1H), 8.68-8.57 (m, 2H), 7.60 (d, *J* = 8.1 Hz, 2H), 4.77-4.67 (m, 3H), 4.62 - 4.55 (m, 1H), 3.89 - 3.83 (m, 1H), 3.80 - 3.75 (m, 1H); ¹⁹F NMR (376 MHz, CDCl₃) δ -222.93; ¹³C NMR (101 MHz, CDCl₃) δ 166.51, 157.96, 143.70, 131.10, 128.62, 128.34, 127.86, 84.09, 82.40, 72.89, 69.97, 69.78; HRMS (MALDI-TOF) calculated for C₁₁H₁₁FN₄O [M+H]⁺: 235.0989, found: 235.0995.

### Synthesis of compound:2-Fluoro-4-(1,2,4,5-tetrazin-3-yl)-2-fluoroethyl-benzoate (3)

### 2-Fluoro-4-(1,2,4,5-tetrazin-3-yl)benzoic acid

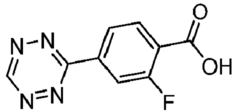

The compound was obtained from 2-fluoro-4-cyanobenzoic acid (0.99 g, 6.00 mmol) as described for compound 2 to give 0.41 g (31%) of the desired product as a pink solid. ¹H-NMR (MeOD, 600 MHz): 10.44 (s, 1H), 8.50 (dd, J = 1.6, 8.1 Hz), 8.40 (dd, J = 1.6, 11.5 Hz, 1H), 8.20 (t, J = 7.74 Hz, 1H); ¹⁹F NMR (376 MHz, MeOD) δ -110.27; ¹³C NMR (151 MHz, MeOD) δ 165.12, 165.09, 165.05, 165.03, 162.96, 161.24, 158.28, 158.21, 138.00, 137.94, 132.85, 132.84, 123.11, 123.08, 122.88, 122.81, 115.91, 115.74; HRMS (MALDI-TOF) calculated for C₉H₅FN₄O₂ [M-H]⁻: 219.0323, found: 219.0318.

### 2-Fluoro-4-(1,2,4,5-tetrazin-3-yl)-2-fluoroethyl-benzoate

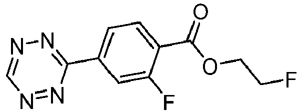

2-Fluoro-4-(1,2,4,5-tetrazin-3-yl)benzoic acid (10 mg, 0.05 mmol, 1.00 equiv) and 2-Fluoroethyl4-methylbenzenesulfonate (30 mg, 0.14 mmol, 3.00 equiv) was dissolved in 2 ml dry DMF and DIPEA (20 uL, 0.10 mmol, 2.00 equiv) was added. The reaction was left at 100°C over night. The reaction was diluted with Et2O (15 mL) and washed with NH₄Cl and H₂O (2x10 mL). The organic phase was dried over MgSO₄ and concentrated *in vacuo.* Purification by flash chromatography (toluene, 100) yielded 6.5 mg (45%) of 2-fluoro-4-(1,2,4,5-tetrazin-3-yl)-2-fluoroethyl-benzoate as a pink liquid that solidified over time. ¹H NMR (400 MHz, CDCl₃) δ 10.31 (s, 1H), 8.51 (dd, *J* = 8.2, 1.6 Hz, 1H), 8.45 (dd, *J* = 11.2, 1.6 Hz, 1H), 8.21 (dd, *J* = 8.2, 7.1 Hz, 1H), 4.88 - 4.79 (m, 1H), 4.77 - 4.65 (m, 2H), 4.65 - 4.57 (m, 1H); ¹⁹F NMR (376 MHz, CDCl₃) δ -106.87, -224.63; ¹³C NMR (101 MHz, CDCl₃) δ 165.23, 163.79, 163.49, 158.34, 133.38, 123.69, 123.65, 122.26, 117.08, 116.83, 82.09, 80.38, 64.76, 64.55. HRMS (MALDI-TOF) calculated for (*in situ* reduced to dihydro tetrazine) C₁₁H₁₀F₂N₄O₂ [M+H]⁺: 269.0844, found: 269.0848.

### Synthesis of compound:2-Fluoroethyl 4-(1,2,4,5-tetrazin-3-yl)benzoate (4)

### 4-(1,2,4,5-tetrazin-3-yl)benzoic acid

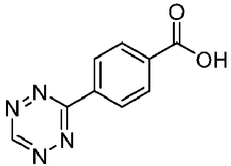

The compound was obtained from 4-cyanobenzoic acid (0.3 g, 2.00 mmol) as described for compound 2 to give 0.08 g (20%) of the desired product as a pink solid. Rf = 0.31 (CH₂Cl₂/MeOH 95/5); ¹H NMR (400 MHz, DMSO) δ 13.32 (s, 1H), 10.66 (s, 1H), 8.62 (d, *J* = 8.5 Hz, 2H), 8.22 (d, *J* = 8.5 Hz, 2H); ¹³C NMR (101 MHz, DMSO) δ 166.67, 165.08, 158.24, 135.70, 134.32, 130.20, 127.97.

### 2-Fluoroethyl 4-(1,2,4,5-tetrazin-3-yl)benzoate

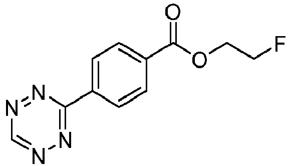

The compound was obtained from 4-(1,2,4,5-tetrazin-3-yl)benzoic acid (0.04 g, 0.20 mmol) as described for compound 2 to give 0.04 g (81%) of the desired product as a pink solid. Rf = 0.23 (n-Heptane/EtOAc 80/20); ¹H NMR (600 MHz, CDCl₃) δ 10.21 (s, 1H), 8.65 (d, *J* = 8.6 Hz, 2H), 8.23 (d, *J* = 8.5 Hz, 2H), 4.89 - 4.72 (m, 1H), 4.69 - 4.64 (m, 1H), 4.63 - 4.57 (m, 1H), 4.57 - 4.50 (m, 1H); ¹³C NMR (101 MHz, CDCl₃) δ 165.92, 165.50, 157.99, 135.75, 133.63, 130.58, 128.27, 81.26 (d, *J* = 171.1 Hz), 64.30 (d, *J* = 20.1 Hz).

### Synthesis of compound:3-(4-(2-Fluoroethoxy)phenyl)-1,2,4,5-tetrazine (5)

### 4-(2-fluoroethoxy)benzonitrile

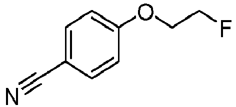

To a solution of 4-hydroxybenzonitrile (0.6 g, 5.00 mmol) and K₂CO₃ (1.38 g, 10.00 mmol) in CH₃CN (20 mL) was added 1-fluoro-2-iodoethane (1.04 g, 6.00 mmol). The reaction was refluxed for 12 h and then concentrated under reduced pressure. The resulting mixture was diluted with water (50 mL), extracted with EtOAc (3 x 50 mL), washed with brine (50 mL), dried over MgSO₄, filtered and concentrated under reduced pressure. Purification by flash chromatography (n-Heptane/EtOAc 80/20) afforded 0.8 g (97%) of the desired compound as a yellow oil. Rf = 0.37 (n-Heptane/EtOAc 70/30); ¹H NMR (400 MHz, CDCl₃) δ 7.62 (d, *J* = 8.9 Hz, 1H), 7.01 (d, *J* = 8.9 Hz, 1H), 5.05 - 4.79 (m, 1H), 4.77 - 4.71 (m, 1H), 4.36 - 4.29 (m, 1H), 4.29 - 4.22 (m, 1H).

### 3-(4-(2-Fluoroethoxy)phenyl)-1,2,4,5-tetrazine

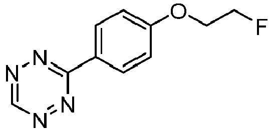

The compound was obtained from 4-(2-fluoroethoxy)benzonitrile (0.73 g, 4.42 mmol) as describedfor compound 2. Purification by flash chromatography (n-Heptane/EtOAc 90/10) afforded 0.36 g (37%) of the desired compound as a red oil. Rf = 0.33 (n-Heptane/EtOAc 80/20); ¹H NMR (400 MHz, CDCl₃) δ 10.07 (s, 1H), 8.53 (d, *J* = 8.9 Hz, 2H), 7.06 (d, *J* = 8.9 Hz, 2H), 4.89 - 4.78 (m, 1H), 4.74 - 4.62 (m, 1H), 4.43 - 4.29 (m, 1H), 4.27 - 4.16 (m, 1H); ¹³C NMR (101 MHz, CDCl₃) δ 166.06, 162.56, 157.40, 130.26, 124.56, 115.38, 81.63 (d, *J* = 171.5 Hz), 67.29 (d, *J* = 20.6 Hz).

### Synthesis of compound:3-(5-((2-Fluoroethoxy)methyl)pyridin-2-yl)-6-(pyridin-2-yl)-1,2,4,5-tetrazine (6)

### 5-((2-Hydroxyethoxy)methyl)picolinonitrile

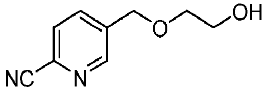

The Compound was obtained from 5-(bromomethyl)picolinonitrile (1 .00 g, 5.07 mmol) as described for compound 2 to give 0.55 g (61%) of the desired product as a pink solid. Rf = 0.31 (Heptame/EtOAc 30/70); ¹H NMR (400 MHz, CDCl₃) δ 8.60 (d, *J* = 2.2 Hz, 1H), 7.82 (dd, *J* = 8.1, 2.2 Hz, 1H), 7.95 - 7.32 (m, 1H), 4.61 (s, 2H), 3.74 (dd, *J* = 5.4, 3.8 Hz, 2H), 3.61 (dd, *J* = 5.4, 3.8 Hz, 2H); ¹³C NMR (101 MHz, CDCl₃) δ 149.94, 138.24, 135.89, 132.41, 128.30, 117.20, 72.47, 69.77, 61.51.

### 2-((6-(6-(Pyridin-2-yl)-1,2,4,5-tetrazin-3-yl)pyridin-3-yl)methoxy)ethan-1-ol

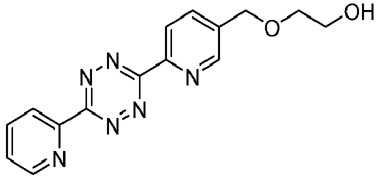

5-((2-Hydroxyethoxy)methyl)picolinonitrile (0.55 g, 3.08 mmol), 2-cyanopyridine (1.6 g, 15.43 mmol) and sulfur (0.2 g, 0.77 mmol) were suspended in EtOH (5 mL), followed by the addition of hydrazine hydrate (2.26 mL, 43.3 mmol). The reaction was heated to 90 °C for 2 h. The mixture was cooled to room temperature and the formed precipitate was removed by filtration. Water (20 mL) and a solution of NaNO₂ (4.26 g, 61.73 mmol) in water 30 mL were added and the mixture was cautiously acidified to pH 2 by addition of AcOH. The mixture was extracted with DCM and the combined organic layer was washed with water and brine, dried over MgSO₄ and concentrated. The residue was purified by flash column chromatography (CH₂Cl₂/MeOH 95/5) to afford 0.4 g (42%) of the desired compound as a pink solid. Rf = 0.21 (CH₂Cl₂/MeOH 95/5); ¹H NMR (400 MHz, CDCl₃) δ 9.03 - 8.94 (m, 1H), 8.90 (d, *J* = 2.1 Hz, 1H), 8.79 - 8.65 (m, 2H), 8.00 (ddd, *J* = 7.9, 5.5, 2.1 Hz, 3H), 7.57 (ddd, *J* = 7.6, 4.7, 1.2 Hz, 1H), 4.74 (s, 2H), 3.88 - 3.78 (m, 2H), 3.70 (dd, *J* = 5.3, 3.9 Hz, 2H), 2.76 (s, 1H); ¹³C NMR (101 MHz, CDCl₃) δ 163.79, 163.69, 150.99, 150.03, 149.30, 137.50, 137.21, 136.47, 126.59, 124.50, 124.25, 72.24, 70.26, 61.803-(5-((2-fluoroethoxy)methyl)pyridin-2-yl)-6-(pyridin-2-yl)-1,2,4,5-tetrazine

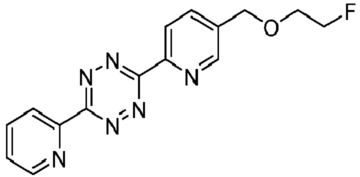

The compound was obtained from 2-((6-(6-(Pyridin-2-yl)-1,2,4,5-tetrazin-3-yl)pyridin-3-yl)methoxy)ethan-1-ol (0.1 g, 0.33 mmol) as described for compound 2to give 0.075 g (75%) of the desired product as a pink solid. Rf = 0.45 (CH₂Cl₂/MeOH 95/5); ¹H NMR (400 MHz, CDCl₃) δ 9.05 - 8.80 (m, 2H), 8.78 - 8.58 (m, 2H), 8.21 - 7.86 (m, 2H), 7.51 (ddd, *J* = 7.6, 4.7, 1.2 Hz, 1H), 4.71 (s, 2H), 4.65 - 4.58 (m, 1H), 4.57 - 4.42 (m, 1H), 3.97 - 3.78 (m, 1H), 3.77 - 3.68 (m, 1H); ¹³C NMR (101 MHz, CDCl₃) δ 163.82, 163.73, 150.98, 150.03, 149.97, 149.36, 137.50, 137.03, 136.43, 126.60, 124.48, 124.26, 82.98 (d, *J* = 169.5 Hz), 70.35, 70.00 (d, *J* = 19.6 Hz).

### Synthesis of compound 2-Fluoroethyl 6-(6-(pyridin-2-yl)-1,2,4,5-tetrazin-3-yl)nicotinate (7)

### 6-(6-(Pyridin-2-yl)-1,2,4,5-tetrazin-3-yl)nicotinic acid

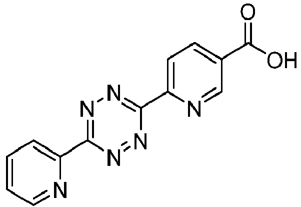

The compound was obtained from 6-cyanonicotinic acid (1.0 g, 6.75 mmol) as described for compound 6 to give 0.52 g (27%) of the desired product as a pink solid. Rf = 0.21 (CH₂Cl₂/MeOH 95/5 + 0.1% AcOH); ¹H NMR (400 MHz, DMSO) δ 9.37 (d, *J* = 2.1 Hz, 1H), 8.96 (q, *J* = 1.6 Hz, 1H), 8.71 (d, *J* = 8.1 Hz, 1H), 8.64 (d, *J* = 7.9 Hz, 1H), 8.58 (dd, *J* = 8.1, 2.1 Hz, 1H), 8.18 (td, *J* = 7.8, 1.8 Hz, 1H), 7.95 - 7.67 (m, 1H); ¹³C NMR (101 MHz, DMSO) δ 165.74, 163.21, 162.97, 153.13, 151.01, 150.70, 149.96, 138.66, 137.86, 128.69, 126.81, 124.53, 124.12.

### 2-Fluoroethyl 6-(6-(pyridin-2-yl)-1,2,4,5-tetrazin-3-yl)nicotinate

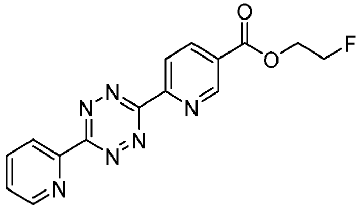

The compound was obtained from 6-(6-(pyridin-2-yl)-1,2,4,5-tetrazin-3-yl)nicotinic acid (0.05 g, 0.18 mmol) as described for compound 3to give 0.55 g (95%) of the desired product as a pink solid. Rf = 0.41 (Heptane/EtOAc 20/80); ¹H NMR (400 MHz, CDCl₃) δ 9.56 (dd, *J* = 2.2, 0.9 Hz, 1H), 9.00 (ddd, *J* = 4.8, 1.8, 0.9 Hz, 1H), 8.85 (dd, *J* = 8.2, 0.9 Hz, 1H), 8.77 (dt, *J* = 7.9, 1.1 Hz, 1H), 8.02 (td, *J* = 7.8, 1.8 Hz, 1H), 7.60 (ddd, *J* = 7.7, 4.7, 1.2 Hz, 1H), 4.88 - 4.79 (m, 1H), 4.72 (td, *J* = 5.9, 3.6 Hz, 2H), 4.64 (dd, *J* = 5.0, 3.0 Hz, 1H); ¹³C NMR (101 MHz, CDCl₃) δ 164.34, 163.84, 163.50, 151.97, 149.86, 137.55, 127.81, 124.78, 123.95, 81.04 (d, *J* = 171.5 Hz), 64.62 (d, *J* = 20.3 Hz).

### Synthesis of compound:3-(5-(2-Fluoroethoxy)pyridin-2-yl)-6-(pyridin-2-yl)-1,2,4,5-tetrazine (8)

### 5-(2-Fluoroethoxy)picolinonitrile

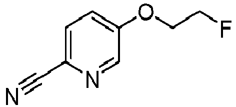

The compound was obtained from 5-hydroxypicolinonitrile (0.5 g, 4.16 mmol) as described for compound 4 to give 0.56 g (81%) of the desired product as a pink solid. Rf = 0.34 (Heptane/EtOAc 70/30); ¹H NMR (400 MHz, CDCl₃) δ 8.34 (d, *J* = 2.9 Hz, 1H), 7.59 (d, *J* = 8.6 Hz, 1H), 7.27 - 7.20 (m, 1H), 4.83 - 4.76 (m, 1H), 4.71 - 4.64 (m, 1H), 4.35 - 4.28 (m, 1H), 4.28 - 4.21 (m, 1H); ¹³C NMR (101 MHz, CDCl₃) δ 155.04, 138.50, 127.74, 123.96, 118.83, 115.55, 79.47 (d, *J* = 172.4 Hz), 66.05 (d, *J* = 20.4 Hz).

### 3-(5-(2-Fluoroethoxy)pyridin-2-yl)-6-(pyridin-2-yl)-1,2,4,5-tetrazine

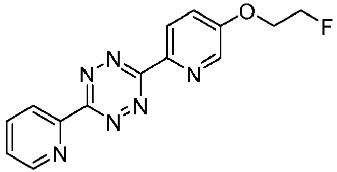

The compound was obtained from 5-(2-fluoroethoxy)picolinonitrile (0.55 g, 3.31 mmol) as described for compound 6followed by purification by preparative HPLC to give 0.04 g (4%) of the desired product as a pink solid. Rf = 0.32 (CH₂Cl₂/MeOH 97/3); ¹H NMR (400 MHz, CDCl₃) δ 8.94 (dt, *J* = 4.7, 1.4 Hz, 1H), 8.70 (d, *J* = 8.5 Hz, 2H), 8.65 (d, *J* = 2.9 Hz, 1H), 8.00 (td, *J* = 7.8, 1.7 Hz, 1H), 7.57 (ddd, *J* = 7.7, 4.8, 1.2 Hz, 1H), 7.46 (dd, *J* = 8.8, 2.9 Hz, 1H), 4.88 - 4.81 (m, 1H), 4.76 - 4.69 (m, 1H), 4.44 - 4.38 (m, 1H), 4.38 - 4.25 (m, 1H); ¹³C NMR (101 MHz, CDCl₃) δ 163.26, 163.21, 157.38, 150.46, 149.69, 142.33, 139.43, 138.19, 126.84, 125.92 124.58, 121.99, 81.39 (d, *J* = 172.7 Hz), 67.99 (d, *J* = 20.4 Hz).

### Synthesis of compound:7 N-(carboxymethyl)-2-fluoro-N-(3-fluoro-5-(1,2,4,5-tetrazin-3-yl)benzyl)ethanami- nium 2,2,2-trifluoroacetate (9)

### Tert-butyl 2-((3-cyano-5-fluorobenzyl)amino)acetate

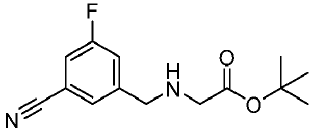

To a solution of 3-(bromomethyl)-5-fluorobenzonitrile (3.34 g, 15.60 mmol) in CH₃CN (40 mL) was added K₂CO₃ (10.78 g, 78.02 mmol) and glycine tert-butyl ester hydrochloride (7.85 g, 46.81 mmol). The reaction mixture was stirred at room temperature overnight. The solvent was removed in vacuo, and the resulting mixture was diluted with water (20 mL), extracted with EtOAc (2 x 25 mL), washed with brine (30 mL), dried over MgSO₄, filtered and concentrated under reduced pressure. Purification by flash chromatography (n-Heptane/EtOAc 90/10) afforded 3.52 g (85%) of the desired compound as a colorless oil. Rf = 0.23 (n-Heptane/EtOAc 80/20); ¹H NMR (400 MHz, CDCl₃) δ 7.46 (d, *J* = 1.5 Hz, 1H), 7.35 (dt, *J* = 9.3, 1.8 Hz, 1H), 7.25 - 7.18 (m, 1H), 3.83 (s, 2H), 3.28 (s, 2H), 1.92 (s, 1H), 1.47 (s, 9H); ¹³C NMR (101 MHz, CDCl₃) δ 171.35, 162.36 (d, *J* = 250.1 Hz), 144.68 (d, *J* = 7.4 Hz), 127.53 (d, *J* = *3.2* Hz), 119.97 (d, *J* = 21.3 Hz), 117.66 (d, *J* = 24.8 Hz), 117.63 (d, *J* = 3.3 Hz), 113.66 (d, *J* = 9.7 Hz), 81.60, 51.96 (d, *J* = 1.8 Hz), 50.82, 28.12.

### Tert-butyl 2-((3-cyano-5-fluorobenzyl)(2-fluoroethyl)amino)acetate

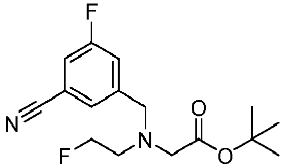

To a solution of *tert*-butyl 2-((3-cyano-5-fluorobenzyl)amino)acetate (1.400 g, 5.23 mmol) and K₂CO₃ (1.83 g, 13.24 mmol) in CH₃CN (40 mL) was added 1-fluoro-2-iodoethane (1.38 g, 7.94 mmol). The reaction was refluxed for 24 h. The solvent was removed under reduced pressure, water (30 mL) was added, and the mixture was extracted with EtOAc (3 x 25 mL). The organic layer was dried over MgSO₄, filtered and evaporated in vacuo to give an oil. The residue was purified by flash column chromatography (n-Heptane/EtOAc 80/20) to afford 1.51 g (92%) of the desired compound as colorless oil. Rf = 0.30 (n-Heptane/EtOAc 60/40); ¹H NMR (400 MHz, CDCl₃) δ 7.41 (d, *J* = 2.0 Hz, 1H), 7.34 (d, *J* = 9.3 Hz, 1H), 7.19 - 7.12 (m, 1H), 4.51 (q, *J* = 4.3, 3.7 Hz, 1H), 4.39 (q, *J* = 4.3, 3.7 Hz, 1H), 3.87 (s, 2H), 3.27 (s, 2H), 2.99 (q, *J* = 4.3, 3.8 Hz, 1H), 2.95 - 2.84 (m, 1H), 1.40 (s, 9H); ¹³C NMR (101 MHz, CDCl₃) δ 170.17, 162.41 (d, *J* = 250.2 Hz), 144.22, 127.89 (d, *J* = 3.0 Hz), 120.42 (d, *J* = 21.6 Hz), 117.85 (d, *J* = 24.9 Hz), 117.64 (d, *J* = 3.2 Hz), 113.64 (d, *J* = 9.9 Hz), 82.80 (d, *J* = 168.2 Hz), 81.50, 57.55, 55.47, 53.67 (d, *J* = 19.8 Hz), 28.16.

### Tert-butyl 2-((3-fluoro-5-(1,2,4,5-tetrazin-3-yl)benzyl)(2-fluoroethyl)amino)acetate

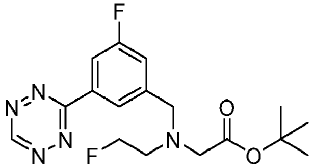

The compound was obtained from *tert*-butyl 2-((3-cyano-5-fluorobenzyl)(2-fluoroethyl)-amino)acetate (1.50 g, 4.83 mmol) as describedfor compound 2. Purification by flash chromatography (n-Heptane/EtOAc 95/5) afforded 0.44 g (25%) of the desired compound as a red oil. Rf = 0.28 (n-Heptane/EtOAc 80/20); ¹H NMR (400 MHz, CDCl₃) δ 10.17 (s, 1H), 8.33 (t, *J* = 1.4 Hz, 1H), 8.18 - 8.06 (m, 1H), 7.40 (dt, *J* = 9.1, 1.9 Hz, 1H), 4.55 (t, *J =* 4.9 Hz, 1H), 4.43 (t, *J* = 4.9 Hz, 1H), 3.97 (s, 2H), 3.33 (s, 2H), 3.05 (t, *J* = 5.0 Hz, 1H), 2.98 (t, *J* = 5.0 Hz, 1H), 1.41 (s, 9H); ¹³C NMR (101 MHz, CDCl₃) δ 170.30, 165.74 (d, *J* = 3.3 Hz), 163.54 (d, *J* = 247.6 Hz), 157.98, 143.55, 133.53 (d, *J* = 8.5 Hz), 123.99 (d, *J* = 2.8 Hz), 120.33 (d, *J* = 21.9 Hz), 114.07 (d, *J* = 24.5 Hz), 82.95 (d, *J* = 168.0 Hz), 81.38, 58.04, 55.54, 53.62 (d, *J* = 20.1 Hz), 28.18.

### N-(carboxymethyl)-2-fluoro-N-(3-fluoro-5-(1,2,4,5-tetrazin-3-yl)benzyl)ethanaminium 2,2,2-trifluoroacetate

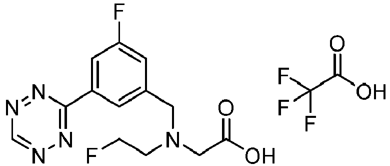

To *tert*-butyl 2-((3-fluoro-5-(1,2,4,5-tetrazin-3-yl)benzyl)(2-fluoroethyl)amino)acetate (0.30 g, 0.86 mmol) in CH₂Cl₂ (5 mL) was added trifluoroacetic acid (5 mL). The reaction was stirred at room temperature for 2 hours. The solvent was then removed under reduced pressure to obtain a pink solid. Purification by preparative HPLC afforded 0.26 g (71%) of the desired compound as a red solid. ¹H NMR (400 MHz, CD₃OD) δ 10.43 (s, 1H), 8.64 (d, *J* = 1.6 Hz, 1H), 8.39 (ddd, *J* = 9.3, 2.5, 1.5 Hz, 1H), 7.69 (dt, *J* = 9.0, 2.1 Hz, 1H), 5.03 - 4.94 (m, 1H), 4.90 - 4.83 (m, 1H), 4.69 (s, 2H), 4.15 (s, 2H), 3.83-3.71 (m, 1H), 3.71 - 3.60 (m, 1H); ¹³C NMR (101 MHz, CD₃OD) δ 167.91, 165.05 (d, *J* = 3.3 Hz), 163.29 (d, *J* = 248.0 Hz), 158.28, 135.34 (d, *J* = 8.6 Hz), 134.32 (d, *J* = 7.7 Hz), 126.16 (d, *J* = 3.1 Hz), 121.83 (d, *J* = 22.8 Hz), 115.73 (d, *J* = 24.3 Hz), 78.98 (d, *J* = 167.7 Hz), 57.82, 54.14 (d, *J* = 19.5 Hz), 52.95; HPLC-MS [M+H]⁺ m/z calc. for [C₁₃H₁₄F₂N₅O₂]⁺: 310.11; Found: 310.14.

### Synthesis of compound:N-(3-(1,2,4,5-tetrazin-3-yl)benzyl)-N-(carboxymethyl)-2-fluoroethanaminium 2,2,2-trifluoroacetate (10)

### Tert-butyl 2-((3-cyanobenzyl)amino)acetate

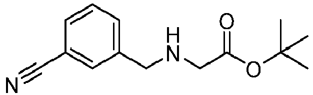

The compound was obtained from 3-bromomethylbenzonitrile (1.80 g, 9.18 mmol) as describedfor compound 9. Purification by flash chromatography (n-Heptane/EtOAc 80/20) afforded 1.32 g (58%) of the desired compound as a colorless oil. Rf = 0.31 (n-Heptane/EtOAc 60/40); ¹H NMR (400 MHz, CDCl₃) δ 7.66 (s, 1H), 7.59 (d, *J* = 7.7 Hz, 1H), 7.54 (d, *J* = 7.7 Hz, 1H), 7.42 (t, *J* = 7.7 Hz, 1H), 3.83 (s, 2H), 3.29 (s, 2H), 1.47 (s, 9H); ¹³C NMR (101 MHz, CDCl₃) δ 171.41, 141.38, 132.62, 131.67, 130.80, 129.16, 118.82, 112.46, 81.44, 52.37, 50.85, 28.10.

### Tert-butyl 2-((3-cyanobenzyl)(2-fluoroethyl)amino)acetate

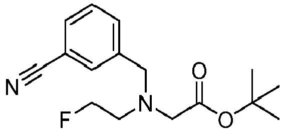

The compound was obtained from *tert*-butyl 2-((3-cyanobenzyl)amino)acetate (1.50 g, 6.09 mmol) as describedfor compound 9. Purification by flash chromatography (n-Heptane/EtOAc 80/20) afforded 1.10 g (62%) of the desired compound as a colorless oil. Rf = 0.62 (n-Heptane/EtOAc 60/40); ¹H NMR (400 MHz, CDCl₃) δ 7.68 (d, *J* = 1.7 Hz, 1H), 7.62 (d, *J* = 7.8 Hz, 1H), 7.54 (dt, *J* = 7.8, 1.5 Hz, 1H), 7.42 (t, *J* = 7.7 Hz, 1H), 4.58 (t, *J* = 4.9 Hz, 1H), 4.46 (t, *J* = 4.9 Hz, 1H), 3.92 (s, 2H), 3.33 (s, 2H), 3.05 (t, *J* = 5.0 Hz, 1H), 2.98 (t, *J* = 5.0 Hz, 1H), 1.47 (s, 9H); ¹³C NMR (101 MHz, CDCl₃) δ 170.36, 140.89, 133.09, 132.12, 130.95, 129.14, 118.88, 112.46, 82.92 (d, *J* = 167.8 Hz), 81.32, 57.87, 55.50, 53.59 (d, *J* = 20.0 Hz), 28.18.

### Tert-butyl 2-((3-(1,2,4,5-tetrazin-3-yl)benzyl)(2-fluoroethyl)amino)acetate

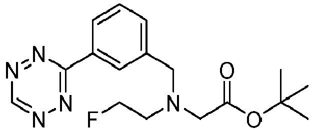

The compound was obtained from *tert*-butyl 2-((3-cyanobenzyl)(2-fluoroethyl)amino)acetate (1.10 g, 3.76 mmol) as described for compound 2 Purification by flash chromatography (n-Heptane/EtOAc 95/5) afforded 0.39 g (30%) of the desired compound as a red oil. Rf = 0.37 (n-Heptane/EtOAc 80/20); ¹H NMR (400 MHz, CDCl₃) δ 10.19 (s, 1H), 8.55 (d, *J* = 1.7 Hz, 1H), 8.48 (dt, *J* = 7.8, 1.5 Hz, 1H), 7.68 (d, *J* = 7.6 Hz, 1H), 7.54 (t, *J* = 7.7 Hz, 1H), 4.59 (t, *J* = 5.0 Hz, 1H), 4.47 (t, *J* = 5.0 Hz, 1H), 4.01 (s, 2H), 3.37 (s, 2H), 3.09 (t, *J* = 5.0 Hz, 1H), 3.03 (t, *J* = 5.0 Hz, 1H), 1.45 (s, 9H); ¹³C NMR (101 MHz, CDCl₃) δ 170.44, 166.47, 157.79, 140.53, 133.67, 131.68, 129.45, 128.53, 127.26, 83.01 (d, *J* = 167.6 Hz), 81.17, 58.36, 55.52, 53.53 (d, *J* = 20.2 Hz), 28.18.

### N-(3-(1,2,4,5-tetrazin-3-yl)benzyl)-N-(carboxymethyl)-2-fluoroethanaminium 2,2,2-trifluoroacetate

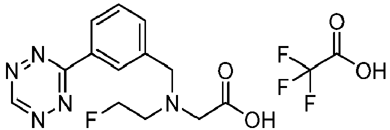

The compound was obtained from *tert*-butyl 2-((3-(1,2,4,5-tetrazin-3-yl)benzyl)(2-fluoroethyl)-amino)acetate (0.30 g, 0.86 mmol) as describedfor compound 9. Purification by preparative HPLC afforded 0.23 g (66%) of the desired compound as a red solid. ¹H NMR (600 MHz, CD₃OD) δ 10.39 (s, 1H), 8.81 (d, *J* = 1.8 Hz, 1H), 8.69 (dt, *J* = 8.0, 1.4 Hz, 1H), 7.89 (dt, *J* = 7.8, 1.4 Hz, 1H), 7.77 (t, *J* = 7.8 Hz, 1H), 5.07 (s, 2H), 5.02 - 4.96 (m, 1H), 4.95 - 4.85 (m, 1H), 4.73 (s, 2H), 3.83 - 3.76 (m, 1H), 3.76 - 3.70 (m, 1H); ¹³C NMR (151 MHz, CD₃OD) δ 167.41, 165.74, 158.11 (d, *J* = 2.1 Hz), 135.28, 133.21, 130.90, 130.49, 130.08, 129.30, 78.67 (d, *J* = 167.9 Hz), 58.38, 54.00 (d, *J* = 19.4 Hz), 52.71; HPLC-MS [M+H]⁺ m/z calc. for [C₁₃H₁₅FN₅O₂]⁺: 292.12; Found: 292.13.

### Synthesis of compound:N-(carboxymethyl)-2-fluoro-N-(2-fluoro-4-(1,2,4,5-tetrazin-3-yl)benzyl)ethanamin-ium 2,2,2-trifluoroacetate (11)

### Tert-butyl 2-((4-cyano-2-fluorobenzyl)amino)acetate

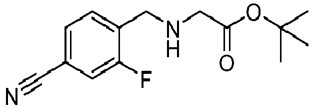

The compound was obtained from 4-(bromomethyl)-3-fluorobenzonitrile (1.00 g, 4.67 mmol) as describedfor compound 9. Purification by flash chromatography (n-Heptane/EtOAc 80/20) afforded 0.52 g (42%) of the desired compound as a colorless oil. Rf = 0.31 (n-Heptane/EtOAc 60/40); ¹H NMR (400 MHz, CDCl₃) δ 7.48 (t, *J* = 7.5 Hz, 1H), 7.32 (dd, *J* = 7.9, 1.5 Hz, 1H), 7.21 (dd, *J* = 9.4, 1.6 Hz, 1H), 3.79 (s, 2H), 3.19 (s, 2H), 1.91 (s, 1H), 1.34 (s, 9H); ¹³C NMR (101 MHz, CDCl₃) δ 171.14, 160.28 (d, *J* = 249.5 Hz), 133.12 (d, *J* = 14.8 Hz), 130.95 (d, *J* = 5.2 Hz), 128.13 (d, *J* = 3.8 Hz), 118.69 (d, *J* = 25.5 Hz), 117.51, 112.03 (d, *J* = 9.6 Hz), 81.23, 50.85, 45.89 (d, *J* = 3.1 Hz).

### Tert-butyl 2-((4-cyano-2-fluorobenzyl)(2-fluoroethyl)amino)acetate

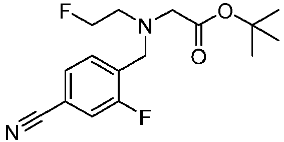

The compound was obtained from *tert*-butyl 2-((4-cyano-2-fluorobenzyl)amino)acetate (1.30 g, 4.92 mmol) as describedfor compound 9. Purification by flash chromatography (n-Heptane/EtOAc 80/20) afforded 0.51 g (33%) of the desired compound as a colorless oil (60/40 unassigned rotamer mixture). Rf = 0.35 (n-Heptane/EtOAc 80/20); ¹H NMR (400 MHz, CDCl₃) δ 7.68 - 7.41 (m, 2H), 7.37 - 7.31 (m, 1H), 4.71 - 4.56 (m, 3H), 4.56 - 4.47 (m, 1H), 4.46 - 4.35 (m, 1H), 4.36 - 4.25 (m, 1H), 3.95 (s, 0.8H), 3.90 (s, 1.2H), 1.44 (s, 43.6H), 1.43 (s, 5.4H); ¹³C NMR (101 MHz, CDCl₃) δ 168.10, 168.02, 160.43 (d, *J* = 249.9 Hz), 160.33 (d, *J* = 250.2 Hz), 156.08, 155.83, 131.82 (d, *J* = 4.8 Hz), 131.22 (d, *J* = 4.7 Hz), 130.28 (d, *J* = 15.0 Hz), 130.20 (d, *J* = 14.9 Hz), 129.50 (d, *J* = 5.3 Hz), 128.39, 128.35, 119.16, 119.08, 118.91, 118.83, 117.41 (d, *J* = 2.8 Hz), 117.36 (d, *J* = 2.9 Hz), 81.48 (d, *J* = 170.8 Hz), 65.07 (d, *J* = 19.8 Hz), 46.18 (d, *J* = 3.5 Hz), 45.63 (d, *J* = 3.7 Hz).

### Tert-butyl 2-((2-fluoro-4-(1,2,4,5-tetrazin-3-yl)benzyl)(2-fluoroethyl)amino)acetate

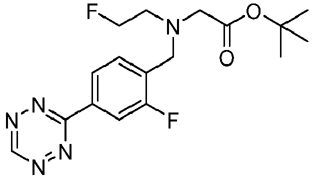

The compound was obtained from *tert*-butyl 2-((4-cyano-2-fluorobenzyl)(2-fluoroethyl)-amino)acetate (0.50 g, 1.61 mmol) as describedfor compound 2. Purification by flash chromatography (n-Heptane/EtOAc 95/5) afforded 0.13 g (22%) of the desired compound as a red oil (60/40 unassigned rotamers mixture). Rf = 0.43 (n-Heptane/EtOAc 80/20); ¹H NMR (400 MHz, CDCl₃) δ 10.23 (s, 1H), 8.49 - 8.35 (m, 1H), 8.35 - 8.12 (m, 1H), 8.00 - 7.46 (m, 1H), 4.74 - 4.58 (m, 3H), 4.56 - 4.48 (m, 1H), 4.46 - 4.27 (m, 2H), 3.99 (s, 0.8H), 3.94 (s, 1.2H), 1.51 -1.38 (m, 9H); ¹³C NMR (101 MHz, CDCl₃) δ 168.25, 168.18, 165.48, 161.53 (d, *J* = 247.6 Hz), 161.42 (d, *J* = 247.9 Hz), 157.97, 156.13, 156.00, 133.45 - 132.72 (m), 131.79 (d, *J* = 4.4 Hz), 131.26 (d, *J* = 4.4 Hz), 129.56 (d, *J* = 9.4 Hz), 129.56 (d, *J* = 20.7 Hz), 124.19, 124.15, 82.16, 82.12, 81.57 (d, *J* = 170.6 Hz), 65.11 (d, *J* = 4.5 Hz), 64.92 (d, *J* = 4.6 Hz), 58.80 (d, *J* = 4.4 Hz), 46.11 (d, *J* = 3.4 Hz), 45.61 (d, *J* = 3.6 Hz), 28.02, 27.92.

### N-(carboxymethyl)-2-fluoro-N-(2-fluoro-4-(1,2,4,5-tetrazin-3-yl)benzyl)ethanaminium 2,2,2-trifluoroacetate

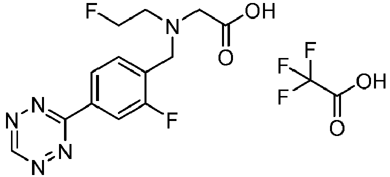

The compound was obtained from *tert*-butyl 2-((2-fluoro-4-(1,2,4,5-tetrazin-3-yl)benzyl)(2-fluoro-ethyl)amino)acetate (0.10 g, 0.43 mmol) as describedfor compound 9. Purification by preparative HPLC afforded 0.05 g (43%) of the desired compound as a red solid. ¹H NMR (600 MHz, CD₃OD) δ 10.37 (s, 1H), 8.39 (ddd, *J* = 8.1, 2.9, 1.6 Hz, 1H), 8.30 - 8.24 (m, 1H), 7.67 (t, *J* = 7.7 Hz, 1H), 4.75 (s, 1H), 4.73 (s, 1H), 4.68 - 4.60 (m, 1H), 4.60 - 4.52 (m, 1H), 4.45 - 4.39 (m, 1H), 4.39 - 4.31 (m, 1H), 4.13 (s, 1H), 4.12 (s, 1H); ¹³C NMR (151 MHz, CD₃OD) δ 171.22 (d, *J* = 5.0 Hz), 165.36 (d, *J* = 2.9 Hz), 161.39 (d, *J* = 246.5 Hz), 161.32 (d, *J* = 246.5 Hz), 158.06 (d, *J* = 3.2 Hz), 156.50, 133.61 (d, *J* = 4.4 Hz), 133.56 (d, *J* = 4.4 Hz), 130.91, 129.42 (d, *J* = 15.1 Hz), 129.25 (d, *J* = 15.1 Hz), 123.64, 114.33 (d, *J* = 3.3 Hz), 114.16 (d, *J* = 3.2 Hz), 81.31 (d, *J* = 168.7 Hz), 65.23 (d, *J* = 7.8 Hz), 65.10 (d, *J* = 8.0 Hz), 48.52 (d, *J* = 58.1 Hz), 45.72 (d, *J =* 3.8 Hz), 45.47 (d, *J* = 3.5 Hz); HPLC-MS [M+H]⁺ m/z calc. for [C₁₃H₁₄F₂N₅O₂]⁺: 310.11; Found: 310.12.

### Synthesis of compound:N-(4-(1,2,4,5-tetrazin-3-yl)benzyl)-N-(carboxymethyl)-2-fluoroethanaminium 2,2,2-trifluoroacetate (12)

### Tert-butyl 2-((4-cyanobenzyl)amino)acetate

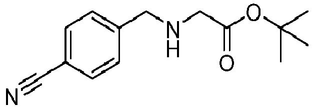

The compound was obtained from 4-bromomethylbenzonitrile (1.80 g, 9.18 mmol) asfor compound 9. Purification by flash chromatography (n-Heptane/EtOAc 80/20) afforded 1.42 g (62%) of the desired compound as a colorless oil. Rf= 0.31 (n-Heptane/EtOAC 60/40). ¹H NMR (400 MHz, CDCl₃) δ 7.61 (d, *J* = 8.4 Hz, 2H), 7.46 (d, *J* = 8.2 Hz, 2H), 3.85 (s, 2H), 3.29 (s, 2H), 1.92 (s, 1H), 1.47 (s, 9H); ¹³C NMR (101 MHz, CDCl₃) δ 171.42, 145.34, 132.24, 128.75, 118.89, 110.94, 81.50, 52.76, 50.90, 28.12.

### Tert-butyl 2-((4-cyanobenzyl)(2-fluoroethyl)amino)acetate

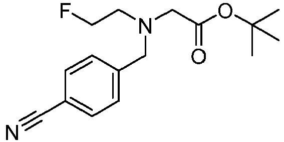

The compound was obtained from *tert*-butyl 2-((4-cyanobenzyl)amino)acetate (1.100 g, 4.46 mmol) as describedfor compound 9. Purification by flash chromatography (n-Heptane/EtOAc 80/20) afforded 0.75 g (57%) of the desired compound as a colorless oil. Rf = 0.22 (n-Heptane/EtOAc 80/20); ¹H NMR (400 MHz, CDCl₃) δ 7.58 (d, *J* = 8.3 Hz, 2H), 7.48 (d, *J* = 8.3 Hz, 2H), 4.55 (t, *J* = 4.9 Hz, 1H), 4.43 (t, *J* = 4.9 Hz, 1H), 3.93 (s, 2H), 3.32 (s, 2H), 3.03 (t, *J* = 4.9 Hz, 1H), 2.96 (t, *J* = 4.9 Hz, 1H), 1.44 (s, 9H); ¹³C NMR (101 MHz, CDCl₃) δ 170.38, 145.05, 132.15, 129.18, 118.91, 110.97, 82.89 (d, *J* = 168.0 Hz), 81.25, 58.30, 55.64, 53.67 (d, *J =* 19.9 Hz), 28.16.

### Tert-butyl 2-((4-(1,2,4,5-tetrazin-3-yl)benzyl)(2-fluoroethyl)amino)acetate

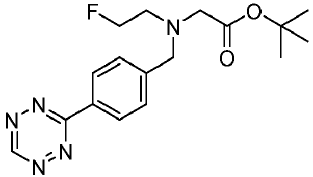

The compound was obtained from *tert*-butyl 2-((4-cyanobenzyl)(2-fluoroethyl)amino)acetate (0.73 g, 2.50 mmol) as described for compound 2. Purification by flash chromatography (n-Heptane/EtOAc 95/5) afforded 0.31 g (36%) of the desired compound as a red oil. Rf = 0.43 (n-Heptane/EtOAc 80/20); ¹H NMR (400 MHz, CDCl₃) δ 10.13 (s, 1H), 8.51 (d, *J* = 8.4 Hz, 2H), 7.56 (d, *J* = 8.1 Hz, 2H), 4.55 (t, *J* = 5.0 Hz, 1H), 4.43 (t, *J* = 5.0 Hz, 1H), 3.95 (s, 2H), 3.13 - 2.77 (m, 2H), 1.41 (s, 9H); ¹³C NMR (101 MHz, CDCl₃) δ 170.48, 166.42, 157.73, 145.06, 130.60, 129.70, 128.41, 82.98 (d, *J =* 165.8 Hz), 81.27, 58.41, 55.63, 53.70 (d, *J* = 20.0 Hz), 28.20.

### N-(4-(1,2,4,5-tetrazin-3-yl)benzyl)-N-(carboxymethyl)-2-fluoroethanaminium 2,2,2-trifluoroacetate

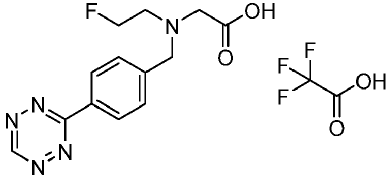

The compound was obtained from *tert*-butyl 2-((2-fluoro-4-(1,2,4,5-tetrazin-3-yl)benzyl)(2-fluoroethyl)amino)acetate (0.15 g, 0.43 mmol) as describedfor compound 9. Purification by preparative HPLC afforded 0.14 g (80%) of the desired compound as a red solid. ¹H NMR (400 MHz, CD₃OD) δ 10.40 (s, 1H), 8.69 (d, *J* = 8.4 Hz, 2H), 7.85 (d, *J* = *8.4* Hz, 2H), 5.04 - 4.97 (m, 1H), 4.92 - 4.86 (m, 1H), 4.70 (s, 2H), 4.19 (s, 2H), 3.83 - 3.75 (m, 1H), 3.75 - 3.66 (m, 1H); ¹³C NMR (101 MHz, CD₃OD) δ 167.39, 165.81, 158.08, 134.15, 133.78, 131.94, 128.46, 78.65 (d, *J* = 167.8 Hz), 58.22 (d, *J* = 1.9 Hz), 54.06 (d, *J* = 19.4 Hz), 52.79 (d, *J* = 2.8 Hz); HPLC-MS [M+H]⁺ m/z calc. for [C₁₃H₁₅FN₅O₂]⁺: 292.12; Found: 292.15.

### Synthesis of compound:1-Carboxy-N-(carboxymethyl)-N-(2-(2-fluoroethoxy)-4-(1,2,4,5-tetrazin-3-yl)benzyl) methanaminium 2,2,2-trifluoroacetate (13)

### 5-Cyano-2-methylphenyl acetate

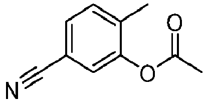

To a solution of 3-hydroxy-4-methylbenzonitrile (3.11 g, 23.28 mmol) and Et₃N (9.74 mL, 7.07 mmol) in CH₂Cl₂ (30 mL) was added acetic anhydride (2.64 mL, 27.93 mmol). The resulting mixture was stirred at room temperature for 12 h. The organic layer was then washed with water (2 x 30 mL) and brine (2 x 30 mL), dried over anhydrous Na₂SO₄, filtered and concentrated under reduced pressure to give 4.05 g (99%) of the desired compound as a white solid. Rf = 0.37 (n-Heptane/EtOAc 80/20); ¹H NMR (400 MHz, CDCl₃) δ 7.77 - 7.34 (m, 1H), 7.33 - 7.03 (m, 2H), 2.45 - 2.27 (m, 3H), 2.26 -2.16 (m, 3H); ¹³C NMR (101 MHz, CDCl₃) δ 168.55, 149.41, 136.64, 132.04, 129.62, 125.74, 118.05, 110.64, 20.64, 16.57.

### 2-(Bromomethyl)-5-cyanophenyl acetate

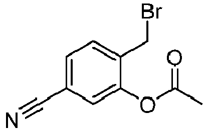

To a solution of 5-cyano-2-methylphenyl acetate (3.00 g, 17.12 mmol) and N-bromo succinimide (4.57 g, 25.68 mmol) in CHCl₃ (50 mL) was added AIBN (1.12 g, 6.85 mmol). The resulting solution was refluxed for 12 h. The reaction was cooled down and the organic layer was washed with water (2 x 30 mL) and brine (2 x 30 mL), dried over anhydrous Na₂SO₄, filtered and concentrated under reduced pressure. Purification by flash chromatography (n-Heptane/EtOAc 90/10) afforded 3.6 g (83%) of the seired compound as a white solid. Rf = 0.35 (n-Heptane/EtOAc 80/20); ¹H NMR (400 MHz, CDCl₃) δ 7.66 - 7.41 (m, 3H), 4.40 (s, 2H), 2.39 (s, 3H); ¹³C NMR (101 MHz, CDCl₃) δ 168.16, 148.95, 135.16, 131.68, 129.83, 126.90, 117.46, 113.41, 25.86, 20.87.

### Di-tert-butyl 2,2'-((2-acetoxy-4-cyanobenzyl)azanediyl)diacetate

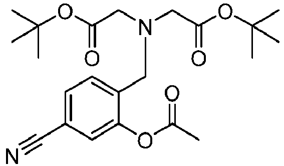

To a solution of 2-(bromomethyl)-5-cyanophenyl acetate (3.61 g, 14.17 mmol) in CH₃CN (50 mL) was added Et₃N (5.92 mL, 42.51 mmol) and di-*tert*-butyl iminodiacetate (3.65 g, 14.87 mmol). The reaction mixture was stirred at room temperature overnight and then the solvent was concentrated under reduced pressure. The resulting mixture was diluted with water (100 mL), extracted with EtOAc (3 x 40 mL), washed with brine (50 mL), dried over MgSO₄, filtered and concentrated under reduced pressure. Purification by flash chromatography (90/10 Heptane/EtOAc) afforded 5.51 g (93%) of the desired compound as a yellow oil. Rf = 0.39 (n-Heptane/EtOAc 80/20); ¹H NMR (400 MHz, CDCl₃) δ 7.83 (d, *J* = 8.0 Hz, 1H), 7.52 (dd, *J* = 8.0, 1.6 Hz, 1H), 7.32 (d, *J* = 1.6 Hz, 1H), 3.90 (s, 2H), 3.37 (s, 4H), 2.33 (s, 3H), 1.45 (s, 9H); ¹³C NMR (101 MHz, CDCl₃) δ 168.36, 167.03, 147.56, 135.47, 129.90, 127.91, 124.34, 116.09, 110.08, 79.34, 53.25, 49.75, 26.27, 18.77.

### Di-tert-butyl 2,2'-((4-cyano-2-hydroxybenzyl)azanediyl)diacetate

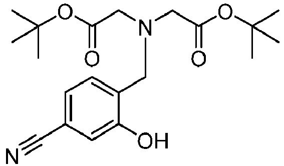

To a solution of di-*tert*-butyl 2,2'-((2-acetoxy-4-cyanobenzyl)azanediyl)diacetate (5.5 g, 13.14 mmol) in CH₃CN (50 mL) was added a 1M NaOH solution (20 mL). The mixture was stirred at room temperature for 12 h. The mixture then concentrated under reduced pressure and neutralized with a 1M HCl solution. The resulting slurry was extracted with EtOAc (3 x 40 mL), washed with brine (50 mL), dried over MgSO₄, filtered and concentrated under reduced pressure to give 4.02 g (81%) of the desired compound as a beige solid. Rf = 0.36 (n-Heptane/EtOAc 80/20); ¹H NMR (600 MHz, CDCl₃) δ 10.05 (s, 1H), 7.14 (d, *J* = 1.5 Hz, 1H), 7.07 (dd, *J* = 7.7, 1.6 Hz, 1H), 7.04 (d, *J* = 7.7 Hz, 1H), 3.98 (s, 2H), 3.37 (s, 4H), 1.47 (s, 9H); ¹³C NMR (151 MHz, CDCl₃) δ 169.99, 157.98, 130.17, 127.17, 122.90, 120.02, 118.82, 112.86, 82.38, 55.54, 54.94, 28.11.

### Di-tert-butyl 2,2'-((4-cyano-2-(2-fluoroethoxy)benzyl)azanediyl)diacetate

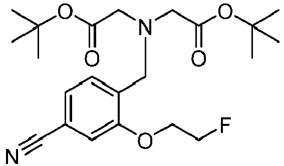

To a solution of di-tert-butyl 2,2'-((4-cyano-2-hydroxybenzyl)azanediyl)diacetate (2.6 g, 6.97 mmol) and K₂CO₃ (1.92 g, 13.94 mmol) in CH₃CN (50 mL) was added 1-fluoro-2-iodoethane (1.33 g, 7.67 mmol). The reaction was refluxed for 12 h and then concentrated under reduced pressure. The resulting mixture was diluted with water (50 mL), extracted with EtOAc (3 x 50 mL), washed with brine (50 mL), dried over MgSO₄, filtered and concentrated under reduced pressure. Purification by flash chromatography (n-Heptane/EtOAc 90/10) afforded 2.71 g (92%) of the desired compound as a colorless oil. Rf = 0.25 (n-Heptane/EtOAc 80/20); ¹H NMR (400 MHz, CDCl₃) δ 7.73 (s, 1H), 7.30 (dd, *J* = 7.8, 1.4 Hz, 1H), 7.05 (d, *J* = 1.4 Hz, 1H), 4.91 - 4.78 (m, 1H), 4.75 - 4.63 (m, 1H), 4.34 - 4.23 (m, 1H), 4.22 - 4.16 (m, 1H), 3.98 (s, 2H), 3.44 (s, 4H), 1.45 (s, 18H); ¹³C NMR (101 MHz, CDCl₃) δ 170.51, 156.46, 134.18, 130.73, 125.48, 118.90, 114.31, 111.31, 81.46 (d, *J* = 171.9 Hz), 81.13, 67.91 (d, *J* = 20.6 Hz), 55.88, 51.69.

### Di-tert-butyl 2,2'-((2-(2-fluoroethoxy)-4-(1,2,4,5-tetrazin-3-yl)benzyl)azanediyl)diacetate

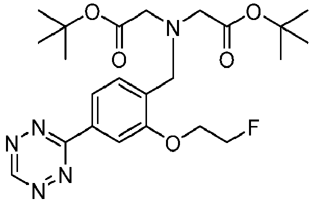

The compound was obtained from di-*tert*-butyl 2,2'-((4-cyano-2-(2-fluoroethoxy)benzyl)azanediyl)-diacetate (2.5 g, 5.91 mmol) as reportedfor compound 2. Purification by flash chromatography (n-Heptane/EtOAc 90/10) afforded 0.74 g (26%) of the desired compound as a red oil. Rf = 0.25 (n-Heptane/EtOAc 80/20); ¹H NMR (400 MHz, CDCl₃) δ 10.12 (s, 1H), 8.22 (dd, *J* = 7.9, 1.6 Hz, 1H), 8.02 (d, *J* = 1.6 Hz, 1H), 7.76 (d, *J* = 7.9 Hz, 1H), 4.84 - 4.76 (m, 1H), 4.71 - 4.65 (m, 1H), 4.41 - 4.31 (m, 1H), 4.30 - 4.23 (m, 1H), 4.00 (s, 2H), 3.43 (s, 4H), 1.40 (s, 18H); ¹³C NMR (101 MHz, CDCl₃) δ 170.67, 166.24, 157.74, 157.25, 133.99, 131.10, 121.58, 110.52, 81.68 (d, *J* = 171.2 Hz), 81.00, 67.83 (d, *J* = 20.7 Hz), 55.88, 51.82, 28.17.

### 1-Carboxy-N-(carboxymethyl)-N-(2-(2-fluoroethoxy)-4-(1,2,4,5-tetrazin-3-yl)benzyl) methanaminium 2,2,2-trifluoroacetate

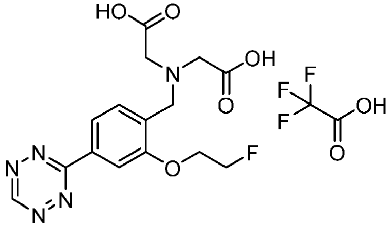

The compound was obtained from di-*tert*-butyl 2,2'-((2-(2-fluoroethoxy)-4-(1,2,4,5-tetrazin-3-yl)benzyl)azanediyl)diacetate (0.50 g, 0.83 mmol) as reportedfor compound 9. Purification by preparative HPLC afforded 0.21 g (52%) of the desired compound as a red solid. ¹H NMR (400 MHz, CD₃OD) δ 10.39 (s, 1H), 8.31 - 8.22 (m, 4H), 7.76 (d, *J* = 7.8 Hz, 1H), 5.02 - 4.93 (m, 1H), 4.92 - 4.82 (m, 1H), 4.74 (s, 2H), 4.60 - 4.56 (m, 1H), 4.54 - 4.42 (m, 1H), 4.21 (s, 4H); ¹³C NMR (101 MHz, CD₃OD) δ 167.64, 158.16 (d, *J* = 7.4 Hz), 135.64, 134.19, 122.40, 120.89, 111.00, 81.42 (d, *J* = 168.8 Hz), 68.38 (d, *J* = 19.9 Hz), 54.10, 53.62; HPLC-MS [M+H]⁺ m/z calc. for [C₁₅H₁₇FN₅O₅]⁺: 366.12; Found: 366.11.

### Synthesis of compound:1 -Carboxy-N-(carboxymethyl)-N-((6-(6-(5-(2-fluoroethoxy)pyridin-2-yl)-1,2,4,5-tetrazin-3-yl)pyridin-3-yl)methyl)methanaminium 2,2,2-trifluoroacetate (14)

### 5-(Bromomethyl)picolinonitrile

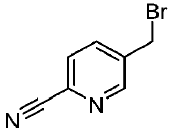

The compound was obtained from 5-methylpicolinonitrile (7.0 g, 59.25 mmol) as reported for compound 13. Purification by flash chromatography (n-Heptane/EtOAc 90/10) afforded 7.1 g (61%) of the desired compound as a white solid. Rf = 0.29 (n-Heptane/EtOAc 80/20); ¹H NMR (400 MHz, CDCl₃) δ 8.72 (d, *J* = 2.2 Hz, 1H), 7.87 (dd, *J* = 8.1, 2.3 Hz, 1H), 7.97 - 7.38 (m, 1H), 4.49 (s, 2H); ¹³C NMR (101 MHz, CDCl₃) δ 151.19, 137.45, 133.40, 128.36, 116.84, 27.87.

### Di-tert-butyl 2,2'-(((6-cyanopyridin-3-yl)methyl)azanediyl)diacetate

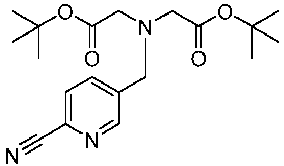

The compound was obtained from 5-(bromomethyl)picolinonitrile (1.5 g, 7.61 mmol) asfor compound 13. Purification by flash chromatography (n-Heptane/EtOAc 90/10) afforded 2.7 g (98%) of the desired compound as a white solid. Rf = 0.33 (n-Heptane/EtOAc 80/20); ¹H NMR (400 MHz, CDCl₃) δ 8.61 (dd, *J* = 2.1, 0.9 Hz, 1H), 7.96 (dd, *J* = 8.0, 2.1 Hz, 1H), 7.60 (dd, *J* = 7.9, 0.9 Hz, 1H), 3.93 (s, 2H), 3.33 (s, 4H), 1.40 (s, 18H); ¹³C NMR (101 MHz, CDCl₃) δ 170.09, 151.41, 138.96, 137.51, 132.77, 128.27, 117.35, 81.52, 55.41, 54.57, 28.16.

### Di-tert-butyl 2,2'-(((6-(6-(5-(2-fluoroethoxy)pyridin-2-yl)-1,2,4,5-tetrazin-3-yl)pyridin-3-yl)methyl)- azanediyl)diacetate

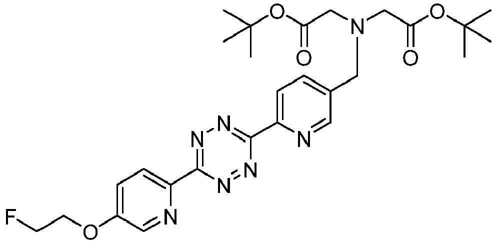

Di-tert-butyl 2,2'-(((6-cyanopyridin-3-yl)methyl)azanediyl)diacetate (0.2 g, 0.55 mmol), 5-(2-fluoroethoxy)picolinonitrile from Example 6 (0.46 g, 2.77 mmol) and sulfur (0.36 g, 0.14 mmol) were suspended in EtOH (3 mL), followed by the addition of hydrazine hydrate (0.40 mL, 8.31 mmol). The reaction was heated to 90 °C for 2 h. The mixture was cooled to room temperature and the formed precipitate was removed by filtration. Water (10 mL) and a solution of NaNO₂ (0.76 g, 11.07 mmol) in water 10 mL were added and the mixture was cautiously acidified to pH 2 by addition of AcOH. The mixture was extracted with DCM and the combined organic layer waswashed with water and brine, dried over MgSO₄ and concentrated. The residue was purified by flash column chromatography (CH₂Cl₂/MeOH 95/5) and crystallized from MeOH to afford 0.07 g (23%) of the desired compound as a pink solid. Rf = 0.36 (CH₂Cl₂/MeOH 95/5); ¹H NMR (600 MHz, DMSO) δ 8.86 (d, *J* = 2.1 Hz, 1H), 8.68 (dd, *J* = 7.9, 3.1 Hz, 1H), 8.62 (d, *J* = 8.8 Hz, 1H), 8.58 (d, *J* = 8.0 Hz, 1H), 8.11 (dd, *J* = 8.1, 2.1 Hz, 1H), 7.76 (dd, *J* = 8.8, 3.0 Hz, 1H), 4.97 - 4.86 (m, 1H), 4.81 (dd, *J* = 4.8, 2.8 Hz, 1H), 4.54 (dd, *J* = 4.8, 2.7 Hz, 1H), 4.51 - 4.43 (m, 1H), 4.01 (s, 2H), 3.45 (s, 4H), 1.43 (s, 18H); ¹³C NMR (151 MHz, DMSO) δ 170.35, 163.47, 163.24, 157.21, 151.20, 149.63, 142.89, 139.86, 138.19, 138.11, 126.00, 124.30, 82.43 (d, *J* = 166.8 Hz), 80.97, 68.39 (d, *J* = 18.9 Hz), 55.57, 54.87, 28.29.

### 1-Carboxy-N-(carboxymethyl)-N-((6-(6-(5-(2-fluoroethoxy)pyridin-2-yl)-1,2,4,5-tetrazin-3-yl)pyridin-3-yl)methyl)methanaminium 2,2,2-trifluoroacetate

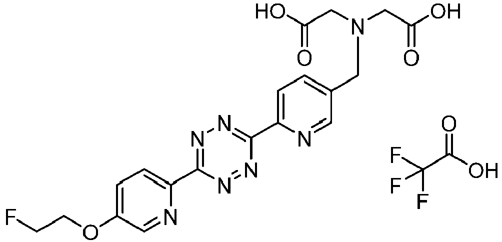

The compound was obtained from di-tert-butyl 2,2'-(((6-(6-(5-(2-fluoroethoxy)pyridin-2-yl)-1,2,4,5-tetrazin-3-yl)pyridin-3-yl)methyl)- azanediyl)diacetate (0.006 g, 0.83 mmol) as reportedfor compound 9. Purification by preparative HPLC afforded 0.35 g (58%) of the desired compound as a red solid. ¹H NMR (600 MHz, DMSO) δ 8.89 (d, *J* = 2.1 Hz, 1H), 8.68 (d, *J* = 2.9 Hz, 1H), 8.62 (d, *J* = 8.8 Hz, 1H), 8.57 (dd, *J* = 8.0, 0.8 Hz, 1H), 8.14 (dd, *J* = 8.1, 2.1 Hz, 1H), 7.76 (dd, J = 8.8, 3.0 Hz, 1H), 4.91 - 4.86 (m, 1H), 4.83 - 4.79 (m, 1H), 4.57 - 4.52 (m, 1H), 4.52 - 4.47 (m, 1H), 4.06 (s, 2H), 3.53 (s, 4H); ¹³C NMR (151 MHz, DMSO) δ 172.62, 163.47, 163.23, 157.22, 151.33, 149.58, 142.89, 139.86, 138.28, 126.00, 124.27, 122.16, 82.43 (d, *J* = 166.8 Hz), 68.39 (d, *J* = 18.8 Hz), 55.00, 54.49.

### Syntheis of compound: 1-Carboxy-N-(carboxymethyl)-N-(2-hydroxy-4-(1,2,4,5-tetrazin-3-yl)benzyl)methanaminium 2,2,2-trifluoroacetate (uuu)

### Di-tert-butyl 2,2'-((2-hydroxy-4-(1,2,4,5-tetrazin-3-yl)benzyl)azanediyl)diacetate

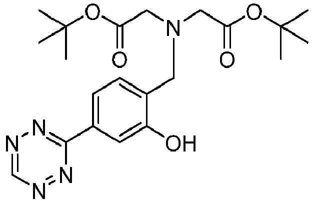

The compound was obtained from di-*tert*-butyl 2,2'-((4-cyano-2-hydroxybenzyl)azanediyl)diacetate (0.4 g, 1.06 mmol) as described for compound 13. Purification by flash chromatography (n-Heptane/EtOAc 80/20) afforded 0.11 g (24%) of the desired compound as a red solid. Rf = 0.38 (n-Heptane/EtOAc 80/20); ¹H NMR (400 MHz, CDCl₃) δ 10.17 (s, 1H), 9.90 (s, 1H), 8.11 (d, *J* = 1.7 Hz, 1H), 8.02 (dd, *J* = 7.8, 1.8 Hz, 1H), 7.18 (d, *J* = 7.9 Hz, 1H), 4.05 (s, 2H), 3.43 (s, 4H), 1.47 (s, 18H); ¹³C NMR (101 MHz, CDCl₃) δ 170.06, 166.43, 158.47, 157.73, 130.51, 127.12, 119.18, 116.26, 82.19, 54.98, 28.12. 1-Carboxy-N-(carboxymethyl)-N-(2-hydroxy-4-(1,2,4,5-tetrazin-3-yl)benzyl)methanaminium 2,2,2-trifluoroacetate

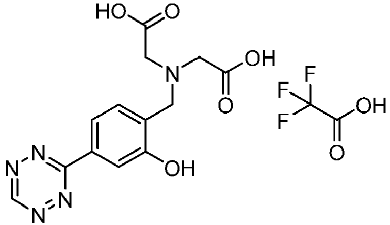

The compound was obtained from di-tert-butyl 2,2'-((2-hydroxy-4-(1,2,4,5-tetrazin-3-yl)benzyl)azanediyl)diacetate (0.09 g, 0.21 mmol) as reportedfor compound 9. Purification by preparative HPLC afforded 0.065 g (72%) of the desire compound as a red solid. ¹H NMR (400 MHz, DMSO) δ 10.57 (s, 1H), 8.89 - 7.81 (m, 2H), 7.48 (d, *J* = 7.9 Hz, 1H), 4.03 (s, 2H), 3.56 (s, 4H); ¹³C NMR (101 MHz, DMSO) δ 172.41, 165.79, 158.58, 157.63, 132.85, 131.88, 118.99, 114.74, 54.20, 53.75; HPLC-MS [M+H]⁺ m/z calc. for [C₁₃H₁₄N₅O₅]⁺: 320.10; Found: 320.12.

### Synthesis of compound: 1-Carboxy-N-(carboxymethyl)-N-(2-methoxy-4-(1,2,4,5-tetrazin-3-yl)benzyl)methanaminium 2,2,2-trifluoroacetate (iii)

### Di-tert-butyl 2,2'-((4-cyano-2-methoxybenzyl)azanediyl)diacetate

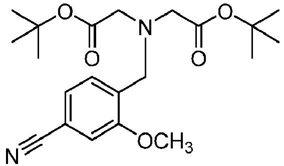

To a solution of compound di-tert-butyl 2,2'-((4-cyano-2-hydroxybenzyl)azanediyl)diacetate (0.4 g, 1.06 mmol) (0.4 g, 1.07 mmol) and K₂CO₃ (0.44 g, 3.19 mmol) in CH₃CN (10 mL) was added CH₃l (0.07 mL, 1.17 mmol). The reaction was refluxed for 12 h and then concentrated under reduced pressure. The resulting mixture was diluted with water (20 mL), extracted with EtOAc (3 x 20 mL), washed with brine (50 mL), dried over MgSO₄, filtered and concentrated under reduced pressure to give 0.41 g (99%) of the desired compound as a yellow oil. Rf = 0.37 (n-Heptane/EtOAc 80/20); ¹H NMR (400 MHz, CDCl₃) δ 7.64 (d, *J* = 7.8 Hz, 1H), 7.20 (dd, *J* = 7.7, 1.5 Hz, 1H), 7.00 (d, *J* = 1.5 Hz, 1H), 3.89 (s, 2H), 3.78 (s, 3H), 3.37 (s, 4H), 1.40 (s, 18H); ¹³C NMR (101 MHz, CDCl₃) δ 168.68, 155.75, 131.63, 128.74, 122.93, 117.23, 111.16, 109.44, 79.14, 53.94, 53.78, 49.49, 26.30.

### Di-tert-butyl 2,2'-((2-methoxy-4-(1,2,4,5-tetrazin-3-yl)benzyl)azanediyl)diacetate

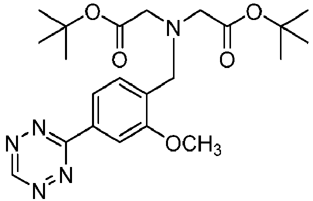

The compound was obtained from di-*tert*-butyl 2,2'-((4-cyano-2-methoxybenzyl)azanediyl)diacetate (0.41 g, 1.05 mmol) as described for compound 13. Purification by flash chromatography (n-Heptane/EtOAc 90/10) afforded 0.11 g (23%) of the desired compound as a red oil. Rf = 0.29 (n-Heptane/EtOAc 80/20); 1H NMR (400 MHz, CDCl₃) δ 10.12 (s, 1H), 8.17 (dd, *J* = 7.9, 1.6 Hz, 1H), 8.02 (d, *J* = 1.7 Hz, 1H), 7.72 (d, *J* = 7.9 Hz, 1H), 3.97 (s, 2H), 3.87 (s, 3H), 3.42 (s, 4H), 1.40 (s, 18H); ¹³C NMR (101 MHz, CDCl₃) δ 170.67, 166.38, 158.41, 157.71, 133.20, 131.26, 131.09, 120.98, 109.28, 80.95, 55.79, 55.65, 51.45, 28.19.

### 1-Carboxy-N-(carboxymethyl)-N-(2-methoxy-4-(1,2,4,5-tetrazin-3-yl)benzyl)methanaminium 2,2,2-trifluoroacetate

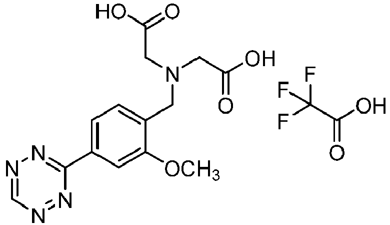

The compound was obtained from di-tert-butyl 2,2'-((2-methoxy-4-(1,2,4,5-tetrazin-3-yl)benzyl)azanediyl)diacetate (0.09 g, 0.21 mmol) as reportedfor compound 9. Purification by preparative HPLC afforded.075 g (80%) of the desired compound as a red solid. ¹H NMR (400 MHz, CD₃OD) δ 10.41 (s, 1H), 8.29 (d, *J* = 7.4 Hz, 2H), 7.75 (d, *J* = 7.9 Hz, 1H), 4.77 (s, 2H), 4.24 (s, 4H), 4.09 (s, 3H); ¹³C NMR (101 MHz, CD₃OD) δ 167.07, 165.67, 159.27, 158.16, 136.00, 134.19, 121.28, 120.52, 109.97, 55.16, 53.62, 53.48; HPLC-MS [M+H]⁺ m/z calc. for [C₁₄H₁₆N₅O₅]⁺: 334.11; Found: 334.13.

### Precursor Compounds

### Syntheis of compound: 2-{[(Methane)sulfonyl]oxy}ethyl benzoate (15)

### 2-Hydroxyethyl benzoate

Sulfuric acid (18.4 M, 0.5 mL, 0.5 equiv) was added to a mixture of benzoic acid (2.40 g, 19.65 mmol, 1.00 equiv) and ethylene glycol (5.40 g, 87.00 mmol, 4.43 equiv). The mixture was heated to 120 °C and left with stirring for 2.5 h. The mixture was cooled and quenched by transferring it dropwise to H₂O (40 mL). The mixture was neutralized with NaHCO₃ and was the further diluted with 60 mL H₂O and extracted with EtOAc (3x100mL). The organic phase was dried over MgSO₄ and concentrated *in vacuo* yielding 2-Hydroxyethyl benzoate (3.14 g, 18.90 mmol, 96%) as a pale semisolid. The compound was used without further purification in the following reactions.

### 2-{[(Methane)sulfonyl]oxy}ethyl benzoate

2-Hydroxyethyl benzoate (400 mg, 2.41 mmol, 1.00 equiv) was dissolved in 5 mL dry DCM and Et₃N (504 uL, 3.61 mmol. 1.50 equiv) and methanesulfonyl chloride (280 uL, 3.61 mmol, 1.50 equiv) was added at 0°C under argon. The reaction was slowly heated to room temperature over 30 min and was stirred for another 60 min. The crude was diluted with 30 mL DCM and then quenched with 20 mL H₂O. The aqueous phase was extracted with DCM 2x20 mL and the combined organic layers were dried over MgSO₄ and concentrated *in vacuo.* Purification by flash chromatography (n-heptane/EtOAc, 50:50) yielded 2-{[(methane)sulfonyl]oxy}ethyl benzoate (580 mg, 2.37 mmol, 98%) as an off-white solid. ¹H NMR (600 MHz, CDCl₃) δ 8.06 (dt, *J* = 8.4, 1.1 Hz, 2H), 7.59 (ddt, *J* = 7.8, 6.9, 1.3 Hz, 1H), 7.49 - 7.43 (m, 2H), 4.62 - 4.58 (m, 2H), 4.58 - 4.54 (m, 2H), 3.06 (s, 3H). ¹³C NMR (151 MHz, CDCl₃) δ 166.28, 133.56, 129.89, 129.51, 128.66, 67.25, 62.48, 37.97; HRMS (MALDI-TOF) calculated for C₁₀H₁₂O₅S⁺ [M+Na]⁺: 267.0297, found: 267.0299.

### Synthesis of compound: 2-{[(Tolouene)sulfonyl]oxy}ethyl benzoate (16)

Benzoic acid (150 mg, 1.23 mmol 1.00 equiv) was mixed with Bu₄NOH (40%wt in MeOH, 1.20 mL, 1.84 mmol, 1.50 equiv), H₂O and toluene (2 mL) respectively was added and the mixture was stirred for 5 min before it was evaporated. The reaction was dissolved in 5 mL dry MeCN and cooled to 0°C. Ethylene di(*p*-toluenesulfonate) (683 mg, 1.84 mmol, 1.50 equiv) was dissolved in 10 mL dry MeCN and added to the reaction. It was allowed to slowly heat to room temperature and left for 3 hours. The reaction mixture was then quenched with NH₄Cl (sat, 20 mL) and extracted with EtOAc. The organic phase was washed with brine dried over MgSO₄ and concentrated *in vacuo.* Purification by flash chromatography (n-Heptane/EtOAc 70:30) yielded 2-{[(Tolouene)sulfonyl]oxy}ethyl benzoate (195 mg, 0.61 mmol, 50%) as a white solid. ¹H NMR (400 MHz, CDCl₃) δ 7.98 - 7.91 (m, 2H), 7.82 - 7.75 (m, 2H), 7.61 - 7.54 (m, 1H), 7.42 (t, *J* = 7.8 Hz, 2H), 7.30 - 7.23 (m, 2H), 4.52 - 4.32 (m, 4H), 2.38 (s, 3H); ¹³C NMR (151 MHz, CDCl₃) δ 166.18, 145.13, 133.45, 132.96, 130.06, 129.91, 129.53, 128.52, 128.08, 67.88, 62.21, 21.79; HRMS (MALDI-TOF) calculated for C₁₆H₁₆O₅S⁺ [M+Na]⁺: 343.0610, found: 343.0610.

### Synthesis of compound 2-{[(4-Nitrobenzene)sulfonyl]oxy}ethyl benzoate (17)

1-Hydroxyl-3-ethylbenzoate (150 mg, 0.90 mmol, 1.00 equiv) was added with nitrobenzenesulfonyl chloride (600 mg, 2.71 mmol, 3.00 equiv) and dissolved in 10 mL of dry DCM under argon at 0°C. A mixture of DIPEA (629 uL, 3.61 mmol, 4.00 euiv) and DMAP (22 mg, 0.18 mmol, 0.20 equiv) in 3 mL dry DCM was subsequently added under argon and the reaction was slowly allowed to heat to room temperature and stirred for 3 hours. The reaction was diluted with 10 mL of DCM and washed with NH₄Cl (sat) 20 mL and H₂O (2x20mL). The organic phase was dried over Na₂SO₄ and concentrated *in vacuo.* Purification by flash chromatography (n-heptane/EtOAc, 60:40) and recrystallization in EtOAc/Heptane yielded 2-{[(4-Nitrobenzene)sulfonyl]oxy}ethyl benzoate (76 mg, 0.22 mmol, 24%) as an off-white solid. ¹H NMR (600 MHz, CDCl₃) δ 8.27 - 8.21 (m, 2H), 8.10 - 8.04 (m, 2H), 7.90 - 7.84 (m, 2H), 7.58 (tt, *J* = 7.4, 1.3 Hz, 1H), 7.45 - 7.37 (m, 2H), 4.55 - 4.50 (m, 4H); ¹³C NMR (151 MHz, CDCl₃) δ 165.97, 150.77, 141.78, 133.81, 133.62, 129.83, 129.74, 129.27, 129.12, 128.68, 128.62, 124.58, 124.49, 69.16, 61.90; HRMS (MALDI-TOF) calculated for C₁₅H₁₃NO₇S [M+H]⁺: 352.0485, found: 352.0490.

### Synthesis of compound4-{{{2-{[(Methane)sulfonyl]oxy}ethoxy}methyl}-1,2,4,5-tetrazin-3-yl}benzene (18)

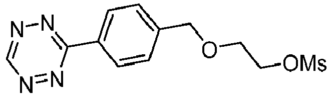

4-{[(2-Hydroxyethoxy)methyl]-1,2,4,5-tetrazin-3-yl}benzene from compound 2 (50 mg, 0.22 mmol) was mixed with methanesulfonyl chloride (50 uL, 0.65 mmol, 3.00 equiv) and dissolved in 4 mL of dry DCM under argon. A mixture of DIPEA (150 uL, 0.86 mmol) and DMAP (5 mg, 0.04 mmol) in 1 mL dry DCM was added at 0°C under argon. The reaction was slowly heated to room temperature and left for 1.5 hours. The reaction was diluted with 10 mL of DCM and washed with NH₄Cl (sat) 20 mL and H₂O (2x20mL) The organic phase was dried over N₂SO₄ and concentrated *in vacuo.* Purification by flash chromatography (n-heptane/EtOAc, 70:30) yielded 56 mg (84%) of the desired compound as a pink liquid that solidified over time. ¹H NMR (600 MHz, CDCl₃) δ 10.22 (s, 1H), 8.65 - 8.60 (m, 2H), 7.58 (d, *J =* 8.3 Hz, 2H), 4.71 (s, 2H), 4.47 - 4.42 (m, 2H), 3.85 - 3.81 (m, 2H), 3.06 (s, 3H); ¹³C NMR (151 MHz, CDCl₃) δ 166.43, 157.98, 143.16, 131.29, 128.66, 128.38, 72.87, 68.97, 68.50, 37.88; HRMS (MALDI-TOF) calculated for C₁₂H₁₄N₄O₄S [M+Na]⁺: 333.0628, found: 333.0627.

### Synthesis of compound:4-{{{2-{[(Toluene)sulfonyl]oxy}ethoxy}methyl}-1,2,4,5-tetrazin-3-yl}benzene (19)

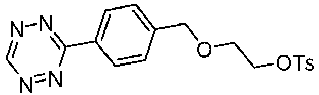

4-{[(2-Hydroxyethoxy)methyl]-1,2,4,5-tetrazin-3-yl}benzene from compound 2 (50 mg, 0.22 mmol, 1.00 equiv) was mixed with toluenesulfonyl chloride (123 mg, 0.65 mmol, 3.00 equiv) and dissolved in 4 mL dry DCM under argon. A mixture of DIPEA (150 uL, 0.86 mmol) and DMAP (5 mg, 0.04 mmol) in 1 mL dry DCM was added at 0°C under argon. The reaction was slowly heated to room temperature and left for 20 hours. The reaction was diluted with 10 mL DCM and washed with NH₄Cl (sat) 20 mL and H₂O (2x20mL). The organic phase was dried over N₂SO₄ and concentrated *in vacuo.* Purification by flash chromatography (n-heptane/EtOAc, 70:30) afforded 31 mg (37%) of the desired compound as a pink solid. ¹H NMR (600 MHz, CDCl₃) δ 10.22 (d, *J* = 1.1 Hz, 1H), 8.61 - 8.57 (m, 2H), 7.84 - 7.80 (m, 2H), 7.52 - 7.48 (m, 2H), 7.35 - 7.31 (m, 2H), 4.62 (s, 2H), 4.28 - 4.23 (m, 2H), 3.78 - 3.73 (m, 2H), 2.44 (s, 3H); ¹³C NMR (151 MHz, CDCl₃) δ 166.47, 157.97, 145.04, 143.41, 133.19, 131.10, 130.00, 128.55, 128.21, 128.14, 72.73, 69.30, 68.22, 21.81; HRMS (MALDI-TOF) calculated for C₁₈H₁₈N₄O₄S [M+Na]⁺: 409.0941, found: 409.0941.

### Synthesis of compound:4-{{{2-{[(4-Nitrobenzene)sulfonyl]oxy}ethoxy}methyl}-1,2,4,5-tetrazin-3-yl}benzene (20)

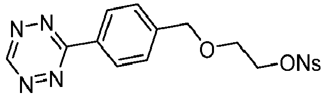

4-{[(2-Hydroxyethoxy)methyl]-1,2,4,5-tetrazin-3-yl}benzene from compound 2(50 mg, 0.22 mmol, 1.00 equiv) was mixed with nitrobenzenesulfonyl chloride (143 mg, 0.65 mmol, 3.00 equiv) and dissolved in 4 mL dry DCM under argon. A mixture of DIPEA (150 uL, 0.86 mmol) and DMAP (5 mg, 0.04 mmol) in 1 mL dry DCM was added at 0°C under argon. The reaction was slowly heated to room temperature and left for 3 hours. The reaction was diluted with 10 mL DCM and washed with NH₄Cl (sat) 20 mL and H₂O (2x20mL). The organic phase was dried over N₂SO₄ and concentrated *in vacuo.* Purification by flash chromatography (n-heptane/EtOAc, 60:40) yielded 83 mg (92%) of the desired compound as a pink solid. ¹H NMR (600 MHz, CDCl₃) δ 10.24 (s, 1H), 8.61 - 8.57 (m, 2H), 8.32 - 8.28 (m, 2H), 8.12 - 8.08 (m, 2H), 7.46 (dd, *J* = 7.4, 1.1 Hz, 2H), 4.58 (s, 2H), 4.41 - 4.37 (m, 2H), 3.79 - 3.75 (m, 2H); ¹³C NMR (151 MHz, CDCl₃) δ 166.34, 158.01, 150.80, 142.86, 142.05, 131.39, 129.38, 128.66, 128.62, 128.57, 128.34, 128.32, 125.17, 124.45, 124.41, 72.83, 70.58, 68.04; HRMS (MALDI-TOF) calculated for C₁₇H₁₅N₅O₆S [M+Na]⁺: 440.0635, found: 440.0636.

### Synthesis of compound:2-Fluoro-4-(1,2,4,5-tetrazin-3-yl)-2-{[(tolouene)sulfonyl]oxy}ethyl-benzoate (21)

### 2-Fluoro-4-(1,2,4,5-tetrazin-3-yl)-2-hydroxyethyl-benzoate

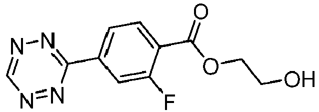

2-Fluoro-4-(1,2,4,5-tetrazin-3-yl)benzoic acid from compound 3(0.07 g, 0.32 mmol) and 2-bromoethanol (0.07 mL, 0.97 mmol) was dissolved in 3 ml Dry DMF and DIPEA (0.17 mL, 0.97 mmol) was dissolved in 1 ml DMF and added dropwise and the reaction was left at 70 C. After the reaction was completed, it was cooled down, diluted with water (30 mL) and extracted with DCM (2 x 20 mL). The crude was purified by flash chromatography (60/40 Heptane/EtOAc) to give 35 mg (41%) of 2-fluoro-4-(1,2,4,5-tetrazin-3-yl)-2-hydroxyethyl-benzoate as a red solid. ¹H NMR (400 MHz, CDCl₃) δ 10.30 (s, 1H), 8.46 (dd, *J* = 8.3, 1.7 Hz, 1H), 8.38 (dd, *J* = 11.3, 1.7 Hz, 1H), 8.16 (t, *J* = 7.7 Hz, 1H), 4.58 - 4.45 (m, 2H), 3.99 (t, *J* = 4.7 Hz, 2H); ¹³C NMR (101 MHz, CDCl₃) δ 165.00, 164.97, 163.86, 163.82, 163.42, 160.83, 158.14, 137.55, 137.46, 133.24, 123.54, 123.50, 122.40, 122.30, 116.83, 116.57, 67.31, 60.98; HRMS (MALDI-TOF) calculated for C₁₁H₉FN₄O₃ [M+H]⁺: 266.0615, found: 266.0617.

### 2-Fluoro-4-(1,2,4,5-tetrazin-3-yl)- 2-{[(tolouene)sulfonyl]oxy}ethyl -benzoate

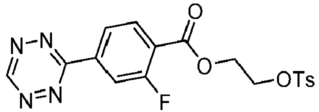

2-Fluoro-4-(1,2,4,5-tetrazin-3-yl)benzoic acid (300 mg, 1.36 mmol) and ethylene di(*p*-toluenesulfonate (1514 mg, 4.09 mmol, 3.00 equiv) was dissolved in 30 ml dry DMF and DIPEA (0.340 mL, 2.18 mmol, 1.60 equiv) was dissolved in 4 ml DMF and added dropwise to the reaction which was left at 100°C for 3 hours. The crude was diluted with Et₂O (30 ml) and washed with NH₄Cl and H₂O (2x30 ml). The organic phase was dried over MgSO₄ and concentrated *in vacuo.* Purification by flash chromatography (toluene/EtOAc, 95:5) yielded 242 mg (42%) of the desired product as a pink liquid that solidified over time. ¹H NMR (600 MHz, CDCl₃) δ 10.32 (d, *J* = 1.5 Hz, 1H), 8.52 - 8.46 (m, 1H), 8.42 (dt, *J =* 11.2, 1.6 Hz, 1H), 8.10 (td, *J* = 7.8, 7.2, 1.5 Hz, 1H), 7.81 (dd, *J* = 8.3, 1.7 Hz, 2H), 7.32 (d, *J* = 7.9 Hz, 2H), 4.59 - 4.54 (m, 2H), 4.42 - 4.36 (m, 2H), 2.41 (s, 3H); ¹⁹F NMR (376 MHz, CDCl₃) δ -106.68; ¹³C NMR (151 MHz, CDCl₃) δ 165.17, 165.15, 163.31, 163.08, 163.05, 161.57, 158.35, 158.33, 145.24, 137.88, 137.82, 133.39, 133.36, 132.90, 130.07, 128.10, 123.62, 123.60, 121.97, 121.90, 116.94, 116.78, 67.44, 62.90, 21.79; HRMS (MALDI-TOF) calculated for (*in situ* reduced to dihydro tetrazine): C₁₈H₁₈FN₄O₅S [M+H]⁺: 421.0982, found: 421.0970.

### Synthesis of compound2-Fluoro-4-(1,2,4,5-tetrazin-3-yl)- 2-{[(4-nitrobenzene)sulfonyl]oxy}ethyl -benzoate (22)

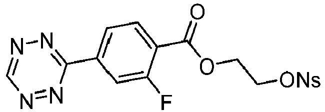

To a solution of 2-fluoro-4-(1,2,4,5-tetrazin-3-yl)-2-hydroxyethyl-benzoate from compound 20 (0.05 g, 0.10 mmol) and DIPEA (0.07 mL, 0.43 mmol) in DCM (20 mL) Nosyl chloride (0.072 g, 0.32 mmol) and DMAP (0.002 g, 0.02 mmol) was added. The reaction was stirred at room temperature for 4 h. Purification by flash chromatography (75/25 Heptane/EtOAc) yielded semi-pure product (60 mg) as a red solid. Recrystallization from EtOAc/Heptane afforded 35 mg (35%) of 2-fluoro-4-(1,2,4,5-tetrazin-3-yl)- 2-{[(4-nitrobenzene)sulfonyl]oxy}ethyl -benzoate as a red solid. ¹H NMR (600 MHz, DMSO-*d*₆) δ 10.73 (s, 1H), 8.43 (dd, *J* = 8.2, 1.7 Hz, 1H), 8.40 - 8.36 (m, 2H), 8.34 (dd, *J* = 11.5, 1.6 Hz, 1H), 8.22 - 8.18 (m, 2H), 8.03 (t, *J* = 7.8 Hz, 1H), 4.59 - 4.54 (m, 4H); ¹³C NMR (151 MHz, DMSO) δ 164.58, 162.74, 162.71, 162.44, 160.72, 158.96, 150.98, 140.99, 138.83, 138.77, 133.39, 129.82, 125.35, 124.09, 124.07, 121.38, 121.31, 116.47, 116.30, 70.38, 63.21; ¹⁹F NMR (376 MHz, DMSO) δ -108.44; HRMS (MALDI-TOF) calculated for C₁₇H₁₂FN₅O₇S [M+H]⁺: 450.0514, found: 450.0515.

### Synthesis of compound:2-(((4-Nitrophenyl)sulfonyl)oxy)ethyl 4-(1,2,4,5-tetrazin-3-yl)benzoate (23)

### 2-Hydroxyethyl 4-(1,2,4,5-tetrazin-3-yl)benzoate

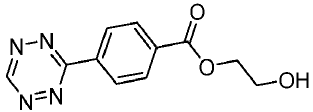

The compound was obtained from 4-(1,2,4,5-tetrazin-3-yl)benzoic acid (0.03 g, 0.15 mmol) as described for compound 21. Purification by flash chromatography (n-Heptane/EtOAc 70/30) afforded 0.03 g (82%) of the desired product as a pink solid. Rf= 0.24 (n-Heptane/EtOAc 70/30); ¹H NMR (600 MHz, CDCl₃) δ 10.28 (s, 1H), 8.72 (d, *J* = 8.1 Hz, 2H), 8.29 (d, *J* = 8.1 Hz, 2H), 4.54 (t, *J =* 4.6 Hz, 2H), 4.02 (t, *J* = 4.7 Hz, 2H), 2.56 - 1.97 (m, 1H), ¹³C NMR (151 MHz, CDCl₃) δ 166.04, 165.93, 158.00, 135.68, 133.87, 130.55, 128.28, 67.10, 61.31.

### 2-(((4-Nitrophenyl)sulfonyl)oxy)ethyl 4-(1,2,4,5-tetrazin-3-yl)benzoate

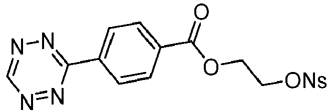

The compound was obtained from 2-hydroxyethyl 4-(1,2,4,5-tetrazin-3-yl)benzoate (0.03 g, 0.12 mmol) as described for compound 20. Purification by flash chromatography (n-Heptane/EtOAc 80/20) afforded 0.035 g (67%) of the desired product as a pink solid. Rf = 0.38 (n-Heptane/EtOAc 70/30); ¹H NMR (600 MHz, CDCl₃) δ 10.31 (s, 1H), 8.71 (d, *J* = 8.5 Hz, 2H), 8.31 (d, *J =* 8.8 Hz, 2H), 8.16 (d, *J =* 8.5 Hz, 2H), 8.14 - 8.09 (m, 2H), 4.64 - 4.58 (m, 2H), 4.58 - 4.53 (m, 2H); ¹³C NMR (151 MHz, CDCl₃) δ 165.77, 165.07, 158.06, 150.73, 141.65, 136.14, 132.92, 130.48, 129.17, 128.27, 124.50, 68.72, 62.28.

### Synthesis of2-(4-(1,2,4,5-Tetrazin-3-yl)phenoxy)ethyl 4-nitrobenzenesulfonate (24)

### 4-(2-Hydroxyethoxy)benzonitrile

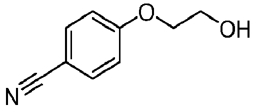

To a solution of 4-hydroxybenzonitrile (1.42 g, 12.00 mmol) and K₂CO₃ (8.29 g, 60.00 mmol) in CH₃CN (20 mL) was added 2-bromoethanol (2.55 ml, 36.00 mmol). The reaction was refluxed for 12 h and then concentrated under reduced pressure. The resulting mixture was diluted with water (50 mL), extracted with EtOAc (3 x 50 mL), washed with brine (50 mL), dried over MgSO₄, filtered and concentrated under reduced pressure. Purification by flash chromatography (n-Heptane/EtOAc 40/60) afforded 1.41 g (72%) of the desired compound as a yellow oil. Rf = 0.18 (n-Heptane/EtOAc 50/50); ¹H NMR (400 MHz, DMSO) δ 7.76 (d, *J* = 8.8 Hz, 2H), 7.11 (d, *J* = 8.8 Hz, 2H), 4.92 (t, *J* = 5.5 Hz, 1H), 4.09 (t, *J* = 5.0 Hz, 2H), 3.73 (q, *J* = 5.0 Hz, 2H), ¹³C NMR (101 MHz, DMSO) δ 162.70, 134.63, 119.64, 116.05, 103.15, 70.56, 59.79.

### 2-(4-(1,2,4,5-tetrazin-3-yl)phenoxy)ethan-1-ol

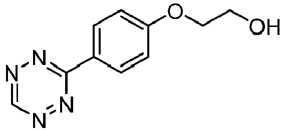

The compound was obtained from 4-(2-hydroxyethoxy)benzonitrile (0.98 g, 6.00 mmol) as described for compound 2. Purification by flash chromatography (n-Heptane/EtOAc 50/50) afforded 0.71 g (54%) of the desired product as a red solid. Rf = 0.27 (n-Heptane/EtOAc 50/50); ¹H NMR (400 MHz, DMSO) δ 10.50 (s, 1H), 8.47 (d, *J* = 9.0 Hz, 2H), 7.23 (d, *J* = 9.0 Hz, 2H), 4.94 (t, *J =* 5.5 Hz, 1H), 4.15 (t, *J* = 4.9 Hz, 2H), 3.78 (q, *J =* 5.2 Hz, 2H); ¹³C NMR (101 MHz, DMSO) δ 165.65, 163.10, 158.16, 130.10, 124.35, 115.95, 70.44, 59.92.

### 2-(4-(1,2,4,5-Tetrazin-3-yl)phenoxy)ethyl 4-nitrobenzenesulfonate

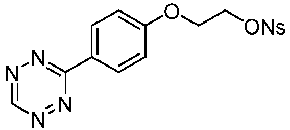

The compound was obtained from 2-(4-(1,2,4,5-tetrazin-3-yl)phenoxy)ethan-1-ol (0.1 g, 0.46 mmol) as described for compound 20. Purification by flash chromatography (n-Heptane/EtOAc 60/40) afforded 0.12 g (65%) of the desired product as a red solid. Rf = 0.41 (n-Heptane/EtOAc 50/50); ¹H NMR (400 MHz, DMSO) δ 10.52 (s, 1H), 8.97 - 8.31 (m, 5H), 8.33 - 7.87 (m, 2H), 7.11 (d, *J =* 8.9 Hz, 2H), 4.97 - 4.50 (m, 2H), 4.48 - 4.06 (m, 2H); ¹³C NMR (101 MHz, DMSO) δ 165.57, 161.88, 158.28, 154.85, 147.71, 130.25, 127.38, 125.26, 123.79, 116.05, 75.01, 67.33.

### Synthesis of compound:2-((6-(6-(pyridin-2-yl)-1,2,4,5-tetrazin-3-yl)pyridin-3-yl)methoxy)ethyl 4-nitrobenzenesulfonate (25)

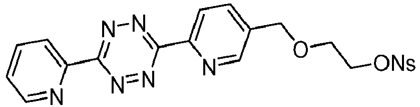

To a solution of 2-((6-(6-(Pyridin-2-yl)-1,2,4,5-tetrazin-3-yl)pyridin-3-yl)methoxy)ethan-1-ol from Example 4 (0.05 g, 0.17 mmol) and TEA (0.94 mL, 0.67 mmol) in CH₂Cl₂ (20 mL) was added Nosyl chloride (0.56 g, 0.25 mmol) and DMAP (0.002 g, 0.017 mmol). The reaction was stirred at room temperature for 4 hours. The solvent was then evaporated under reduced pressure. Purification by flash chromatography (CH₂Cl₂/MeOH 98/2) afforded 0.07 g (84%) of the desired compound as a red oil. Rf = 0.48 (CH₂Cl₂/MeOH 90/10); ¹H NMR (600 MHz, CDCl₃) δ 9.02 (dt, *J* = 4.5, 1.4 Hz, 1H), 8.86 (d, *J* = 2.1 Hz, 1H), 8.80 - 8.63 (m, 2H), 8.41 - 8.33 (m, 1H), 8.17 - 8.11 (m, 1H), 8.04 (td, *J* = 7.7, 1.8 Hz, 1H), 7.93 (dd, *J* = 8.0, 2.2 Hz, 1H), 7.61 (ddd, *J* = 7.6, 4.7, 1.1 Hz, 1H), 4.69 (s, 2H), 4.44 - 4.38 (m, 2H), 3.90 - 3.82 (m, 2H); ¹³C NMR (151 MHz, CDCl₃) δ 163.90, 163.68, 151.09, 150.76, 150.11, 150.00, 149.76, 141.81, 137.50, 136.40, 136.21, 129.25, 126.62, 124.58, 124.41, 124.22, 70.44, 70.15, 68.20.

### Synthesis of compound:2-(((4-Nitrophenyl)sulfonyl)oxy)ethyl 6-(6-(pyridin-2-yl)-1,2,4,5-tetrazin-3-yl)nicotinate (26)

### 2-Hydroxyethyl 6-(6-(pyridin-2-yl)-1,2,4,5-tetrazin-3-yl)nicotinate

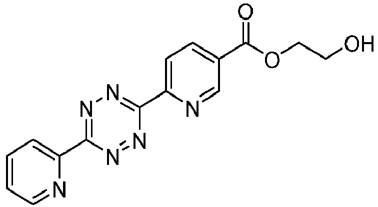

The compound was obtained from 6-(6-(Pyridin-2-yl)-1,2,4,5-tetrazin-3-yl)nicotinic acid from compound 6 (0.04 g, 0.14 mmol) as reported for compound 21. Purification by flash chromatography (CH₂Cl₂/MeOH 90/10) afforded 0.035 g (76%) of the desired compound as a red solid. The compound is pure enough for the next step. Rf = 0.31 (CH₂Cl₂/MeOH 90/10); ¹H NMR (400 MHz, CDCl₃) δ 9.47 (dd, *J* = 2.1, 0.9 Hz, 1H), 9.01 - 8.90 (m, 1H), 8.86 - 8.74 (m, 1H), 8.70 (dd, *J* = 7.9, 1.1 Hz, 1H), 8.55 (dd, *J* = 8.2, 2.1 Hz, 1H), 7.96 (ddt, *J* = 10.4, 7.7, 2.2 Hz, 1H), 7.62 - 7.48 (m, 1H), 4.76 - 4.45 (m, 2H), 4.26 - 3.68 (m, 2H), 3.24 (s, 1H).

### 2-(((4-Nitrophenyl)sulfonyl)oxy)ethyl 6-(6-(pyridin-2-yl)-1,2,4,5-tetrazin-3-yl)nicotinate

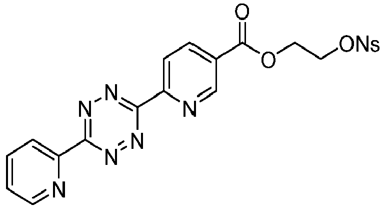

The compound was obtained 2-hydroxyethyl 6-(6-(pyridin-2-yl)-1,2,4,5-tetrazin-3-yl)nicotinate (0.035 g, 0.11 mmol) as reported for compound 20. Purification by flash chromatography (CH₂Cl₂/MeOH 95/5) afforded 0.035 g (64%) of the desired compound as a red solid. Rf = 0.35 (CH₂Cl₂/MeOH 95/5); ¹H NMR (400 MHz, DMSO) δ 9.25 (d, *J* = 2.2 Hz, 1H), 8.76 (d, *J* = 8.4 Hz, 2H), 8.66 (d, *J* = 7.8 Hz, 1H), 8.50 (dd, *J* = 8.2, 2.2 Hz, 1H), 8.40 (d, *J* = 8.9 Hz, 2H), 8.24 (d, *J =* 8.9 Hz, 2H), 8.22 - 8.17 (m, 1H), 7.87 - 7.68 (m, 1H), 4.63 (s, 4H); ¹³C NMR (101 MHz, DMSO) δ 164.1, 163.71, 163.51, 154.88, 154.14, 151.23, 150.39, 141.06, 139.06, 138.39, 129.87, 127.37, 125.42, 125.08, 124.59, 123.78, 70.42, 63.28.

### Synthesis of compound:2-((6-(6-(Pyridin-2-yl)-1,2,4,5-tetrazin-3-yl)pyridin-3-yl)oxy)ethyl 4-nitrobenzenesulfonate (27)

### 5-(2-Hydroxyethoxy)picolinonitrile

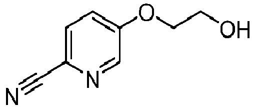

The compound was obtained from 5-hydroxypicolinonitrile (1.4 g, 11.65 mmol) as described for compound 24. Purification by flash chromatography (n-Heptane/EtOAc 40/60) afforded 1.45 g (76%) of the desired product as a pink solid. Rf = 0.21 (Heptane/EtOAc 40/60); ¹H NMR (400 MHz, CDCl₃) δ 8.32 (d, *J* = 2.9 Hz, 1H), 7.59 (d, *J* = 8.6 Hz, 1H), 7.42 - 6.71 (m, 1H), 4.66 - 4.05 (m, 2H), 3.97 (dd, *J* = 5.1, 3.9 Hz, 2H), 2.27 (s, 1H); ¹³C NMR (101 MHz, CDCl₃) δ 157.27, 140.42, 129.65, 125.41, 120.58, 117.39, 70.21, 60.86.

### 2-((6-(6-(Pyridin-2-yl)-1,2,4,5-tetrazin-3-yl)pyridin-3-yl)oxy)ethan-1-ol

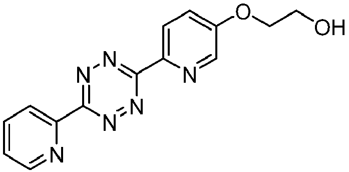

The compound was obtained from 5-(2-Hydroxyethoxy)picolinonitrile (0.5 g, 3.04 mmol) as described for compound 5. Purification by flash chromatography (CH₂Cl₂/MeOH 97/3) afforded 0.27 g (30%) of the desired product as a pink solid. Rf = 0.25 (CH₂Cl₂/MeOH 95/5); ¹H NMR (600 MHz, DMSO) δ 8.93 (ddd, *J* = 4.7, 1.8, 0.9 Hz, 1H), 8.65 (dd, *J* = 2.9, 0.6 Hz, 1H), 8.62 - 8.49 (m, 2H), 8.16 (td, *J* = 7.7, 1.8 Hz, 1H), 7.82 - 7.56 (m, 2H), 5.00 (s, 1H), 4.50 - 3.97 (m, 2H), 3.81 (td, *J* = 5.4, 4.3 Hz, 2H); ¹³C NMR (151 MHz, DMSO) δ 163.49, 163.30, 157.81, 151.05, 150.71, 142.37, 139.97, 138.25, 127.00, 126.05, 124.59, 121.95, 70.96, 59.88.

### 2-((6-(6-(Pyridin-2-yl)-1,2,4,5-tetrazin-3-yl)pyridin-3-yl)oxy)ethyl 4-nitrobenzenesulfonate

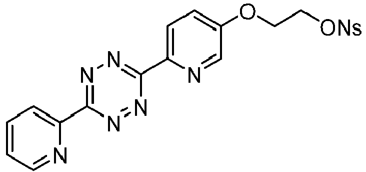

The compound was obtained 2-((6-(6-(pyridin-2-yl)-1,2,4,5-tetrazin-3-yl)pyridin-3-yl)oxy)ethan-1-ol (0.05 g, 0.17 mmol) as reported for compound 20. Purification by flash chromatography (CH₂Cl₂/MeOH 98/2) afforded 0.03 g (37%) of the desired compound as a red solid (mixture of conformers 70/30). Rf = 0.61 (CH₂Cl₂/MeOH 90/10); ¹H NMR (600 MHz, DMSO) δ 8.94 (ddd, *J* = 4.7, 1.8, 0.9 Hz, 1H), 8.70 (d, *J* = 2.9 Hz, 0.3H), 8.65 (d, *J* = 8.5 Hz, 0.3H), 8.60 (d, *J =* 7.8 Hz, 1H), 8.56 (d, *J* = 8.8 Hz, 0.7H), 8.51 (d, *J* = 2.9 Hz, 0.7H), 8.47 - 8.43 (m, 1.4H), 8.27 - 8.22 (m, 1.4H), 8.21 - 8.18 (m, 0.6H), 8.16 (td, *J* = 7.8, 1.8 Hz, 1H), 7.90 - 7.81 (m, 0.6H), 7.78 (dd, *J* = 8.8, 2.9 Hz, 0.3H), 7.73 (ddd, *J* = 7.6, 4.7, 1.2 Hz, 1H), 7.64 (dd, *J* = 8.8, 3.0 Hz, 0.7H), 4.81 - 4.71 (m, 0.6H), 4.68 - 4.59 (m, 1.4H), 4.57 - 4.54 (m, 0.6H), 4.51 - 4.43 (m, 1.4H); ¹³C NMR (151 MHz, DMSO) δ 163.57, 163.53, 163.26, 163.21, 156.74, 156.60, 154.89, 151.16, 151.08, 150.66, 147.70, 143.22, 142.95, 140.95, 139.86, 139.77, 138.28, 129.93, 127.38, 127.08, 127.05, 126.08, 125.89, 125.43, 124.63, 123.78, 122.36, 122.12, 74.80, 70.58, 67.80, 66.45.

### Synthesis of compound:Di-tert-butyl 2,2'-((2-(2-(((4-nitrophenyl)sulfonyl)oxy)ethoxy)-4-(1,2,4,5-tetrazin-3-yl)benzyl)azanediyl)diacetate (28)

### 3-(2-Hydroxyethoxy)-4-methylbenzonitrile

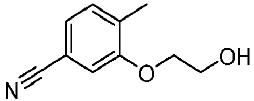

3-Hydroxy-4-methylbenzonitrile (2.0 g, 10.0 mmol) was dissolved in NaOH aqueous solution (25 mL, 15.0 mmol), 2-bromoethanol (1.06 mL, 15.0 mmol) was added. The resulting mixture was heated at 90 °C for 12 hours. The reaction was then cooled to room temperature, diluted with water (30 mL) and extracted with CH₂Cl₂ (2 x 50 mL), The organic layer was washed with 10% NaOH (2 x 30 mL), water (30 mL) and brine (30 mL) and dried over Na₂SO₄. The solvent was evaporated under reduced pressure to give 1.72 (65%) of the desired compound as white solid. Rf = 0.25 (n-Heptane/EtOAc 70/30); ¹H NMR (400 MHz, CDCl₃) δ 7.24 - 7.15 (m, 2H), 7.04 (d, *J* = 1.4 Hz, 1H), 4.10 (dd, *J* = 5.1, 3.8 Hz, 2H), 4.01 (dd, *J* = 5.2, 3.7 Hz, 2H), 2.29 (s, 3H), 1.95 (s, 1H); ¹³C NMR (101 MHz, CDCl₃) δ 156.81, 133.17, 131.38, 125.01, 119.05, 113.82, 110.30, 69.70, 61.30, 16.65.

### 4-(Bromomethyl)-3-(2-hydroxyethoxy)benzonitrile

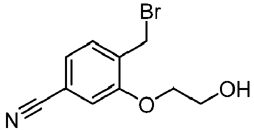

To a solution of 3-(2-hydroxyethoxy)-4-methylbenzonitrile (1.30 g, 7.33 mmol) and N-bromosuccinmide (1.43g, 8.07 mmol) in CHCl₃ (40 mL) was added AIBN (0.48 g, 2.93 mmol). The reaction was refluxed for 24 h. The solvent was removed under vacuum and the crude purified by flash chromatography (n-Heptane/EtOAc 80/20) to give 1.10g (58%) of the desired compound as a white solid. Rf = 0.25 (n-Heptane/EtOAc 70/30); ¹H NMR (400 MHz, CDCl₃) δ 7.42 (d, *J* = 7.8 Hz, 1H), 7.30 - 7.22 (m, 1H), 7.13 (d, *J* = 1.4 Hz, 1H), 4.53 (s, 2H), 4.21 (dd, *J* = 4.9, 3.8 Hz, 2H), 4.04 (d, *J* = 4.5 Hz, 2H); ¹³C NMR (101 MHz, CDCl₃) δ 156.70, 131.64, 131.33, 125.11, 118.24, 114.93, 113.64, 70.36, 61.13.

### Di-tert-butyl 2,2'-((4-cyano-2-(2-hydroxyethoxy)benzyl)azanediyl)diacetate

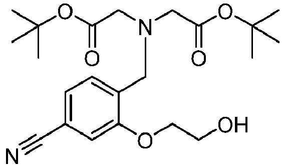

The compound was obtained from 4-(bromomethyl)-3-(2-hydroxyethoxy)benzonitrile (0.90 g, 3.51 mmol) as reported for compound 12. Purification by flash chromatography (n-Heptane/EtOAc 60/40) afforded 0.90 g (61 %) of the desired compound as a colorless oil. Rf = 0.24 (n-Heptane/EtOAc 60/40); ¹H NMR (400 MHz, CDCl₃) δ 7.35 (d, *J* = 7.7 Hz, 1H), 7.16 (dd, *J* = 7.7, 1.4 Hz, 1H), 7.04 (d, *J =* 1.5 Hz, 1H), 4.08 (dd, *J* = 4.8, 3.2 Hz, 2H), 3.91 (s, 2H), 3.87 (t, *J* = 4.7 Hz, 2H), 3.32 (s, 4H), 1.38 (s, 18H); ¹³C NMR (101 MHz, CDCl₃) δ 170.47, 157.89, 132.74, 132.00, 124.71, 118.70, 115.34, 112.32, 81.39, 71.02, 60.86, 55.66, 52.48, 28.12.

### Di-tert-butyl 2,2'-((2-(2-hydroxyethoxy)-4-(1,2,4,5-tetrazin-3-yl)benzyl)azanediyl)diacetate

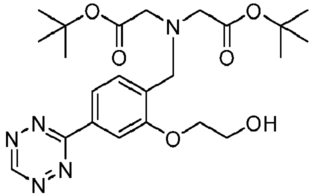

The compound was obtained from di-*tert*-butyl 2,2'-((4-cyano-2-(2-hydroxyethoxy)benzyl)-azanediyl)diacetate (0.9 g, 2.14 mmol) as reported for compound 2. Purification by flash chromatography (n-Heptane/EtOAc 70/30) afforded 0.26 g (25%) of the desired compound as a red oil (obtained with a 20% of inseparable impurity). Rf = 0.21 (n-Heptane/EtOAc 60/40); ¹H NMR (400 MHz, CDCl₃) δ 10.13 (s, 1H), 8.14 (dd, *J* = 7.9, 1.6 Hz, 1H), 8.07 (d, *J =* 1.6 Hz, 1H), 7.44 (d, *J =* 7.8 Hz, 1H), 4.24 (dd, *J* = 4.9, 3.2 Hz, 2H), 4.00 (s, 2H), 3.93 - 3.87 (m, 2H), 3.39 (s, 4H), 2.29 (s, 1H), 1.39 (s, 18H); ¹³C NMR (101 MHz, CDCl₃) δ 170.54, 166.17, 158.69, 157.78, 132.53, 132.50, 132.24, 120.90, 111.86, 81.31, 71.13, 61.09, 55.60, 52.65, 28.16.

### Di-tert-butyl 2,2'-((2-(2-(((4-nitrophenyl)sulfonyl)oxy)ethoxy)-4-(1,2,4,5-tetrazin-3-yl)benzyl) azanediyl)diacetate

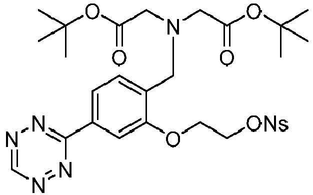

To a solution of di-*tert*-butyl 2,2'-((2-(2-hydroxyethoxy)-4-(1,2,4,5-tetrazin-3-yl)benzyl)azanediyl)-diacetate (0.09 g, 0.19 mmol) and DIPEA (0.13 mL, 0.75 mmol) in CH₂Cl₂ (20 mL) was added Nosyl chloride (0.12 g, 0.57 mmol) and DMAP (0.005 g, 0.04 mmol). The reaction was stirred at room temperature for 4 hours. The solvent was then evaporated under reduced pressure. Purification by flash chromatography (n-Heptane/EtOAc75/25) afforded 0.075 g (60%) of the desired compound as a red oil. Rf = 0.31 (n-Heptane/EtOAc 60/40); ¹H NMR (400 MHz, CDCl₃) δ 10.20 (s, 1H), 8.41 (d, *J* = 8.8 Hz, 2H), 8.27 (dd, *J* = 8.0, 1.5 Hz, 1H), 8.18 (d, *J* = 8.8 Hz, 2H), 7.96 (d, *J* = 1.5 Hz, 1H), 7.80 (d, *J* = 8.0 Hz, 1H), 4.56 (dd, *J* = 5.7, 3.4 Hz, 2H), 4.36 (dd, *J* = 5.7, 3.4 Hz, 2H), 3.97 (s, 2H), 3.47 (s, 4H), 1.46 (s, 18H); ¹³C NMR (101 MHz, CDCl₃) δ 170.56, 166.04, 157.79, 156.63, 150.85, 141.68, 133.96, 131.25, 131.05, 129.32, 124.61, 121.94, 110.49, 81.10, 69.02, 65.97, 55.74, 51.88, 28.18; HPLC-MS [M+H]⁺ m/z calc. for [C₂₉H₃₇N₆O₁₀S]⁺: 661.23; Found: 661.24.

### Synthesis of compound:Di-tert-butyl 2,2'-(((6-(6-(5-(2-(((4-nitrophenyl)sulfonyl)oxy)ethoxy)pyridin-2-yl)-1,2,4,5-tetrazin-3-yl)pyridin-3-yl)methyl)azanediyl)diacetate (29)

### Di-tert-butyl 2,2'-(((6-(6-(5-(2-hydroxyethoxy)pyridin-2-yl)-1,2,4,5-tetrazin-3-yl)pyridin-3-yl)methyl)azanediyl)diacetate

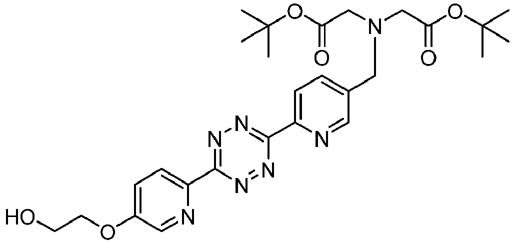

Di-tert-butyl 2,2'-(((6-cyanopyridin-3-yl)methyl)azanediyl)diacetate from compound 14 (1.05 g, 2.92 mmol), 4-(2-Hydroxyethoxy)benzonitrile from compound 27 (0.12 g, 0.73 mmol) and sulfur (0.05 g, 0.18 mmol) were suspended in EtOH (3 mL), followed by the addition of hydrazine hydrate (0.53 mL, 10.96 mmol). The reaction was heated to 90 °C for 2 h. The mixture was cooled to room temperature and the formed precipitate was removed by filtration. Water (10 mL) and a solution of NaNO₂ (1.0 g, 14.62 mmol) in water 10 mL were added and the mixture was cautiously acidified to pH 2 by addition of AcOH. The mixture was extracted with DCM and the combined organic layer was washed with water and brine, dried over MgSO₄ and concentrated. The residue was purified by flash column chromatography (CH₂Cl₂/MeOH 95/5) to give 0.11 g (27%) of the desired compound as a pink solid. Rf = 0.33 (CH₂Cl₂/MeOH 95/5); ¹H NMR (400 MHz, MeOD) δ 8.88 (d, *J* = 2.0 Hz, 1H), 8.81 - 8.69 (m, 2H), 8.56 (d, *J* = 2.9 Hz, 1H), 8.22 (dd, *J* = 8.1, 2.1 Hz, 1H), 7.71 (dd, *J* = 8.9, 2.9 Hz, 1H), 4.45 - 4.25 (m, 2H), 4.10 (s, 2H), 4.04 - 3.93 (m, 2H), 3.50 (s, 4H), 3.33 (t, *J* = 1.6 Hz, 1H), 1.50 (s, 18H); ¹³C NMR (101 MHz, MeOD) δ 170.55, 163.15, 163.04, 158.24, 150.67, 148.89, 141.66, 139.29, 138.58, 138.49, 125.57, 123.75, 121.49, 81.08, 70.31, 59.99, 55.12, 54.74, 27.04.

### Di-tert-butyl 2,2'-(((6-(6-(5-(2-(((4-nitrophenyl)sulfonyl)oxy)ethoxy)pyridin-2-yl)-1,2,4,5-tetrazin-3-yl)pyridin-3-yl)methyl)azanediyl)diacetate

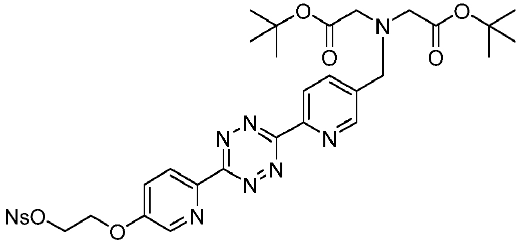

The compound was obtained Di-tert-butyl 2,2'-(((6-(6-(5-(2-hydroxyethoxy)pyridin-2-yl)-1,2,4,5-tetrazin-3-yl)pyridin-3-yl)methyl)azanediyl)diacetate (0.05 g, 0.09 mmol) as reported for compound 20. Purification by flash chromatography (Heptane/EtOAc 40/60) afforded 0.045 g (67%) of the desired compound as a red solid. Rf = 0.26 (Heptane/EtOAc 30/70); ¹H NMR (600 MHz, CDCl₃) δ 8.82 (d, *J* = 2.1 Hz, 1H), 8.70 - 8.59 (m, 2H), 8.48 (d, *J* = 2.9 Hz, 1H), 8.35 (d, *J* = 8.8 Hz, 2H), 8.15 - 7.98 (m, 3H), 7.32 (d, *J* = 2.9 Hz, 1H), 4.76 - 4.47 (m, 2H), 4.41 - 4.31 (m, 2H), 4.01 (s, 2H), 3.40 (s, 4H), 1.41 (s, 18H); ¹³C NMR (151 MHz, CDCl₃) δ 169.22, 162.64, 162.15, 155.32, 150.32, 149.95, 148.26, 142.30, 140.49, 138.34, 137.23, 137.03, 128.33, 124.54, 123.55, 123.24, 120.47, 80.37, 67.51, 65.00, 54.31, 53.61, 27.18.

### Example 9 Radiochemistry

### [¹⁸F]Fluoride production and general methods

[¹⁸F]Fluoride was produced by a cyclotron CTI Siemens Eclipse Rigshospitalet, Denmark, by irradiating [¹⁸O]H₂O via a (p,n) reaction. Automated synthesis was performed on a Scanys synthesis module (Scansys Laboratorieteknik, Denmark) and analytical HPLC was performed on a Thermo Fisher UltiMate 3000 equipped with a C18 column (Luna 5 µm C18(2) 100 A, 150 mm × 4.6 mm). Eluents: A, H₂O with 0.1% TFA; B, MeCN with 0.1% TFA. Gradient from 100% A to 100% B over 15 min, back to 100% A over 4 min, flow rate 1.5 mL/min. Detection by UV absorption at λ = 254 nm on a UVD 170U detector and radioactivity was analyzed with a flow-through GM tube based radiodetector (Scansys).

### Radiolabeling

The aqueous [¹⁸F]fluoride solution received from the cyclotron was passed through a preconditioned anion exchange resin (Sep-Pak Light QMA cartridge). The QMA was preconditioned by flushing it with 10 mL 0.5 M K₃PO₄ and washing it with 10 mL H₂O afterwards. [¹⁸F]F⁻ was eluted from the QMA into a 4 mL v-shaped vial with 1 mL Bu₄NOMs dissolved in MeOH. The eluate was dried at 100 °C for 5 min under N₂-flow. Compound **28** was dissolved in 167 µL DMSO and then diluted with 833 µL tBuOH. The solution was added to the dried fluoride solution an allowed to react for 5 min at 100 °C. The reaction was cooled to 50 °C with air before addition of 3 mL H₂O. This mixture was applied to a Sep-pak plus C18 solid phase extraction (SPE) cartridge that was preconditioned by flushing it with 10 mL EtOH followed by 10 mL of H₂O. The SPE was flushed with another 5 mL of H₂O and dried with N₂. The intermediate was eluted from the SPE with 2 mL MeCN into a 7 mL v-shaped vial containing 600 µL TFA. This mixture was reacted for 10 min at 80 °C. The reaction was then concentrated under N₂-flow for 20 min to reduce the solvent volume to <0.1 mL. To this crude product mixtre, 2.5 mL of H₂O was added, and this solution purified by semipreparative HPLC (Luna 5 µm C18(2) 100 Å, 250 mm × 10 mm, isocratic, 70% EtOH in H₂O with 0.1% TFA 3 mL/min (rt: 13 min). The product ([18F]13) was collected in a 20 mL vial and diluted with 100 mM sterile phosphate buffer to adjust the pH to 5-8. The max EtOH concentration was 5% and the activity concentration was 30-80 MBq/mL. [¹⁸F]**13** was labeled in a radiochemical yield (RCY) of 13 ± 4 % (n = 3) with a radiochemical purity (RCP) of >98% and a molar activity (A_{M}) of 55 ± 8 GBqlµmol. The total synthesis was approximately 90 min including separation and formulation of the final product. Maximum isolated amount was 1.1 GBq (Figure 4).

### Tetrazine core reactivity test

The reaction between [¹⁸F]13 TCO-PNP was performed by mixing the formulated [¹⁸F]**13** (200 µL) with 5 µL of the commercially available TCO-PNP ester dissolved in DMF (5 mg/mL) in an analytical HPLC vial. The solution was gently shaken and left for 1 min before it was injected on the analytical HPLC for analysis.

### Example 10 Pretargeted Imaging

Pretargeted imaging of [¹⁸F]**13** as tested *in vivo* using the TAG-72 targeting antibody CC49 in human colorectal cancer xenograft tumors LS174T. Tumors were established in 7-8 week old Balb/c nude mice and after one week, the animals were injected i.v. with either 50 ug (3.4 nmol) TCO-modified CC49 (CC49-TCO, ~7 TCO/mAb), or non-modified CC49 (control) (n = 4 per group). 72 hours later, ¹⁸F-13 (1.74 ± 0.319 (mean ± SD) MBq/100 uL (55.3 GBq/µmol) were injected i.v., followed by PET/CT-scan (Inveon^{®}, Siemens Medical Solutions), one hour post injection. PET data was acquired in an energy window of 350-650 KeV and a time resolution of 6 ns followed by a CT scan (360 projections, 65 kV, 500 µA and 400 ms). PET and CT images were aligned by rigid affine registration, after which 3D regions of interest (ROI) were created on the full CT tumor volume, as well as heart and muscle tissue to quantify uptake of [¹⁸F]**13** (Figure 6). Additionally, selected organs were harvested for *ex vivo* biodistribution. The radioactivity in the tissues were determined using a gamma counter (Wizard², Perkin Elmer). Data was corrected for decay, tissue weight and the injected dose of radioactivity. GraphPad Prism 9 (GraphPad Software) was used for statistical analyses and plotting data. Statistical difference between mean %ID/g values were analyzed using Welch's T-test. Results were considered significant when *p* < 0.05.

## Claims

1. A method for aliphatic ¹⁸F-labelling of a precursor comprising the steps of:
a) Pre-conditioning an anion exchange cartridge by flushing the cartridge with a solution comprising a non-nucleophilic anion selected from the group comprising phosphate, hydrogen phosphate or dihydrogen phosphate
b) Trapping ¹⁸F ions on the anion exchange cartridge by passing an aqueous ¹⁸F fluoride solution through the anion exchange cartridge
c) Eluting the ¹⁸F ions by using a solution comprising a non-basic anion selected from the group comprising sulfonate esters such as MsO⁻, TsO⁻ or TfO⁻ in combination with a suitable counterion such as Bu₄N⁺ or K⁺/K₂₂₂.
d) Removing the solvent from the eluate from step c) by subjecting the eluate to a drying step
e) Labelling of the precursor molecule with the dried ¹⁸F from step d) in a solvent selected from the group comprising sterically hindered polar protic solvents such as *t*-BuOH, amyl alcohol or Thexyl alcohol or any combination thereof
wherein, the precursor molecule is a tetrazine compound with a reaction kinetic constant in the range of 30.000 M⁻¹ S⁻¹ to 200.000 M⁻¹ S⁻¹ for reacting with unsubstituted TCO measured in PBS at 37 °c and wherein the tetrazine compound comprises at least one aliphatic group comprising a leaving group for nucleophilic substitution.

2. A method according to claim 1 wherein the non-basic anion of step c) is selected from the group consisting of Bu₄NH₂PO₄, Bu₄NOMs and Bu₄NOTf.

3. A method according to any of the preceding claims wherein the labelling of the precursor molecule in step e) is performed in a solvent mixture comprising at least one sterically hindered polar protic solvent such as *t-*BuOH, amyl alcohol or Thexyl alcohol in combination with at least one polar aprotic solvent such as DMSO, MeCN, DMF, NMP or DMA.

4. A tetrazine compound, having the formula I: 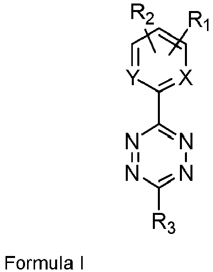
wherein R1 is H or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
wherein, R3 is H or 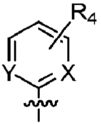 and wherein, X and Y are independently selected from: -CH₂- and -N- and wherein the curly sign indicates the link to the tetrazine,
and wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein, at least one of R2 or R4 is a moiety and wherein R5 is H or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 18F or 19F labelled C1-C6 aliphatic groups comprising fluoromethyl, fluoroethyl, 1-fluoropropyl, 1-fluorobutyl, 1-fluoropentyl, 1-fluorohexyl and wherein the tetrazine compound of formula I has a lipophilicity of cLogD_{7.4} < -0.5.

5. A Tetrazine compound according to claim 4 wherein R1 is a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and wherein R1 is connected to the Tetrazine compound by means of direct covalent bonding to the aromatic ring or connected to the aromatic ring via COO, (CH₂)ₙCOO, CONH, (CH₂)ₙCNH, CONCH₃, (CH₂)ₙCONCH₃, O, (CH₂)ₙO, NH, (CH₂)ₙNH, NCH₃, (CH₂)ₙNCH₃, S or (CH₂)ₙS where n is 1, 2, 3, 4 or wherein R5 is a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl and wherein R5 is connected to the Tetrazine compound by means of conjugation to a benzylic amine via direct covalent bonding or via COO, (CH2)nCOO, CONH, (CH2)nCNH, CONCH3, (CH2)nCONCH3, (CH2)nO, (CH2)nNH, (CH2)nNCH3, or (CH2)nS wherein n is 1, 2, 3, 4.

6. A tetrazine compound according to any of claim 4 or claim 5, wherein the moiety is selected from: -OH, NR6R7, CH2N(CH2COOH)2, CH2NHCH2COOH, CH2NR7CH2COOH, COOH, CONR6R7, SO3H, SO2NH2, and SO2NH wherein R6 is H, CH3, CH2CH3, CH2CH2CH3 or H2COOH; and wherein R7 is H, CH3, CH2CH3, CH2CH2CH3, CH2COOH, or a group selected from the group consisting of 18F or 19F labelled C1-C6 aliphatic groups including fluoromethyl, fluoroethyl, 1-fluoropropyl, 1-fluorobutyl, 1-fluoropentyl, 1-fluorohexyl.

7. Tetrazine compounds according to any of claim 4 to claim 6 having the formula

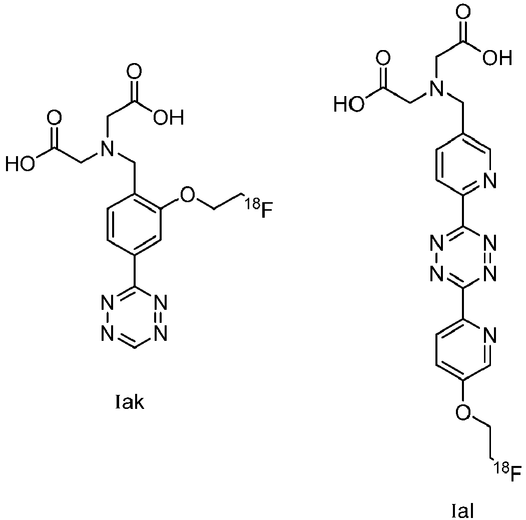

8. A precursor molecule, having the formula II: 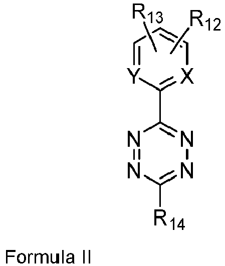
wherein R12 is H or an C1-C6 aliphatic group such as methyl, ethyl, propyl, butyl, pentyl, hexyl comprising a sulfonate leaving group having any suitable substituent such as triflate, nosylate, mesylate or tosylate and wherein, X and Y are independently selected from: -CH₂- and -N- and
wherein, R14 is H or 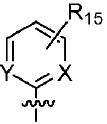 and wherein, X and Y are independently selected from: -CH₂- and -N- and wherein the curly sign indicates the link to the tetrazine,
And wherein R13 and R15 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R16, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein, at least one of R13 or R15 is a moiety and wherein R16 is H or C1-C6 aliphatic group such as methyl, ethyl, propyl, butyl, pentyl, hexyl comprising a sulfonate leaving group having any suitable substituent such as triflate, nosylate, mesylate or tosylate and wherein, at least one of R12 and R16 is an aliphatic group comprising a sulfonate leaving group having any suitable substituent such as triflate, nosylate, mesylate or tosylate and wherein, any functional groups comprising OH, NH or SH are substituted with a protective group selected from the group comprising Boc, Fmoc, CH2C5H5, (CH2)nCH3, C(CH3)3, COCH3, COCF3, C(C5H5)3 and wherein, the precursor molecule has a lipophilicity of ClogD_{7.4} < -0.5 after deprotection .

9. A precursor molecule according to claim 8 having the formula

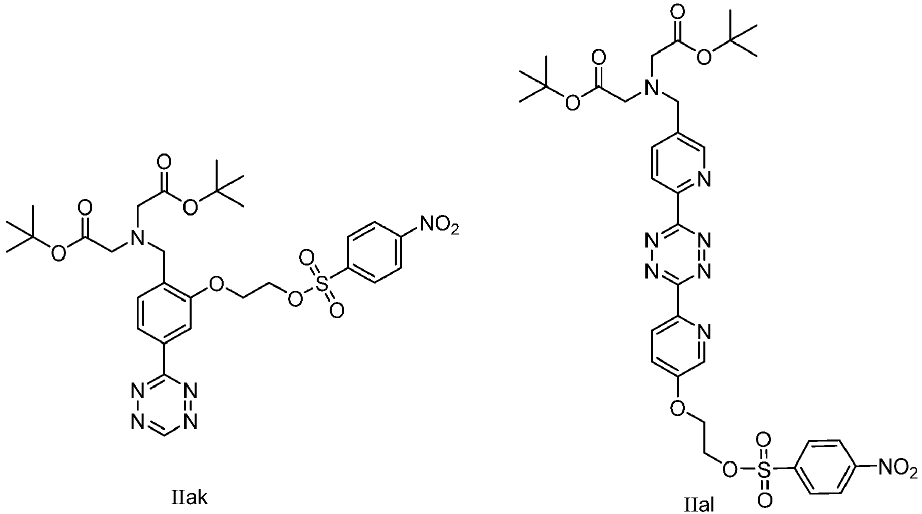

10. Use of a tetrazine compound for PET imaging and/or diagnostics, wherein said A tetrazine compound, having the formula III: 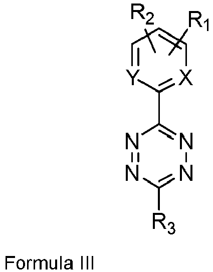
wherein R1 is a group selected from the group consisting of 18F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
wherein, R3 is H or 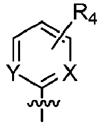 and wherein, X and Y are independently selected from: -CH₂- and -N- and wherein the curly sign indicates the link to the tetrazine,
and wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R5 is H or a group selected from the group consisting of labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a labelled C1-C6 aliphatic groups comprising fluoromethyl, fluoroethyl, 1-fluoropropyl, 1-fluorobutyl, 1-fluoropentyl, 1-fluorohexyl and wherein, the tetrazine compound of formula I has a lipophilicity of ClogD_{7.4} < - 0.5.

11. Use of a Tetrazine compound according to any of claim 4 to claim 7 for PET imaging and/or diagnotics wherein the tetrazine compound is labelled with 18F.

12. Use of a Tetrazine compound for quality control, wherein the Tetrazine compound, having the formula IV: 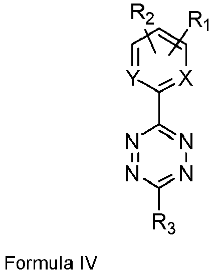
wherein R1 is a group selected from the group consisting of 19F labelled C1-C6 aliphatic groups including fluoromethyl, fluoroethyl, 1-fluoropropyl, 1-fluorobutyl, 1-fluoropentyl, 1-fluorohexyl and wherein, X and Y are independently selected from: -CH₂- and -N- and
wherein, R3 is H or 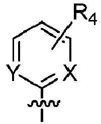 and wherein, X and Y are independently selected from: -CH₂- and -N- and wherein the curly sign indicates the link to the tetrazine, and wherein R2 and R4 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R5, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R5 is H or a group selected from the group consisting of 19F labelled C1-C6 aliphatic groups including fluoromethyl, fluoroethyl, 1-fluoropropyl, 1-fluorobutyl, 1-fluoropentyl, 1-fluorohexyl and wherein at least one of R1 and R5 is a 19F labelled C1-C6 aliphatic groups comprising fluoromethyl, fluoroethyl, 1-fluoropropyl, 1-fluorobutyl, 1-fluoropentyl, 1-fluorohexyl and wherein, the tetrazine compound of formula I has a lipophilicity of ClogD_{7.4} < - 0.5.

13. Use of a tetrazine compound according to any of claim 4 to claim 7 for quality control wherein the tetrazine compound is labelled with 19F.

14. Use of a precursor molecule for aliphatic ¹⁸F-labelling according to the method described in any of claim 1 to 3, wherein the precursor molecule, have the formula V: 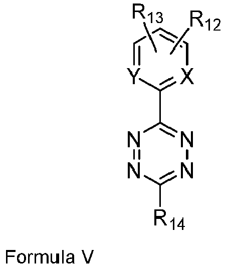
wherein R12 is an C1-C6 aliphatic group such as methyl, ethyl, propyl, butyl, pentyl, hexyl comprising a sulfonate leaving group having any suitable substituent such as triflate, nosylate, mesylate or tosylate and wherein, X and Y are independently selected from: -CH₂- and -N- and
wherein, R14 is H or 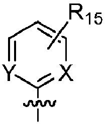 and wherein, X and Y are independently selected from: -CH₂- and -N- and wherein the curly sign indicates the link to the tetrazine,
and wherein R13 and R15 are independently selected from H or a moiety selected from the group consisting of a hydroxy group, a sulfonamide, a carboxyl group, a sulfonyl group, amine, a substituted amine with 1-5 polyethylene glycol unit(s), a -(O-CH2-CH2)1-5-OCH2-COOH, Methyl, Ethyl, Propyl, optionally substituted heteroaryl, and optionally substituted arylalkyl; wherein optionally substituted in relation to said substituted amine means one or more substituents selected from R16, a halogen, a hydroxy group, a sulfonamide, a carboxyl group, a sulfonyl group, amine, (C1-C10) alkyl, (C2-C10)alkenyl, (C2-C10)alkynyl, (C1-C10)alkylene, (C1-C10)alkoxy, (C2-C10)dialkylamino, (C1-C10)alkylthio, (C2-C10)heteroalkyl, (C2-C10)heteroalkylene, (C3-30 C10)cycloalkyl, (C3-C10)heterocycloalkyl, (C3-10)cycloalkylene, (C3-C10)heterocycloalkylene, (C1-C10)haloalkyl, (C1-C10)perhaloalkyl, (C2-C10)-alkenyloxy, (C3-C10)-alkynyloxy, aryloxy, arylalkyloxy, heteroaryloxy, heteroarylalkyloxy, (C1-C6)alkyloxy-(C1-C4)alkyl, optionally substituted aryl, optionally substituted heteroaryl, and optionally substituted arylalkyl; wherein optionally substituted means one or more substituents selected from a halogen, a hydroxy group, a sulfonamide, a carboxyl group, a sulfonyl group, amine, a substituted amine with 1-5 polyethylene glycol unit(s), a -(O-CH2-CH2)1-5-OCH2-COOH, H, Methyl, Ethyl, Propyl, optionally substituted heteroaryl, and optionally substituted arylalkyl; wherein optionally substituted in relation to said substituted amine means one or more substituents selected from a halogen, a hydroxy group, a sulfonamide, a carboxyl group, a sulfonyl group, amine
and wherein R16 is H or C1-C6 aliphatic group such as methyl, ethyl, propyl, butyl, pentyl, hexyl comprising a sulfonate leaving group having any suitable substituent such as triflate, nosylate, mesylate or tosylate and wherein, at least one of R12 and R16 is an aliphatic group comprising a sulfonate leaving group having any suitable substituent such as triflate, nosylate, mesylate or tosylate and wherein, any functional groups comprising OH, NH or SH are substituted with a protective group selected from the group comprising Boc, Fmoc, CH2C5H5, (CH2)nCH3, C(CH3)3, COCH3, COCF3, C(C5H5)3 and wherein the precursor molecule has a lipophilicity of ClogD_{7.4} < -0.5 after deprotection .

15. Use of a precursor molecule according to any of claim 8 or claim 9 for aliphatic ¹⁸F-labelling according to the method described in any of claim 1 to 3.
